(19) Europäisches Patentamt / European Patent Office / Office européen des brevets

(11) EP 2 459 571 B1

(12) **EUROPEAN PATENT SPECIFICATION**

(45) Date of publication and mention of the grant of the patent:
**11.03.2015 Bulletin 2015/11**

(21) Application number: **10742955.7**

(22) Date of filing: **30.07.2010**

(51) Int Cl.:
***C07D 498/04*** *(2006.01)* ***A61K 31/5365*** *(2006.01)*
***A61P 31/06*** *(2006.01)* ***A61P 31/04*** *(2006.01)*

(86) International application number:
**PCT/US2010/043906**

(87) International publication number:
**WO 2011/014774 (03.02.2011 Gazette 2011/05)**

(54) **NITROIMIDAZOOXAZINES AND THEIR USES IN ANTI-TUBERCULAR THERAPY**

NITROIMIDAZOOXAZINE UND IHRE VERWENDUNG ZUR BEHANDLUNG VON TUBERKULOSE

NITROIMIDAZOOXAZINES ET LEURS UTILISATIONS EN THÉRAPIE ANTITUBERCULEUSE

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **31.07.2009 US 230396 P**

(43) Date of publication of application:
**06.06.2012 Bulletin 2012/23**

(73) Proprietor: **Global Alliance For Tb Drug Development**
**New York, NY 10005 (US)**

(72) Inventors:
- **DENNY, William, Alexander**
  **Pakuranga**
  **Auckland 2010 (NZ)**
- **THOMPSON, Andrew, M.**
  **Auckland 1051 (NZ)**
- **BLASER, Adrian**
  **Maitakere City 0614 (NZ)**
- **MA, Zhenkun**
  **Westfield**
  **NJ 07090 (US)**
- **PALMER, Brian, Desmond**
  **Waitakere City 0618 (NZ)**
- **SUTHERLAND, Hamish, Scott**
  **Auckland 0604 (NZ)**
- **KMENTOVA, Iveta**
  **93021 Jahodna (SK)**

(74) Representative: **Gates, Marie Christina Esther et al**
**Tomkins & Co.**
**5 Dartmouth Road**
**Dublin 6 (IE)**

(56) References cited:
**WO-A1-97/01562**
**WO-A1-2006/043121**
**WO-A1-2009/120789**
**WO-A2-2007/075872**
**US-A- 6 087 358**

- **ANDREW M. THOMPSON, ADRIAN BLASER, ROBERT F. ANDERSON ET AL.: "Synthesis, Reduction Potentials, and Antitubercular Activity of Ring A/B Analogues of the Bioreductive Drug (6S)-2-Nitro-6-{[4-(trifluoromethoxy)benzy l] oxy}-6,7-dihydro-5H-imidazo[2,1-b][1,3]o xazine (PA-824)", J. MED. CHEM., vol. 52, 17 December 2008 (2008-12-17), XP002606769, 2009**
- **HAMISH S. SUTHERLAND ET AL.: "Synthesis and Structure-activity Relationships of Antitubercular 2-Nitroimidazooxazines Bearing Heterocyclic Side Chains", JOURNAL OF MEDICINAL CHEMISTRY, vol. 53, 12 July 2009 (2009-07-12), pages 855-866, XP002615097, DOI: 10.1021/jm901378u**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### BACKGROUND

The present invention relates to novel nitroimidazooxazines, to their preparation, and to their use as drugs for treating *Mycobacterium tuberculosis* and other microbial infections, either alone or in combination with other anti-infective treatments.

Tuberculosis remains a leading infectious cause of death worldwide (mortality estimated to be 1.3 million in 2008), with a recent resurgence attributable to an enhanced susceptibility in HIV patients, the increasing incidence of multidrug-resistant strains and the emergence of extensively drug resistant strains. Current drug therapy for tuberculosis is long and complex, involving multidrug combinations (usually isoniazid, rifampin, pyrazinamide and ethambutol) given daily for in excess of 6 months. Furthermore, these drugs are relatively ineffective against the persistent form of the disease, which is suggested to occur in a significant proportion of cases (Ferrara et al., 2006). Second-line drugs used in lengthy combination therapies for multidrug resistant disease (typically over 2 years) mostly have reduced potency or greater toxicity than existing first-line agents. Frequently, incomplete treatment is administered, leading to high relapse rates and increased drug resistance, underscoring the urgent need for new, more effective drugs.

It is an object of the present invention to provide new nitroimidazooxazines with unexpectedly high potency against both aerobic (replicating) and hypoxic (latent or persistent) cultures of *Mycobacterium tuberculosis* and unexpectedly high efficacy in mouse models of *Mycobacterium tuberculosis* infection for use as anti-tubercular drugs and for the treatment of other microbial infections.

### SUMMARY

The current invention pertains to nitroimidazooxazine compounds, their methods of preparation, and uses of the compounds as treatment for tuberculosis and other microbial infections.

The recent introduction of the nitroimidazooxazine PA-824 to clinical trial is significant, as this compound shows good in vitro and in vivo activity against *Mycobacterium tuberculosis* in both its active and persistent forms (Tyagi et al., 2005). A related 2-nitroimidazo[2,1-*b*]oxazole, OPC-67683 is also in clinical trial (Sasaki et al., 2006). The structures of these compounds are shown in Figure 1. Without wanting to be bound by theory, the mechanism of action of PA-824 is suggested to involve the release of nitric oxide (Singh et al., 2008), following a reductive step, in a process dependent on the bacterial glucose-6-phosphate dehydrogenase (FGD1) and its cofactor F420 (Stover et al., 2000). Microarray studies on mutant strains wild-type for both FGD1 and F420 show that a 151-amino acid (17.37 kDa) protein of unknown function, Rv3547, appears to be critical for this activation (Manjunatha et al., 2006). Recent mechanistic studies of the reductive chemistry of PA-824 support this contention (Anderson et al., 2008). Nitroimidazooxazine analogues and their use in tuberculosis have been previously reported (U.S. Patent Nos. 5,668,127 (1997) and 6,087,358 (2000); Jiricek et al., WO 2007075872A2 (2007); Li et al., 2008; Kim et al., 2009).

In a first aspect, the present invention pertains to a compound having a general structure of Formula I:

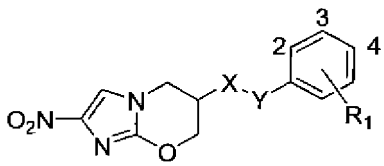

I

wherein X represents O, $OCH_2$, $OCH_2CH{=}CH$ or $OCH_2C{=}C$;

Y represents any one of formulae IIa-IIc shown below, where 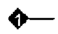 signifies the attachment to X and Formula IIa comprises a ring having R2 as a substituent;

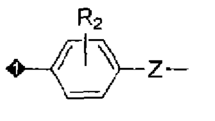
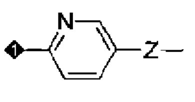
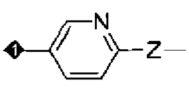

IIa IIb IIc

Z in Formulae IIa-IIc represents $CH_2$, CH=CH, C≡C or a direct bond;

numbers 2, 3, and 4 are ring positions on a terminal ring having $R_1$ as a substituent, the terminal ring of Formula I comprises C, CH or aza at each ring position, and $R_1$ and $R_2$ in Formulae I and IIa each represents any one, two or three substituents located at any available ring position and are independently H, F, Cl, $CF_3$, $OCF_2H$, $OCF_3$, or combinations thereof.

A preferred subclass of compounds has a general structure of Formula I above wherein:

X represents O, $OCH_2$, $OCH_2CH=CH$ or $OCH_2C{\equiv}C$;
Y represents any one of formulae IIa-IIc shown below, where ◆— signifies the attachment to X and Formula IIa comprises a ring having $R_2$ as a substituent;

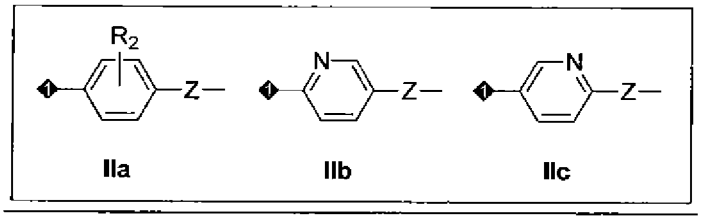

IIa IIb IIc

Z in Formulae IIa-IIc represents $CH_2$, CH=CH, C≡C or a direct bond;

numbers 2, 3, and 4 are ring positions on a terminal ring having $R_1$ as a substituent;

the terminal ring of Formula I comprises C, or CH at each ring position, or comprises aza at the 2-position and C, or CH at each remaining ring position, or comprises aza at the 3-position and C, or CH at each remaining ring positions;

$R_1$ in Formula I is any one or more of F located at ring position 4, $OCF_3$ located at ring position, 4, Cl located at ring position 2, Cl located at ring position 3, F located at ring position 3, $CF_3$ located at ring position, 4, or combinations thereof; and

$R_2$ in Formula IIa represents any one or two of H or F at any of the available ring positions.

These compounds, as well as mixtures thereof, isomers, and physiologically functional salt derivatives are useful in prevention of or therapy for treating *Mycobacteriun tuberculosis* and other microbial infections.

Figure 13 shows a general synthetic scheme for preparing representative compounds;

Figure 14 shows a general synthetic scheme for preparing representative compounds;

Figure 15 shows a general synthetic scheme for preparing representative compounds;

Figure 16 shows a general synthetic scheme for preparing representative compounds;

Figure 17 shows a general synthetic scheme for preparing representative compounds;

Figure 18 shows a general synthetic scheme for preparing representative compounds;

Figure 19 shows a general synthetic scheme for preparing representative compounds;

Figure 20 shows a general synthetic scheme for preparing representative compounds;

Figure 21 shows a general synthetic scheme for preparing representative compounds;

Figure 22 shows a general synthetic scheme for preparing representative compounds;

Figure 23 shows a general synthetic scheme for preparing representative compounds;

Figure 24 shows a general synthetic scheme for preparing representative compounds;

Figure 25 shows a general synthetic scheme for preparing representative compounds;

Figure 26 shows a general synthetic scheme for preparing representative compounds;

Figure 27 shows the structures of representative compounds 1-18 referred to in Table 1 and Examples 1-3; and

Figure 28 shows the structures of representative compounds 19-33 referred to in Table 1 and Examples 1-3.

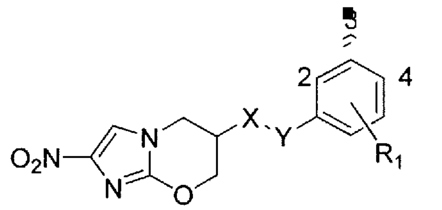

I

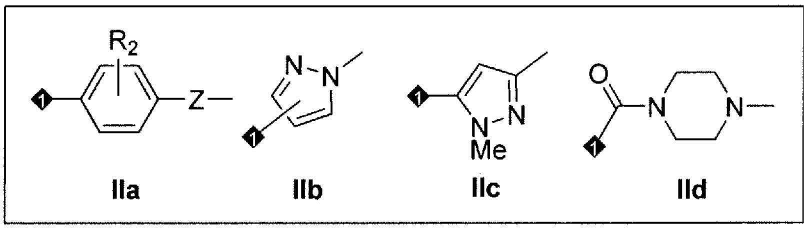

IIa IIb IIc IId

## Detailed Description

The current invention pertains to nitroimidazooxazine compounds, their methods of preparation, and uses of the compounds as treatment for tuberculosis and other microbial infections.

In a first aspect, the present invention pertains to a compound having a general structure of Formula I:

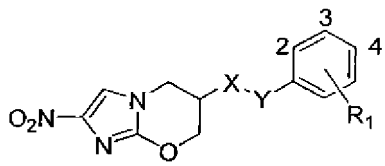

I

wherein X represents O, $OCH_2$, $OCH_2CH{=}CH$ or $OCH_2C{\equiv}C$;

Y represents any one of formulae IIa-IIc shown below, where signifies the attachment to X and Formula IIa comprises a ring having R2 as a substituent;

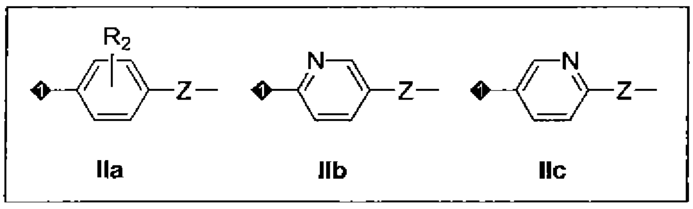

IIa IIb IIc

Z in Formulae IIa-IIc represents $CH_2$, CH=CH, C≡C or a direct bond; numbers 2, 3, and 4 are ring positions on a terminal ring having $R_1$ as a substituent, the terminal ring of Formula I comprises C, CH or aza at each ring position, and $R_1$ and $R_2$ in Formulae I and IIa each represents any one, two or three substituents located at any available ring position and are independently H, F, Cl, $CF_3$, $OCF_2H$, $OCF_3$, or combinations thereof.

A preferred subclass of compounds has a general structure of Formula I above wherein:

X represents O, $OCH_2$, $OCH_2CH=CH$ or $OCH_2C≡C$;
Y represents any one of formulae IIa-IIc shown below, where ◆— signifies the attachment to X and Formula IIa comprises a ring having $R_2$ as a substituent;

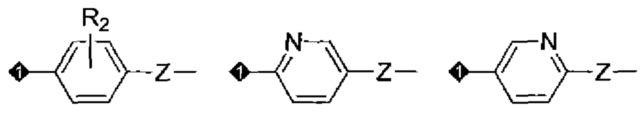

IIa IIb IIc

Z in Formula IIa-IIc represents $CH_2$, CH=CH, C=C or a direct bond;
numbers 2, 3, and 4 are ring positions on a terminal ring having $R_1$ as a substituent; the terminal ring of Formula I comprises C, or CH at each ring position, or comprises aza at the 2-position and C, or CH at each remaining ring position, or comprises aza at the 3-position and C, or CH at each remaining ring position;
$R_1$ in Formula I is any one or more of F located at ring position 4, $OCF_3$ located at ring position 4, Cl located at ring position 2, Cl located at ring position 3, F located at ring position 3, $CF_3$ located at ring position 4, or combinations thereof; and
$R_2$ in Formula IIa represents any one or two of H or F at any of the available ring positions.

The most highly preferred of the compounds described by Formula I are:

A. (6*S*)-6-{[2'-Chloro-4'-(trifluoromethoxy)[1,1'-biphenyl]-4-yl]methoxy}-2-nitro-6,7-dihydro-5*H*-imidazo[2,1-*b*][1,3]oxazine (compound 1 of Table 1 and Figure 27);

B. (6*S*)-6-{[3'-Fluoro-4'-(trifluoromethoxy)[1,1'-biphenyl]-4-yl]methoxy}-2-nitro-6,7-dihydro-5*H*-imidazo[2,1-*b*][1,3]oxazine (compound 2 of Table 1 and Figure 27);

C. (6*S*)-2-Nitro-6-{[4'-(trifluoromethoxy)[1,1'-biphenyl]-4-yl]methoxy}-6,7-dihydro-5*H*-imidazo[2,1-*b*][1,3]oxazine (compound 3 of Table 1 and Figure 27);

D. (6*S*)-2-Nitro-6-({4-[5-(trifluoromethyl)-2-pyridinyl]benzyl}oxy)-6,7-dihydro-5*H*-imidazo[2,1-*b*][1,3]oxazine (compound 8 of Table 1 and Figure 27);

E. (6*S*)-2-Nitro-6-({4-[6-(trifluoromethyl)-3-pyridinyl]benzyl}oxy)-6,7-dihydro-5*H*-imidazo[2,1-*b*][1,3]oxazine (compound 9 of Table 1 and Figure 27);

F. (6*S*)-6-{[3-Fluoro-4'-(trifluoromethoxy)[1,1'-biphenyl]-4-yl]methoxy}-2-nitro-6,7-dihydro-5*H*-imidazo[2,1-*b*][1,3]oxazine (compound 12 of Table 1 and Figure 27);

G. (6*S*)-2-Nitro-6-{[4'-(trifluoromethoxy)[1,1'-biphenyl]-4-yl]oxy}-6,7-dihydro-5*H*-imidazo[2,1-*b*][1,3]oxazine (compound 13 of Table 1 and Figure 27);

H. (6*S*)-6-({2-Fluoro-4-[5-(trifluoromethyl)-2-pyridinyl]benzyl}oxy)-2-nitro-6,7-dihydro-5*H*-imidazo[2,1-*b*][1,3]oxazine (compound 14 of Table 1 and Figure 27);

I. (6*S*)-6-{[2-Fluoro-4'-(trifluoromethoxy)[1,1'-biphenyl]-4-yl]methoxy}-2-nitro-6,7-dihydro-5*H*-imidazo[2,1-*b*][1,3]oxazine (compound 15 of Table 1 and Figure 27);

J. (6*S*)-2-Nitro-6-({4-[4-(trifluoromethoxy)benzyl]benzyl}oxy)-6,7-dihydro-5*H*-imidazo[2,1-*b*][1,3]oxazine (compound 17 of Table 1 and Figure 27);

K. (6*S*)-2-Nitro-6-({(2*E*)-3-[4'-(trifluoromethoxy)[1,1'-biphenyl]-4-yl]-2-propenyl}oxy)-6,7-dihydro-5*H*-imidazo[2,1-

*b*][1,3]oxazine (compound 19 of Table 1 and Figure 28);

L. (6*S*)-6-({2-Fluoro-4-[6-(trifluoromethyl)-3-pyridinyl]benzyl}oxy)-2-nitro-6,7-dihydro-5*H*-imidazo[2,1-*b*][1,3]oxazine (compound 23 of Table 1 and Figure 28);

M. (6*S*)-6-{[4-(5-Fluoro-2-pyridinyl)benzyl]oxy}-2-nitro-6,7-dihydro-5*H*-imidazo[2,1-*b*][1,3]oxazine (compound 26 of Table 1 and Figure 28);

N. (6*S*)-2-Nitro-6-({3-[4'-(trifluoromethoxy)[1,1'-biphenyl]-4-yl]-2-propynyl}oxy)-6,7-dihydro-5*H*-imidazo[2,1-*b*][1,3]oxazine (compound 30 of Table 1 and Figure 28);

O. (6*S*)-2-Nitro-6-[(4-{(*E*)-2-[4-(trifluoromethoxy)phenyl]ethenyl}benzyl)oxy]-6,7-dihydro-5*H*-imidazo[2,1-*b*][1,3]oxazine (compound 31 of Table I and Figure 28); and

P. (6S)-2-Nitro-6-[(4-{[4-(trifluoromethoxy)phenyl]ethynyl}benzyl)oxy]-6,7-dihydro-5*H*-imidazo[2,1-*b*][1,3]oxazine (compound 32 of Table 1 and Figure 28).

Additional highly preferred compounds described by Formula I are;

A. (6*S*)-6- {[6-(4-Fluorophenyl)-3-pyridinyl]methoxy}-2-nitro-6,7-dihydro-5*H*-imidazo[2,1-*b*][1,3]oxazine (compound 5 of Table 1 and Figure 27);
B. (6*S*)-2-Nitro-6-({6-[4-(trifluoromethoxy)phenyl]-3-pyridinyl}methoxy)-6,7-dihydro-5*H*-imidazo[2,1-*b*][1,3]oxazine (compound **6** of Table 1 and Figure 27);
C. (6*S*)-2-Nitro-6-({5-[4-(trifluoromethoxy)phenyl]-2-pyridinyl}methoxy)-6,7-dihydro-5*H*-imidazo[2,1-*b*][1,3]oxazine (compound 7 of Table 1 and Figure 27);
D. (6*S*)-2-Nitro-6-[(5-{[4-(trifluoromethoxy)phenyl]ethynyl}-2-pyridinyl)methoxy]-6,7-dihydro-5*H*-imidazo[2,1-*b*][1,3]oxazine (compound **18** of Table 1 and Figure 27);
E. (6*S*)-6-({6-[3-Fluoro-4-(trifluoromethoxy)phenyl]-3-pyridinyl}methoxy)-2-nitro-6,7-dihydro-5*H*-imidazo[2,1-*b*][1,3]oxazine (compound **21** of Table 1 and Figure 28);
F. (6S)-6-({5-[3-Fluoro-4-(trifluoromethoxy)phenyl]-2-pyridinyl}methoxy)-2-nitro-6,7-dihydro-5*H*-imidazo[2,1-*b*][1,3]oxazine (compound **22** of Table 1 and Figure 28);
G. (6*S*)-2-Nitro-6-[(5-{[6-(trifluoromethyl)-3-pyridinyl]ethynyl}-2-pyridinyl)methoxy]-6,7-dihydro-5*H*-imidazo[2,1-*b*][1,3]oxazine (compound **25** of Table 1 and Figure 28);
H. (6*S*)-6-({6-[3-Chloro-4-(trifluoromethoxy)phenyl]-3-pyridinyl}methoxy)-2-nitro-6,7-dihydro-5*H*-imidazo[2,1-*b*][1,3]oxazine (compound **28** of Table 1 and Figure 28): and
I. (6*S*)-2-Nitro-6-[(6-{[4-(trifluoromethoxy)phenyl]ethynyl}-3-pyridinyl)methoxy]-6,7-dihydro-5*H*-imidazo[2,1-*b*][1,3]oxazine (compound **33** of Table 1 and Figure 28).

Compounds of Formula I may occur in different geometric and enantiomeric forms, and both pure forms and mixtures of these separate isomers are included in the scope of this invention, as well as any physiologically functional or pharmacologically acceptable salt derivatives or prodrugs thereof. Production of these alternate forms would be well within the capabilities of one skilled in the art.

The current invention also pertains to methods of prevention or therapy for microbial infections, such as *Mycobacterium tuberculosis,* including the step of administering a compound of Formula I.

In another aspect of the present invention there is provided a pharmaceutical composition including a therapeutically effective amount of a compound of Formula I as defined above and a pharmaceutically acceptable excipient, adjuvant, carrier, buffer or stabiliser. A "therapeutically effective amount" is to be understood as an amount of a compound of Formula I that is sufficient to show anti-bacterial or anti-microbial effects. The actual amount, rate and time-course of administration will depend on the nature and severity of the disease being treated. Prescription of treatment is within the responsibility of general practitioners and other medical doctors. The pharmaceutically acceptable excipient, adjuvant, carrier, buffer or stabiliser should be non-toxic and should not interfere with the efficacy of the active ingredient. The precise nature of the carrier or other material will depend on the route of administration, which may be oral, or by injection, such as cutaneous, subcutaneous, or intravenous injection, or by dry powder inhaler.

Pharmaceutical compositions for oral administration may be in tablet, capsule, powder or liquid form. A tablet may comprise a solid carrier or an adjuvant. Liquid pharmaceutical compositions generally comprise a liquid carrier such as water, petroleum,

In Scheme 1, shown in Figure 3, reagents and conditions were (i) 2M $Na_2CO_3$, toluene, EtOH, Pd(dppf)$Cl_2$ under $N_2$, 88 °C, 1-2.5 h; (ii) 30% HBr/AcOH, 20 °C, 6-11 h; (iii) NaH, DMF, 0-20 °C, 3 h. Suzuki couplings of 4-(hydroxymethyl)phenylboronic acid **(34)** with halides **35** and **36** in the presence of Pd(dppf)$Cl_2$ gave the biphenyl alcohols **37** and **38,** which were converted to the corresponding bromomethyl compounds **39** and **40.** Coupling of these with the known alcohol **41** (reported in US Patent No. 5,668,127 via 4 steps, starting from 2,4-dinitroimidazole and *tert*-butyldimethylsilyl (*S*)-glycidyl ether) gave the desired compounds 1 and 2 of Table 1.

In Scheme 2, shown in Figure 4, reagents and conditions were (i) NaH, DMF, 5-20 °C, 2 h; (ii) 2M $K_2CO_3$, toluene, EtOH, Pd(dppf)$Cl_2$ under $N_2$, reflux, 30 min. Similar NaH-assisted coupling of alcohol **41** with 4-iodobenzyl bromide **(42)** gave the known 4-iodobenzyl ether **43** (reported in US Patent No. 6,087,358 via the same procedure), which underwent Suzuki coupling as in Scheme 1 with 4-(trifluoromethoxy)phenylboronic acid **(44)** to give compound 3 of Table 1.

In Scheme 3, shown in Figure 5, reagents and conditions were (i) MsCl, $Et_3N$, THF, 0 °C, 30 min, then NaI, acetone, reflux, 1 h; (ii) NaH, DMF, -78 to 0 °C, 1 h; (iii) 2M $K_2CO_3$, toluene, EtOH, Pd(dppf)$Cl_2$ under $N_2$, reflux, 30 min. NaH-assisted coupling of alcohol **41** with 2-chloro-5-(iodomethyl)pyrazine **(46)** (prepared from the known (5-chloro-2-pyrazinyl)methanol **(45)** (obtained by chlorination and reduction of 5-hydroxypyrazine-2-carboxylic acid, as reported by Kiener et al., 1994) by reaction with MsCl followed by NaI) gave chloride **47.** This underwent Suzuki coupling with 4-(trifluoromethoxy)phenylboronic acid **(44)** to give compound **4** of Table 1.

In Scheme 4, shown in Figure 6, reagents and conditions were: (i) NaH, DMF, 5-20 °C, 16 h; (ii) **44,** 2M $K_2CO_3$, DME, Pd(dppf)$Cl_2$ under $N_2$, 90 °C, 2 days; (iii) NBS, $PPh_3$, $CH_2Cl_2$, 20 °C, 3.5 h; (iv) **41,** NaH, DMF, 0-20 °C, 2.5 h; (v) **55-57,** 2M $Na_2CO_3$, toluene, EtOH, Pd(dppl)$Cl_2$ under $N_2$, 90 °C, 20-120 min; (vi) aq $NaNO_2$, 25% $H_2SO_4$, 0 °C, 12 min, then aq KI, 20 °C, 10 min, then 52 °C, 2 h; (vii) n-BuLi, B(O*i*Pr)$_3$, toluene, THF, -78 to -20 °C, 5 h, then 2N HCl. NaH-assisted coupling of 2-chloro-5-(chloromethyl)pyridine **48** with alcohol **41** gave the chloride 49, which was Suzuki coupled with 4-(trifluoromethoxy)phenylboronic acid **(44)** to give compound 6 of Table 1. Bromination of commercial (6-bromo-3-pyridinyl)methanol **(50)** with NBS/$PPh_3$ gave the bromomethylpyridine **51,** which was similarly NaH-coupled with alcohol **41** to give bromide **52.** This was Suzuki coupled with boronic acids **55** (obtained from aniline **53** via the novel iodide **54), 56** or **57** to give respectively compounds **28, 21** and 5 of Table 1.

In Scheme 5, shown in Figure 7, reagents and conditions were: (i) NaH, DMF, 5-20 °C, 2 h; (ii) **44,** 2M $K_2CO_3$, toluene, EtOH, Pd(dppf)$Cl_2$ under $N_2$, 90 °C, 30 min. NaH-assisted coupling of 5-bromo-2-(chloromethyl)pyridine **(58)** (prepared by chlorination of (5-bromo-2-pyridinyl)methanol, as reported by van den Heuvel et al., 2004) with alcohol **41** gave the bromide **59,** which was Suzuki coupled with 4-(trifluoromethoxy)phenylboronic acid **(44)** to give compound 7 of Table 1.

In Scheme 6, shown in Figure 8, reagents and conditions were: (i) NaH, DMF, 20 °C, 1 h; (ii) bis(pinacolato)diboron, Pd(dppf)$Cl_2$ under $N_2$, KOAc, DMSO, 90 °C, 1 h; (iii) 2-chloro-5-(trifluoromethyl)pyridine or 5-bromo-2-(trifluoromethyl)pyridine, 2M $K_2CO_3$, toluene, EtOH, Pd(dppf)$Cl_2$ under $N_2$, reflux, 30 min. The bromide **61** was prepared by NaH-assisted coupling of alcohol **41** with 4-bromobenzyl bromide **(60).** Reaction of **61** with bis(pinacolato)diboron gave the 4-boronate ester **62,** which underwent Suzuki coupling with 2-chloro-5-(trifluoromethyl)pyridine or 5-bromo-2-(trifluoromethyl)pyridine to give respectively compounds **8** and **9** of Table 1.

In Scheme 7, shown in Figure 9, reagents and conditions were: (i) Aqueous pyridine, -5 °C, 30 min; (ii) bicyclo[2.2.1]hepta-2,5-diene, $Et_3N$, toluene, 70 °C, 1 h, then xylene, reflux, 2 h; (iii) $LiAlH_4$, $Et_2O$, 0-20 °C, 1 h; (iv) $PBr_3$, $Et_2O$, 20 °C, 17 h; (v) **41,** NaH, DMF, 0 °C, 2 h. Ethyl (2Z)-chloro{[4-(trifluoromethoxy)phenyl]hydrazono}ethanoate **(65)** [from 4-(trifluoromethoxy)benzenediazonium tetrafluoroborate **(63)** and ethyl 2-chloroacetoacetate **(64)**] was reacted with bicyclo[2.2.1]hepta-2,5-diene to give the carboxylate **66.** This was reduced ($LiAlFL_4$) to alcohol **67,** which was then brominated with $PBr_3$ to give bromide **68.** NaH-assisted coupling with alcohol **41** then gave compound **10** of Table 1.

In Scheme 8, shown in Figure 10, reagents and conditions were: (i) CuI, $PdCl_2(PPh_3)_2$, methylhydrazine sulfate, aqueous $NaHCO_3$, THF, 20 °C, 2 days in CO atmosphere; (ii) 4N HCl, THF, 80 °C, 16 h; (iii) $PBr_3$, $Et_2O$, 0-20 °C, 16 h; (iv) **41,** NaH, DMF, 0 °C, 2 h. Pyrazole **71** was prepared by the reaction of 2-(2-propynyloxy)tetrahydro-2*H*-pyran **(69),** 1-iodo-4-(trifluoromethoxy)benzene **(70)** and methylhydrazine in the presence of CuI and $PdCl_2(PPh_3)_2$ and an atmosphere of CO. Hydrolysis of THP ether **71** to alcohol **72,** followed by bromination with $PBr_3$, gave bromide **73,** which underwent NaH-assisted coupling with alcohol **41** to give compound **11** of Table 1.

In Scheme 9, shown in Figure 11, reagents and conditions were: (i) NaH, DMF, 0-20 °C, 3 h; (ii) $ArB(OH)_2$, 2M $Na_2CO_3$, toluene, EtOH, Pd(dppf)$Cl_2$ under $N_2$, 85-90 °C, 1-3 h; (iii) bis(pinacolato)diboron, Pd(dppf)$Cl_2$ under $N_2$, KOAc, DMSO, 89 °C, 5 h; (iv) 2-chloro-5-(trifluoromethyl)pyridine, 2M $Na_2CO_3$, toluene, EtOH, Pd(dppf)$Cl_2$ under $N_2$, 90 °C, 120 min. NaH-assisted coupling of 4-bromo-2-fluorobenzyl bromide (74) with alcohol 41 gave the bromide **75,** which was Suzuki coupled with the appropriate arylboronic acids to give compounds **12** and **23** of Table 1. Reaction of bromide **75** with bis(pinacolato)diboron gave the boronate ester **76,** which underwent Suzuki coupling with 2-chloro-5-(trifluoromethyl)pyridine to give compound **14** of Table 1.

In Scheme 10, shown in Figure 12, reagents and conditions were: (i) cat. CsF, $PhCH_2OH$, 120 °C, 16 h; (ii) TIPSCl, imidazole, DMF, 20 °C, 16 h; (iii) 4'-(trifluoromethoxy)[1,1'-biphenyl]-4-ol, DIAD, $PPh_3$, benzene, 5-20 °C, 18 h; (iv) $H_2$, 5% Pd-C, EtOAc, EtOH, 60 psi, 4 h; (v) $I_2$, $PPh_3$, imidazole, benzene, 20 °C, 1 h; (vi) 2-bromo-4(5)-nitroimidazole, $K_2CO_3$, DMF, 87 °C, 20 h; (vii) TBAF, THF, 20 °C, 1 h; (viii) NaH, DMF, 5-20 °C, 30 min. Reaction of (*S*)-glycidol (77)

and benzyl alcohol in the presence of CsF gave diol **78,** which was mono-protected with TIPS chloride and the resulting alcohol **79** was Mitsunobu coupled with 4'-(trifluoromethoxy)[1,1'-biphenyl]-4-ol (reported by Edsall et al., 2003, via Suzuki coupling of 4-bromophenol and boronic acid **44)** to give ether **80.** This was debenzylated by hydrogenolysis, and the resulting alcohol **81** was iodinated with $I_2$/$PPh_3$ to give **82.** This was coupled with 2-bromo-4(5)-nitroimidazole, and the resulting compound **83** was desilylated with TBAF and ring closed with NaH to give compound **13** of Table 1.

In Scheme 11, shown in Figure 13, reagents and conditions were: (i) $NaBH_4$, $I_2$, THF, 0-20 °C, 14 h; (ii) 30% HBr/AcOH, 20 °C, 20 h; (iii) NaH, DMF, 0-20 °C, 3.5 h; (iv) 2M $Na_2CO_3$, toluene, EtOH, Pd(dppf)$Cl_2$ under $N_2$, 90 °C, 6 h. NaH-assisted coupling of 4-bromo-3-fluorobenzyl bromide **(86)** (prepared from the acid **84** via the known alcohol **85** (reported by deSolms et al., 2003, via borane reduction of **84**) with oxazine alcohol **41** gave the bromide **87,** which underwent Suzuki coupling with 4-(trifluoromethoxy)phenylboronic acid **(44)** to give compound **15** of Table 1.

In Scheme 12, shown in Figure 14, reagents and conditions were: (i) **44,** aqueous $Na_2CO_3$, toluene, EtOH, $Pd(PPh_3)_4$ under $N_2$, reflux, 18 h; (ii) n-BuLi, THF, -95 °C, 0.5 min, then DMF, -90 °C, 20 min; (iii) $NaBH_4$, MeOH, 0 °C, 1 h; (iv) MsCl, $Et_3N$, THF, 0 °C, 1 h, then LiBr, $Me_2CO$, reflux, 1 h; (v) **41,** NaH, DMF, -78 to 0 °C, 1 h. Suzuki coupling of boronic acid **44** and 2-iodo-5-bromopyrimidine **(88)** gave the bromide **89,** which was treated with n-BuLi and DMF to give the aldehyde **90.** This was reduced with $NaBH_4$ to the alcohol **91,** which was reacted with MsCl followed by LiBr to give the bromide **92.** Coupling of **92** with alcohol **41** gave compound 16 of Table 1.

In Scheme 13, shown in Figure 15, reagents and conditions were: (i) **44,** 2M $K_2CO_3$, DME, $Pd(PPh_3)_4$ under $N_2$, 105 °C, 24 h; (ii) $LiAlH_4$, $Et_2O$, 20 °C, 3 h; (iii) $PBr_3$, $CH_2Cl_2$, 20 °C, 2 h; (iv) **41,** NaH, DMF, 20 °C, 3 h. Suzuki coupling of methyl 4-(bromomethyl)benzoate **(93)** and 4-(trifluoromethoxy)phenylboronic acid **(44)** gave the methyl benzoate **94.** This was reduced with $LiAlH_4$ to the alcohol **95,** which gave the bromide 96 on treatment with $PBr_3$. Coupling of this bromide with alcohol **41** then gave compound **17** of Table 1.

In Scheme 14, shown in Figure 16, reagents and conditions were: (i) HC≡CTMS, $Et_3N$, DMF, CuI, $PdCl_2(PPh_3)_2$ under $N_2$, 50 °C, 18 h, then TBAF, THF, 0-20 °C, 2 h; (ii) **70** or **98,** $Et_3N$, DMF, CuI, $PdCl_2(PPh_3)_2$ under $N_2$, 20 or 50 °C, 0.5 h. Sonogashira coupling of bromide **59** (see Scheme 5) with ethynylTMS in the presence of $Et_3N$, CuI and $PdCl_2(PPh_3)_2$, followed by desilylation with TBAF gave the acetylene **97,** which was similarly coupled with 1-iodo-4-(trifluoromethoxy)benzene **(70)** or 5-bromo-2-(trifluoromethyl)pyridine (98) to give respectively compounds **18** and **25** of Table 1.

In Scheme 15, shown in Figure 17, reagents and conditions were: (i) **44,** dioxane, 2M $K_2CO_3$, Pd(dppf)$Cl_2$ under $N_2$, reflux, I h; (ii) DIBAL-H, toluene, -78 to 20 °C, 1 h; (iii) $PBr_3$, $Et_2O$, 0-20 °C, 1 h; (iv) **41,** NaH, DMF, -78 to 0 °C, 1 h. Bromide **99** was Suzuki coupled to boronic acid **44** (see Scheme 3) to give ester **100,** which was reduced with DIBAL-H in toluene to give alcohol **101.** Bromination of **101** with $PBr_3$ gave **102,** which underwent NaH-assisted coupling with alcohol **41** to give compound **19** of Table 1.

In Scheme 16, shown in Figure 18, reagents and conditions were: (i) Triphosgene, $Et_3N$, 0-20 °C, 105 min; (ii) THF, 20 °C, 2 h. Alcohol **41** was treated with triphosgene, and the crude carbonyl chloride **103** was reacted directly with 1-[4-(trifluoromethoxy)phenyl]piperazine **(104)** to give compound **20** of Table 1.

In Scheme 17, shown in Figure 19, reagents and conditions were: (i) **56,** 2M $Na_2CO_3$, toluene, EtOH, Pd(dppf)$Cl_2$ under $N_2$, 89 °C, 2 h; (ii) NBS, $PPh_3$, $CH_2Cl_2$, 20 °C, 3 h; (iii) NaH, DMF, 0-20 °C, 2.5 h. Suzuki coupling of bromide **105** with boronic acid **56** (see Scheme 4) gave alcohol **106,** which was brominated with NBS/$PPh_3$ to give **107.** This underwent NaH-assisted coupling with alcohol **41 to** give compound **22** of Table 1.

In Scheme 18, shown in Figure 20, reagents and conditions were: (i) NaH, DMF, -42 °C, 1 h; (ii) **44,** 2M $K_2CO_3$, toluene, EtOH, Pd(dppl)$Cl_2$ under $N_2$, reflux, 0.5 h. Low-temperature reaction of oxazine alcohol **41** and 3-(bromomethyl)-6-chloropyridazine **(108)** (obtained via free radical bromination of 3-chloro-6-methylpyridazine, as reported by Ohshita J., EP 1555259, 2005) gave the chloride **109,** which was Suzuki coupled with boronic acid **44** (see Scheme 3) to give compound **24** of Table 1.

In Scheme 19, shown in Figure 21, reagents and conditions were: (i) 2M $Na_2CO_3$, toluene, EtOH, Pd(dppf)$Cl_2$ under $N_2$, 89 °C, 200 min; (ii) NBS, $PPh_3$, $CH_2Cl_2$, 20 °C, 3 h; (iii) **41,** NaH, DMF, 0-20 °C, 135 min. Suzuki coupling of bromide **110** with boronic acid **34** gave alcohol **111,** which was brominated to **112** and this was then coupled to alcohol **41** to give compound **26** of Table 1.

In Scheme 20, shown in Figure 22, reagents and conditions were: (i) aqueous NaOAc, AcOH, 100 °C, 15 h; (ii) $(CH_3)_2CH(CH_2)_2ONO$, THF, reflux, 20 h; (iii) LiAlH4, $Et_2O$, reflux, 2 h; (iv) $PBr_3$, $Et_2O$, 0 °C, 2 h; (v) **41,** NaH, DMF, 0 °C, 2 h. Reaction of ethyl (2*E*)-2-cyano-3-ethoxy-2-propenoate **113** and hydrazine **114** gave the pyrazolecarboxylate **115,** which was deaminated with isoamyl nitrite. The resulting carboxylate **116** was reduced to the alcohol **117,** which was then brominated with $PBr_3$ to give **118.** This underwent NaH-assisted coupling with alcohol **41** to give compound **27** of Table 1.

In Scheme 21, shown in Figure 23, reagents and conditions were: (i) NBS, AIBN, $CCl_4$, 60 °C, 3 h; (ii) NaH, DMF, -78 to 0 °C, 0.5 h; (iii) **44,** 2M $K_2CO_3$, toluene, EtOH, Pd(dppf)$Cl_2$ under $N_2$, reflux, 0.5 h. Bromination of 5-bromo-2-methylpyrimidine **(119)** gave **120** which underwent NaH-assisted coupling with alcohol **41** to give the bromide **121.** Suzuki coupling **of 121** with boronic acid **44** (see Scheme 3) gave compound **29** of Table 1.

In Scheme 22, shown in Figure 24, reagents and conditions were: (i) NaH, DMF, 0 °C, 1 h; (ii) 44, 2M $K_2CO_3$, toluene, EtOH, Pd(dppl)$Cl_2$ under $N_2$, reflux, 0.5 h. NaH-assisted coupling of alcohol **41** and 1-bromo-4-(3-bromo-1-propynyl)benzene **(122)** (prepared in two steps from 1-bromo-4-iodobenzene and propargyl alcohol, as described in WO 9524400) gave the bromide **123,** which was Suzuki coupled with boronic acid **44** to give compound **30** of Table 1.

In Scheme 23, shown in Figure 25, reagents and conditions were: (i) $K_2CO_3$, 18-crown-6, THF, $CH_2Cl_2$, reflux, 18 h; (ii) $LiAlH_4$, $Et_2O$, 0-20 °C, 0.5 h; (iii) $PBr_3$, $CH_2Cl_2$, 0-20 °C, 1 h; (iv) **41,** NaH, DMF, -78 to 0 °C, 1 h. Wittig reaction of aldehyde 125 and phosphonium salt **124** gave ester **126,** which was reduced with reduced with $LiAlH_4$ to give alcohol **127.** Bromination of **127** with $PBr_3$ gave **128,** which underwent NaH-assisted coupling with alcohol **41** to give compound **31** of Table 1.

In Scheme 24, shown in Figure 26, reagents and conditions were: (i) HC≡CTMS, $Et_3N$, DMF, CuI, $PdCl_2(PPh_3)_2$ under $N_2$, 20 °C, 0.5-18 h, then TBAF, THF, 0-20 °C, 2 h; (ii) **70,** $Et_3N$, DMF, CuI, $PdCl_2(PPh_3)_2$ under $N_2$, 20 °C, 0.5 h. Sonogashira couplings of iodide **43** (see Scheme 2) or bromide **52** (see Scheme 4) with ethynylTMS in the presence of $Et_3N$, CuI and $PdCl_2(PPh_3)_2$, followed by desilylation with TBAF, gave the acetylenes **129** or **130,** respectively, which were similarly coupled with 1-iodo-4-(trifluoromethoxy)benzene **(70)** to give compounds **32** and **33** of Table 1.

## EXAMPLE 2. METHODS OF PREPARATION

**A. Synthesis of (6*S*)-6-{[2'-chloro-4'-(trifluoromethoxy)[1,1'-biphenyl]-4-yl]methoxy}-2-nitro-6,7-dihydro-5*H*-imidazo[2,1-*b*][1,3]oxazine (1) by the method of Scheme 1.**

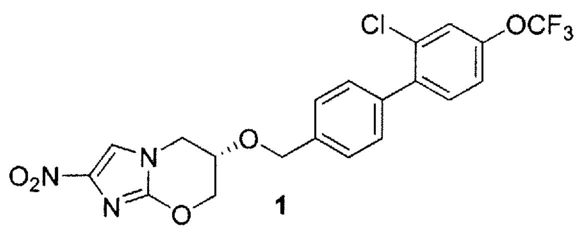

**1**

A stirred mixture of 4-(hydroxymethyl)phenylboronic acid (34) (308 mg, 2.03 mmol) and Pd(dppf)$Cl_2$ (191 mg, 0.261 mmol) in toluene (22 mL) and EtOH (11 mL) was degassed for 8 min (vacuum pump) and then $N_2$ was added. An aqueous solution of 2M $Na_2CO_3$ (4.4 mL, 8.8 mmol) was added by syringe and the stirred mixture was again degassed for 8 min, and then $N_2$ was added. 2-Chloro-1-iodo-4-(trifluoromethoxy)benzene (35) (585 mg, 1.81 mmol) was added by syringe and the resulting mixture was stirred at 88 °C for 60 min. The cooled mixture was then diluted with aqueous $NaHCO_3$ (100 mL) and extracted with $CH_2Cl_2$ (5x 100 mL). The extracts were evaporated to dryness and the residue was chromatographed on silica gel. Elution with 0-50% $CH_2Cl_2$/petroleum ether firstly gave foreruns, and then further elution with 50% $CH_2Cl_2$/petroleum ether gave 2'-chloro-4'-(trifluoromethoxy)[1,1'-biphenyl]-4-yl]methanol **(37)** (537 mg, 98%) as a white solid: mp (pentane) 38-39 °C; $^1$H NMR ($CDCl_3$) δ 7.46 (br d, *J* = 8.2 Hz, 2 H), 7.42 (dt, *J* = 8.3, 2.0 Hz, 2 H), 7.37 (br s, 1 H), 7.36 (d, *J* = 8.5 Hz, 1 H), 7.19 (m, 1 H), 4.77 (d, *J* = 5.9 Hz, 2 H), 1.70 (t, *J* = 5.9 Hz, 1 H); HREIMS calcd for $C_{14}H_{10}ClF_3O_2$ *m/z* ($M^+$) 304.0292, 302.0321, found 304.0294, 302.0317.

HBr in AcOH (5 mL of 33% w/w) was added to a solution of alcohol **37** (618 mg, 2.04 mmol) in glacial AcOH (2.5 mL), and the mixture was stirred at room temperature for 11 h. The resulting orange solution was added slowly to ice-water (50 mL) with stirring, and then the mixture was extracted with pentane (6x 50 mL). The extracts were washed with ice-water (50 mL) and then evaporated to give 4-(bromomethyl)-2'-chloro-4'-(trifluoromethoxy)-1,1'-biphenyl **(39)** (743 mg, 100%) as an oil; $^1$H NMR ($CDCl_3$) δ 7.47 (dt, *J* = 8.3, 1.9 Hz, 2 H), 7.39 (dt, *J* = 8.3, 1.9 Hz, 2 H), 7.37 (m, 1 H), 7.35 (d, *J* = 8.5 Hz, 1 H), 7.19 (m, 1 H), 4.55 (s, 2 H); HREIMS calcd for $C_{14}H_9BrClF_3O$ *m/z* ($M^+$) 367.9427, 365.9457, 363.9477, found 367.9428, 365.9453, 363.9485.

A stirred solution of (6*S*)-2-nitro-6,7-dihydro-5*H*-imidazo[2,1-*b*][1,3]oxazin-6-ol (41) (reported in US Patent No. 5,668,127 via 4 steps, starting from 2,4-dinitroimidazole and tert-butyldimethylsilyl (*S*)-glycidyl ether) (342 mg, 1.85 mmol) and bromide 39 (741 mg, 2.03 mmol) in anhydrous DMF (7 mL) under $N_2$ at 0 °C was treated with 60% NaH (111 mg, 2.78 mmol), then quickly degassed and resealed under $N_2$. After stirring at room temperature for 3 h, the reaction was cooled ($CO_2$/acetone), quenched with ice/aqueous $NaHCO_3$ (20 mL), added to brine (80 mL) and extracted with $CH_2Cl_2$ (6x 80 mL). The combined extracts were evaporated to dryness and the residue was chromatographed on silica gel, eluting with $CH_2Cl_2$, to give 1 (694 mg, 80%) as a light yellow solid: mp ($CH_2Cl_2$/pentane) 80-82 °C; $^1$H NMR ($CDCl_3$) δ 7.45-7.35 (m, 6 H), 7.34 (d, *J* = 8.5 Hz, 1 H), 7.20 (m, 1 H), 4.79 (d, *J* = 12.0 Hz, 1 H), 4.68 (d, *J* = 12.1 Hz, 1 H), 4.65 (ddd, *J* = 12.1, 2.9, 2.5 Hz, 1 H), 4.37 (br d, *J* = 11.8 Hz, 1 H), 4.24-4.12 (m, 3 H). Anal. ($C_{20}H_{15}ClF_3N_3O_5$) C, H, N.

**B. Synthesis of 6*S*)-6-{[3'-fluoro-4'-(trifluoromethoxy)[1,1'-biphenyl]-4-yl]methoxy}-2-nitro-6,7-dihydro-5*H*-imidazo[2,1-*b*][1,3]oxazine (2) by the method of Scheme 1.**

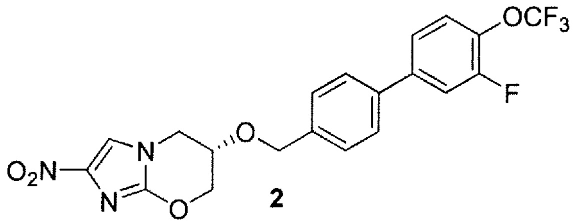

**2**

Suzuki coupling of 4-(hydroxymethyl)phenylboronic acid (34) and 4-bromo-2-fluoro-1-(trifluoromethoxy)benzene **(36)** as in Example 2A for 2.5 h, followed by chromatography of the product on silica gel, eluting with 0-40% $CH_2Cl_2$/petroleum ether (foreruns) and then 40% $CH_2Cl_2$/petroleum ether, gave 3'-fluoro-4'-(trifluoromethoxy)[1,1'-biphenyl]-4-yl]methanol **(38)** (73%) as a cream solid: mp ($CH_2Cl_2$/pentane) 70-71 °C; $^1H$ NMR ($CDCl_3$) $\delta$ 7.54 (dt, *J* = 8.3, 1.8 Hz, 2 H), 7.46 (br d, *J* = 8.2 Hz, 2 H), 7.41 (br d, *J* = 11.2 Hz, 1 H), 7.40-7.32 (m, 2 H), 4.76 (d, *J* = 5.9 Hz, 2 H), 1.69 (t, *J* = 5.9 Hz, I H); HREIMS calcd for $C_{14}H_{10}F_4O_2$ *m*/*z* ($M^+$) 286.0617, found 286.0616.

Bromination of alcohol 38 as in Example 2A for 6 h gave 4-(bromomethyl)-3'-fluoro-4'-(trifluoromethoxy)-1,1'-biphenyl (40) (100%) as a cream solid that was used directly in the next step; $^1H$ NMR ($CDCl_3$) $\delta$ 7.52 (dt, *J* = 8.5, 2.2 Hz, 2 H), 7.48 (dt, *J* = 8.5, 2.2 Hz, 2 H), 7.43-7.32 (m, 3 H), 4.54 (s, 2 H); HRAPCIMS calcd for $C_{14}H_9F_4O$ *m*/*z* $[M - Br]^+$ 269.0584, found 269.0572.

Reaction of bromide 40 (0.99 equiv.) with alcohol 41 as in Example 2A, followed by chromatography of the product on silica gel, eluting with 0-2% EtOAc/$CH_2Cl_2$ (foreruns) and then 2% EtOAc/$CH_2Cl_2$, gave 2 (76%) as a cream solid: mp ($CH_2Cl_2$/pentane) 169-171 °C; $^1H$ NMR ($CDCl_3$) $\delta$ 7.54 (dt, *J* = 8.3, 1.8 Hz, 2 H), 7.43-7.32 (m, 6 H), 4.78 (d, *J* = 12.0 Hz, 1 H), 4.67 (d, *J* = 11.9 Hz, 1 H), 4.64 (ddd, *J* = 12.1, 3.7, 2.1 Hz, 1 H), 4.37 (dd, *J* = 12.1, 1.3 Hz, 1 H), 4.23-4.12 (m, 3 H). Anal. ($C_{20}H_{15}F_4N_3O_5$) C, H, N.

**C. Synthesis of (6*S*)-2-nitro-6-{[4'-(trifluoromethoxy)[1,1'-biphenyl]-4-yl]methoxy}-6,7-dihydro-5*H*-imidazo[2,1-*b*][1,3]oxazine (3) by the method of Scheme 2.**

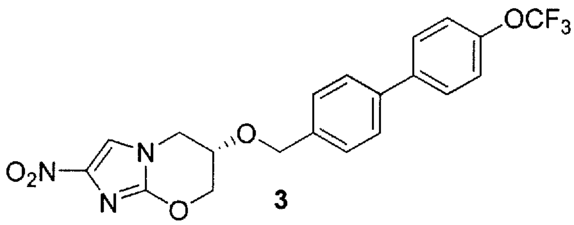

**3**

Reaction of alcohol 41 with 4-iodobenzyl bromide (42) and NaH in DMF at room temperature for 2 h gave (6*S*)-6-[(4-iodobenzyl)oxy]-2-nitro-6,7-dihydro-5*H*-imidazo[2,1-*b*][1,3]oxazine (43) (reported in US Patent No. 6,087,358 via the same procedure) (97%) as a pale yellow solid: mp (EtOAc/petroleum ether) 210-212 °C; $^1H$ NMR $[(CD_3)_2SO]$ $\delta$ 8.01 (s, 1 H), 7.71 (dt, *J* = 8.3, 2.0 Hz, 2 H), 7.13 (br d, *J* = 8.3 Hz, 2 H), 4.67-4.60 (m, 2 H), 4.59 (d, *J* = 12.2 Hz, 1 H), 4.46 (d, *J* = 12.0 Hz, 1 H), 4.27-4.19 (m, 3 H). Anal. ($C_{13}H_{12}N_3O_4$) C, H, N.

Suzuki coupling of iodide 43 and 4-(trifluoromethoxy)phenylboronic acid (44) as in Example 2D below gave **3** (86%) as a cream solid: mp ($CH_2$/$Cl_2$/hexane) 199-201 °C; $^1H$ NMR $[(CD_3)_2SO]$ $\delta$ 8.03 (s, 1 H), 7.78 (dt, *J* = 8.8, 2.6 Hz, 2 H), 7.66 (br d, *J* = 8.3 Hz, 2 H), 7.43 (br t, *J* = 8.5 Hz, 4 H), 4.72 (d, *J* = 12.2 Hz, 1 H), 4.70-4.66 (m, 2 H), 4.49 (d, *J* = 11.9 Hz, 1 H), 4.31-4.21 (m, 3 H). Anal. ($C_{20}H_{16}F_3N_3O_5$) C, H, N.

**D. Synthesis of (6*S*)-2-nitro-6-({5-[4-(trifluoromethoxy)phenyl]-2-pyrazinyl}methoxy)-6,7-dihydro-5*H*-imidazo[2,1-*b*][1,3]oxazine (4) by the method of Scheme 3.**

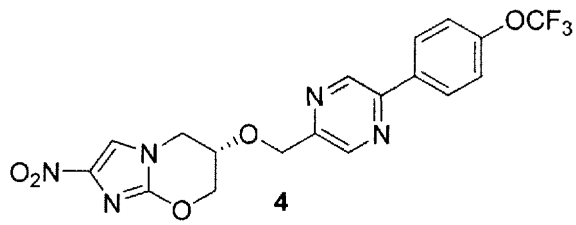

4

$Et_3N$ (4.17 mL, 29.9 mmol) and mesyl chloride (1.57 mL, 20.3 mmol) were added to a solution of (5-chloro-2-pyrazinyl)methanol (45) (obtained by chlorination and reduction of 5-hydroxypyrazine-2-carboxylic acid, as reported by Kiener et al., 1994) (1.443 g, 9.98 mmol) in anhydrous THF (20 mL) at 0 °C. The mixture was stirred at 0 °C for 0.5 h, then partitioned between EtOAc and water. The organic fraction was dried ($MgSO_4$) and the solvent was removed under reduced pressure to give the crude mesylate. The mesylate was dissolved in acetone (40 mL), sodium iodide (7.5 g, 50 mmol) was added, and the mixture was refluxed for 1 h. The solvent was removed under reduced pressure and the residue was partitioned between EtOAc and water. The organic fraction was concentrated under reduced pressure and the residue was chromatographed on silica gel (eluting with $CH_2Cl_2$) to give 2-chloro-5-(iodomethyl)pyrazine **(46)** (1.54 g, 61%), which was used immediately due to its instability.

NaH (60% w/w, 0.36 g, 9.0 mmol) was added to a solution of oxazine alcohol 41 (0.93 g, 5.02 mmol) and iodide **46** (1.54 g, 6.05 mmol) in DMF (10 mL) at -78 °C. The mixture was stirred at 0 °C for 1 h and then quenched with ice. EtOAc (200 mL) was added, the organic layer was dried ($MgSO_4$) and concentrated under reduced pressure. The residue was chromatographed on silica gel, eluting with a gradient of 0-5% MeOH/EtOAc, to give (6*S*)-6-[(5-chloro-2-pyrazinyl)methoxy]-2-nitro-6,7-dihydro-5*H*-imidazo[2,1-*b*][1,3]oxazine (47) (1.015 g, 65%) as a white solid: mp 181-183 °C; $^1H$ NMR [$(CD_3)_2SO$] $\delta$ 8.76 (d, *J* = 1.4 Hz, 1 H), 8.50 (d, *J* = 1.4 Hz, 1 H), 8.02 (s, 1 H), 4.85 (d, *J* = 13.7 Hz, 1 H), 4.81 (d, *J* = 13.7 Hz, 1 H), 4.70 (dt, *J* = 12.1, 2.6 Hz, 1 H), 4.49 (br d, *J* = 12.0 Hz, 1 H), 4.29-4.38 (m, 2 H), 4.25 (dd, *J* = 13.5, 3.3 Hz, 1 H). Anal. ($C_{11}H_{10}ClN_5O_4$) C, H, N.

A stirred mixture of chloride **47** (0.100 g, 0.32 mmol) and 4-(trifluoromethoxy)phenylboronic acid **(44)** (0.080 g, 0.39 mmol) in aqueous $K_2CO_3$ (1 mL, 2M), EtOH (3 mL) and toluene (5 mL) was purged with $N_2$ for 5 min. $Pd(dppf)Cl_2$ (5 mg, 6.25 $\mu$mol) was added and the mixture was refluxed under $N_2$ for 0.5 h. The solution was partitioned between EtOAc and water, and the organic layer was dried ($MgSO_4$) and concentrated under reduced pressure. The residue was chromatographed on silica gel, initially eluting with EtOAc to remove foreruns, and then elution with EtOAc:MeOH (95:5) gave **4** (0.115 g, 82%) as a white solid: mp 182-184 °C; $^1H$ NMR [$(CD_3)_2SO$] $\delta$ 9.23 (d, *J* = 1.4 Hz, 1 H), 8.72 (d, *J* = 1.4 Hz, 1 H), 8.25 (d, *J* = 8.9 Hz, 2 H), 8.03 (s, 1 H), 7.53 (d, *J* = 8.9 Hz, 2 H), 4.89 (d, *J* = 13.3 Hz, 1 H), 4.85 (d, *J* = 13.3 Hz, 1 H), 4.74 (dt, *J* = 12.0, 2.6 Hz, 1 H), 4.52 (br d, *J* = 11.9 Hz, 1 H), 4.33-4.43 (m, 2 H), 4.27 (dd, *J* = 13.5, 3.2 Hz, 1 H). Anal. ($C_{18}H_{14}F_3N_5O_5$) C, H, N.

**E. Synthesis of (6*S*)-2-nitro-6-({6-[4-(trifluoromethoxy)phenyl]-3-pyridinyl}methoxy)-6,7-dihydro-5*H*-imidazo[2,1-*b*][1,3]oxazine (6) by the method of Scheme 4.**

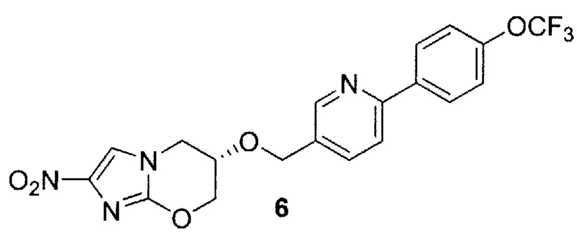

6

NaH (60% w/w, 0.584 g, 14.6 mmol) was added to a solution of oxazine alcohol **41** (2.073 g, 11.2 mmol) and 2-chloro-5-(chloromethyl)pyridine (48) (2.0 g, 12.3 mmol) in anhydrous DMF (40 mL) at 5 °C. The resulting mixture was stirred at room temperature for 16 h and then quenched with water (150 mL). The precipitate was filtered off, washed with water and dried to give (6*S*)-6-[(6-chloro-3-pyridinyl)methoxy]-2-nitro-6,7-dihydro-5*H*-imidazo[2,1-*b*][1,3]oxazine (49) (3.39 g, 97%) as a light yellow solid: mp 191-193 °C; $^1H$ NMR [$(CD_3)_2SO$] $\delta$ 8.37 (d, *J* = 2.3 Hz, 1 H), 8.02 (s, 1 H), 7.79 (dd, *J* = 8.3, 2.4 Hz, 1 H), 7.51 (br d, *J* = 8.2 Hz, 1 H), 4.74 (d, *J* = *12.4* Hz, 1 H), 4.69-4.64 (m, 2 H), 4.47 (d, *J*= 11.8 Hz, 1 H), 4.29-4.21 (m, 3 H). HRESIMS calcd for $C_{12}H_{12}ClN_4O_4$ *m/z* $[M + H]^+$ 313.0513, 311.0542, found 313.0518, 311.0545.

Chloride **49** (1.0 g, 3.22 mmol) and 4-(trifluoromethoxy)phenylboronic acid **(44)** (0.788 g, 3.82 mmol) were suspended in DME (50 mL) and an aqueous solution of $K_2CO_3$ (2M, 10 mL) was added. The mixture was purged with $N_2$ and then treated with Pd(dppf)$Cl_2$ (50 mg, 0.068 mmol) and stirred at 85 °C in an $N_2$ atmosphere for 1 day, monitoring by MS. Further **44** (0.150 g, 0.728 mmol) was added and the mixture was stirred at 85 °C in an $N_2$ atmosphere for 1 day. The resulting mixture was diluted with water (50 mL), and extracted with EtOAc (3 x 100 mL). The dried ($MgSO_4$) organic layers were adsorbed onto silica gel and chromatographed on silica gel, eluting with EtOAc. Trituration of the product in $Et_2O$ gave 6 (0.942 g, 67%) as a white powder: mp 217-219 °C; $^1$H NMR [$(CD_3)_2SO$] δ 8.63 *(d, J* = 1.7 Hz, 1 H), 8.20 (dt, *J* = 8.9, 2.1 Hz, 2 H), 8.03 (s, 1 H), 7.99 (dd, *J* = 8.2, 0.5 Hz, 1 H), 7.84 (dd, *J* = 8.2, 2.2 Hz, 1 H), 7.47 (dd, J = 8.8, 0.8 Hz, 2 H), 4.77 *(d, J* = 12.3 Hz, 1 H), 4.71-4.68 (m, 2 H), 4.49 (d, *J* = 11.7 Hz, 1 H), 4.31-4.26 (m, 3 H). Anal. ($C_{19}H_{15}F_3N_4O_5$) C, H, N. HPLC purity: 98.9%.

**F. Synthesis of (6*S*)-6-{[6-(4-fluorophenyl)-3-pyridinyl]methoxy}-2-nitro-6,7-dihydro-5*H*-imidazo[2,1-*b*][1,3]oxazine (5) by the method of Scheme 4.**

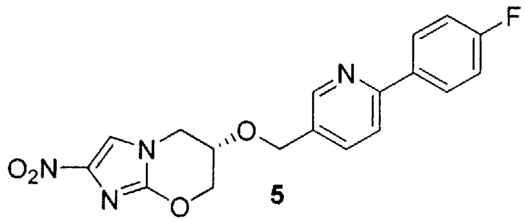

**5**

A solution of (6-bromo-3-pyridinyl)methanol **(50)** (2.503 g, 13.3 mmol) and triphenylphosphine (4.026 g, 15.4 mmol) in anhydrous $CH_2Cl_2$ (100 mL) was carefully treated with recrystallized N-bromosuccinimide (2.732 g, 15.4 mmol) (water bath cooling), and the mixture was stirred at room temperature for 3.5 h. The resulting solution was concentrated, and then added to excess petroleum ether at the top of a silica gel column (100 g in petroleum ether), rinsing on with minimal extra $CH_2Cl_2$. Elution with petroleum ether firstly gave foreruns, and then further elution with 15-25% $Et_2O$/pentane gave pure 2-bromo-5-(bromomethyl)pyridine (51) (Schubert et al., 1999) (3.045 g, 91%) as a lachrymatory white solid that was used directly in the next step; $^1$H NMR ($CDCl_3$) δ 8.38 (d, *J* = 2.5 Hz, 1 H), 7.59 (dd, *J* = 8.2, 2.6 Hz, 1 H), 7.48 (d, *J* = 8.2 Hz, 1 H), 4.42 (s, 2 H).

A solution of oxazine alcohol **41** (2.224 g, 12.0 mmol) and bromide **51** (3.045 g, 12.1 mmol) in anhydrous DMF (46 mL) under $N_2$ at 0 °C was treated with 60% NaH (639 mg, 16.0 mmol) then quickly degassed and resealed under $N_2$. After stirring at room temperature for 2.5 h, the reaction was cooled ($CO_2$/acetone), quenched with ice/aqueous $NaHCO_3$ (50 mL), added to brine (250 mL) and extracted with $CH_2Cl_2$ (12x 200 mL). The combined extracts were evaporated to dryness and the residue was chromatographed on silica gel. Elution with 0-1% MeOH/$CH_2Cl_2$ firstly gave foreruns, and then further elution with 1-1.5% MeOH/$CH_2Cl_2$ gave (6*S*)-6-[(6-bromo-3-pyridinyl)methoxy]-2-nitro-6,7-dihydro-5*H*-imidazo[2,1-*b*][1,3]oxazine (52) (3.739 g, 88%) as a cream solid: mp (MeOH/$CH_2Cl_2$/hexane) 200-203 °C; $^1$H NMR [$(CD_3)_2SO$] δ 8.35 (dd, *J* = 2.3, 0.4 Hz, 1 H), 8.02 (s, 1 H), 7.69 (dd, *J* = 8.2, 2.5 Hz, 1 H), 7.63 (dd, *J* = 8.1, 0.5 Hz, 1 H), 4.72-4.62 (m, 3 H), 4.47 (br d, *J* = 11.8 Hz, 1 H), 4.31-4.19 (m, 3 H). Anal. ($C_{12}H_{11}BrN_4O_4$) C, H, N. HPLC purity: 100%.

Bromide **52** (0.100 g, 0.28 mmol) and 4-fluorophenylboronic acid (57) (69 mg, 0.49 mmol) were suspended in toluene/EtOH (5 mL / 2 mL) and an aqueous solution of $K_2CO_3$ (2M, 1 mL) was added. The stirred mixture was purged with $N_2$ and then treated with Pd(dppf)$Cl_2$ (5 mg, 6.83 μmol) and heated under reflux in an $N_2$ atmosphere for 20 min. The resulting mixture was diluted with water (10 mL) and extracted with EtOAc (3x 15 mL). The dried ($MgSO_4$) organic layers were adsorbed onto silica gel and chromatographed on silica gel, eluting with EtOAc. Trituration of the product in $Et_2O$ gave 5 (90 mg, 86%): mp 194-196 °C; $^1$H NMR [$(CD_3)_2SO$] δ 8.60 (d, *J* = 1.7 Hz, 1 H), 8.14-8.10 (m, 2 H), 8.03 (s, 1 H), 7.95 (dd, *J* = 8.2, 0.6 Hz, 1 H), 7.81 (dd, *J* = 8.2, 2.3 Hz, 1 H), 7.31 (br t, *J* = 8.9 Hz, 2 H), 4.75 (d, *J* = 12.2 Hz, 1 H), 4.71-4.68 (m, 2 H), 4.49 (d, *J* = 11.7 Hz, 1 H), 4.31-4.26 (m, 3 H). Anal. ($C_{18}H_{15}FN_4O_4 \cdot 1.5H_2O$) C, N, F. H: calcd, 4.57; found, 3.87. HPLC purity: 99.4%.

**G. Synthesis of (6S)-6-({6-[3-fluoro-4-(trifluoromethoxy)phenyl]-3-pyridinyl}methoxy)-2-nitro-6,7-dihydro-5H-imidazo[2,1-b][1,3]oxazine (21) by the method of Scheme 4.**

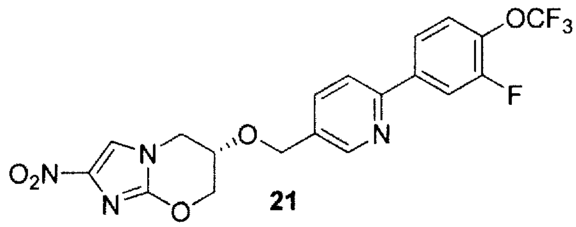

21

A stirred mixture of bromide **52** (see Example 2F) (502 mg, 1.41 mmol), 3-fluoro-4-(trifluoromethoxy)phenylboronic acid **(56)** (450 mg, 2.01 mmol) and Pd(dppf)$Cl_2$ (130 mg, 0.178 mmol) in toluene (20 mL) and EtOH (10 mL) was degassed for 12 min (vacuum pump) and then $N_2$ was added. An aqueous solution of 2M $Na_2CO_3$ (3.8 mL, 7.6 mmol) was added by syringe and the stirred mixture was again degassed for 15 min, and then $N_2$ was added. The resulting mixture was stirred at 90 °C for 2 h, and then cooled, diluted with aqueous $NaHCO_3$ (100 mL) and extracted with $CH_2Cl_2$ (6x 100 mL). The extracts were evaporated to dryness and the residue was chromatographed on silica gel. Elution with 0-0.5% MeOH/$CH_2Cl_2$ firstly gave foreruns, and then further elution with 0.5% MeOH/$CH_2Cl_2$ gave **21** (573 mg, 89%) as a cream solid: mp ($CH_2Cl_2$/pentane) 187-189 °C; $^1$H NMR ($CDCl_3$) $\delta$ 8.62 (d, *J* = 1.5 Hz, 1 H), 7.90 (dd, *J* = 11.3, 2.1 Hz, 1 H), 7.78 (ddd, *J* = 8.6, 2.0, 1.3 Hz, 1 H), 7.75 (dd, *J* = 8.2, 2.2 Hz, 1 H), 7.71 (dd, *J* = 8.2, 0.8 Hz, 1 H), 7.41 (s, 1 H), 7.40 (ddq, *J* = 8.7, 7.6, 1.2 Hz, 1 H), 4.80 (d, *J* = 12.0 Hz, 1 H), 4.70 (d, *J* = 11.8 Hz, 1 H), 4.68 (ddd, *J* = 12.2, 3.5, 2.3 Hz, 1 H), 4.40 (dd, *J* = 12.2, 1.1 Hz, 1 H), 4.25 (dd, *J* = 13.3, 4.5 Hz, 1 H), 4.22-4.15 (m, 2 H). Anal. ($C_{19}H_{14}F_4N_4O_5$) C, H, N.

**H. Synthesis of (6*S*)-6-({6-[3-chloro-4-(trifluoromethoxy)phenyl]-3-pyridinyl}methoxy)-2-nitro-6,7-dihydro-5*H*-imidazo[2,1-*b*][1,3]oxazine (28) by the method of Scheme 4.**

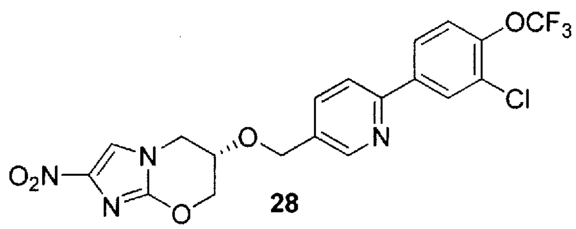

28

An ice-cold mixture of 98% $H_2SO_4$ (0.75 mL) and water (2.25 mL) was added to 3-chloro-4-(trifluoromethoxy)aniline **(53)** (1.00 g, 4.73 mmol) and the resulting salt was crushed (using a glass rod) and cooled in an ice bath. A solution of $NaNO_2$ (359 mg, 5.20 mmol) in cold water (0.75 mL, then 0.25 mL) was added drop-wise, and the mixture was stirred at 0 °C for 12 min. A solution of urea (42.6 mg, 0.709 mmol) in cold water (0.25 mL) was added, and the mixture was stirred at 0 °C for 3 min. Finally, a solution of KI (1.65 g, 9.94 mmol) in cold water (1.6 mL, then 0.2 mL) was added slowly, and the mixture was stirred at room temperature for 10 min, and then at 52 °C for 2 h. The resulting cooled mixture was diluted with ice-water (45 mL) and extracted with $CH_2Cl_2$ (4x 50 mL). The extracts were sequentially washed with an aqueous solution of $Na_2SO_3$ (30 mL of 0.5%) and then with water (40 mL) and finally concentrated carefully under reduced pressure at 17 °C. The resulting oil was chromatographed on silica gel, eluting with pentane, to give 2-chloro-4-iodo-1-(trifluoromethoxy)benzene (54) (1.24 g, 81%) as a colourless oil (a white solid on freezing); $^1$H NMR ($CDCl_3$) $\delta$ 7.82 (d, *J* = 2.1 Hz, 1 H), 7.61 (dd, *J* = 8.6, 2.1 Hz, 1 H), 7.05 (dq, *J* = 8.6, 2.0 Hz, 1 H); HRAPCIMS calcd for $C_7H_3ClF_3IO$ *m/z* ($M^+$) 323.8834, 321.8864, found 323.8834, 321.8861.

Triisopropylborate (0.76 mL, 3.29 mmol) and iodide 54 (815 mg, 2.53 mmol) were successively added via syringe to a mixture of anhydrous toluene (4 mL) and anhydrous distilled THF (1 mL) under $N_2$ and the mixture was cooled to -78 °C. n-Butyllithium (1.08 mL of a 2.5 M solution in hexanes, 2.70 mmol) was added drop-wise over 75 min to the stirred solution (at -78 °C), and the mixture was stirred at -78 °C for an additional 3 h, and then slowly warmed to -20 °C (over 1.5 h). 2N HCl (2.6 mL) was added and the mixture was stirred at room temperature for 30 min, and then diluted with water (40 mL) and extracted with EtOAc (5x 50 mL). The extracts were washed with brine (50 mL) and then evaporated to dryness. The residue was triturated in pentane (~3-4 mL), cooled to -78 °C, and rapidly filtered cold (washing with pentane cooled to -78 °C) to give 3-chloro-4-(trifluoromethoxy)phenylboronic acid (55) (459 mg, 76%) as a white solid (a 1:1 mixture of the trimeric boroxine and the boronic acid by NMR): mp 202-204 °C; $^1$H NMR ($CDCl_3$) $\delta$ 8.26 (d, *J* = 1.5 Hz, 3 H, boroxine), 8.12 (dd, *J* = 8.2, 1.5 Hz, 3 H, boroxine), 7.85 (d, *J* = 1.5 Hz, 1 H, boronic acid), 7.65

(dd, *J* = 8.2, 1.6 Hz, 1 H, boronic acid), 7.48 (dq, *J* = 8.2, 1.5 Hz, 3 H, boroxine), 7.35 (dq, *J* = 8.2, 1.5 Hz, 1 H, boronic acid), 4.57 (s, 2 H, boronic acid).

Suzuki coupling of bromide **52** and boronic acid **55** as in Example 2G, followed by chromatography of the product on silica gel, eluting with 0-0.5% MeOH/$CH_2Cl_2$ (foreruns) and then 0.5% MeOH/$CH_2Cl_2$ gave **28** (90%) as a cream solid: mp ($CH_2Cl_2$/pentane) 169-171 °C; $^1$H NMR ($CDCl_3$) $\delta$ 8.63 (br d, *J* = 1.3 Hz, 1 H), 8.15 (d, *J* = 2.2 Hz, 1 H), 7.91 (dd, *J* = 8.6, 2.2 Hz, 1 H), 7.75 (dd, *J* = 8.2, 2.1 Hz, 1 H), 7.71 (dd, *J* = 8.2, 0.8 Hz, 1 H), 7.44-7.39 (m, 2 H), 4.80 (d, *J* = 12.0 Hz, 1 H), 4.70 (d, *J* = 11.8 Hz, 1 H), 4.68 (ddd, *J* = 12.3, 3.5, 2.2 Hz, 1 H), 4.39 (dd, *J* = 12.2, 1.2 Hz, 1 H), 4.25 (dd, *J* = 13.3, 4.5 Hz, 1 H), 4.22-4.15 (m, 2 H). Anal. ($C_{19}H_{14}ClF_3N_4O_5$) C, H, N.

**I. Synthesis of (6S)-2-nitro-6-({5-[4-(trifluoromethoxy)phenyl]-2-pyridinyl}methoxy)-6,7-dihydro-5H-imidazo[2,1-b][1,3]oxazine (7) by the method of Scheme 5.**

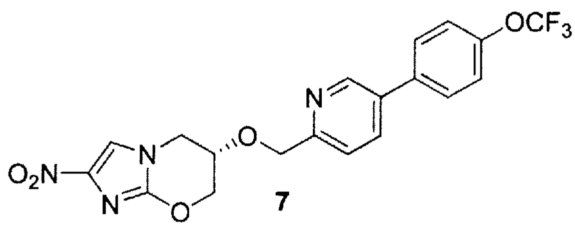

**7**

NaH (0.525 g, 13.1 mmol, 60% in mineral oil) was added to a solution of alcohol **41** (1.872, 10.1 mmol) and 5-bromo-2-(chloromethyl)pyridine (58) (prepared by chlorination of (5-bromo-2-pyridinyl)methanol, as reported by van den Heuvel et al., 2004) (2.5 g, 12.1 mmol) in anhydrous DMF (40 mL) at 5 °C. The resulting mixture was stirred at room temperature for 2 h and then quenched with water (300 mL). The precipitate was filtered off, washed with water and dried to give (6*S*)-6-[(5-bromo-2-pyridinyl)methoxy]-2-nitro-6,7-dihydro-5*H*-imidazo[2,1-*b*][1,3]oxazine (59) (3.087 g, 86%) as a light brown solid: mp 171-173 °C; $^1$H NMR [$(CD_3)_2SO$] $\delta$ 8.65 (dd, *J* = 2.3, 0.4 Hz, 1 H), 8.04 (dd, *J* = 8.4, 2.4 Hz, 1 H), 8.02 (s, 1 H), 7.35 (dd, *J* = 8.4, 0.4 Hz, 1 H), 4.72-4.66 (m, 3 H), 4.49 (br d, *J* = 12.0 Hz, 1 H), 4.35-4.21 (m, 3 H). Anal. ($C_{12}H_{11}BrN_4O_4$) C, H, N. HPLC purity: 99.4%.

Bromide **59** (0.100 g, 0.28 mmol) and 4-(trifluoromethoxy)phenylboronic acid **(44)** (0.075 g, 0.366 mmol) were suspended in toluene/EtOH (5 mL / 2 mL) and an aqueous solution of $K_2CO_3$ (1 mL; 2M) was added. The stirred mixture was purged with $N_2$ and then treated with Pd(dppf)$Cl_2$ (5 mg, 6.83 $\mu$mol) and heated under reflux in an $N_2$ atmosphere for 30 min. The resulting mixture was diluted with water (10 mL) and extracted with EtOAc (3x 15 mL). The dried ($MgSO_4$) organic layers were adsorbed onto silica gel and chromatographed on silica gel, eluting with 5% MeOH/EtOAc. Trituration of the product in $Et_2O$ gave 7 (97 mg, 79%): mp 157-159 °C; $^1$H NMR [$(CD_3)_2SO$] $\delta$ 8.85 (d, *J* = 2.0 Hz, 1 H), 8.11 (dd, *J* = 8.1, 2.4 Hz, 1 H), 8.03 (s, 1 H), 7.85 (dt, *J* = 8.8, 2.0 Hz, 2 H), 7.48 (t, *J* = 7.7 Hz, 3 H), 4.82 (d, *J* = 13.2 Hz, 1 H), 4.78 (d, *J* = 13.2 Hz, 1 H), 4.72 (dt, *J* = 12.0, 2.6 Hz, 1 H), 4.51 (d, *J* = 12.0 Hz, 1 H), 4.37-4.24 (m, 3 H). Anal. ($C_{19}H_{15}F_3N_4O_5$) C, H, N, F. HPLC purity: 100%.

**J. Synthesis of (6*S*)-2-nitro-6-({4-[5-(trifluoromethyl)-2-pyridinyl]benzyl}oxy)-6,7-dihydro-5*H*-imidazo[2,1-*b*][1,3]oxazine (8) by the method of Scheme 6.**

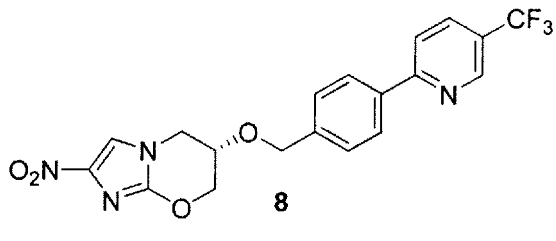

**8**

Reaction of oxazine alcohol **41** (5.00 g, 27.0 mmol) with 4-bromobenzyl bromide (60) (7.62 g, 30.5 mmol) and NaH (60% w/w, 1.40 g, 35.0 mmol) in DMF (100 mL) for 2 h at room temperature gave (6*S*)-6-[(4-bromobenzyl)oxy]-2-nitro-6,7-dihydro-5*H*-imidazo[2,1-*b*][1,3]oxazine (61) (8.368 g, 88%) as a light yellow solid: mp ($Et_2O$) 188-190 °C; $^1$H

NMR [$(CD_3)_2SO$] δ 8.01 (s, 1 H), 7.54 (dt, *J* = 8.4, 2.2 Hz, 2 H), 7.13 (dt, *J* = 8.5, 2.2 Hz, 2 H), 4.67-4.62 (m, 2 H), 4.61 (d, *J* = 12.2 Hz, 1 H), 4.46 (d, *J* = 12.0 Hz, 1 H), 4.28-4.19 (m, 3 H). Anal. ($C_{13}H_{12}BrN_3O_4$) C, H, N.

A mixture of bromide **61** (2.00 g, 5.65 mmol), bis(pinacolato)diboron (1.59 g, 6.29 mmol) and KOAc (3.40 g, 34.7 mmol) in DMSO (40 mL) was purged with $N_2$. Pd(dppf)$Cl_2$ (0.14 g, 0.17 mmol) was added and the mixture was purged with $N_2$ through the solution while heating to 90 °C. After 1 h the reaction was partitioned between EtOAc and water, and the organic extract was purified by chromatography on silica gel, eluting with EtOAc. The product was triturated in $Et_2O$ to give (6*S*)-2-nitro-6-{[4-(4,4,5,5-tetramethyl-1,3,2-dioxaborolan-2-yl)benzyl]oxy}-6,7-dihydro-5*H*-imidazo[2,1-*b*][1,3]oxazine **(62)** (1.158 g, 51%): mp 150-153 °C; $^1H$ NMR [$(CD_3)_2SO$] δ 8.01 (s, 1 H), 7.65 (d, *J* = 8.0 Hz, 2 H), 7.32 (d, *J* = 8.0 Hz, 2 H), 4.70 (d, *J* = 12.5 Hz, 1 H), 4.67-4.63 (m, 2 H), 4.46 (d, *J* = 11.9 Hz, 1 H), 4.29-4.20 (m, 3 H), 1.29 (s, 12 H). Anal. ($C_{19}H_{24}BN_3O_6$) C, H, N.

A mixture of boronate ester **62** (0.094 g, 0.23 mmol) and 2-chloro-5-(trifluoromethyl)pyridine (53 mg, 0.29 mmol) in toluene (5 mL), EtOH (3 mL) and aqueous $K_2CO_3$ (2M, 1 mL, 2 mmol) was purged with $N_2$. Pd(dppf)$Cl_2$ (8 mg, 0.01 mmol) was added and the mixture was refluxed under $N_2$ for 0.5 h, then partitioned between EtOAc and water. The organic layer was dried and evaporated, and then column chromatography on silica gel using gradient elution (1:1 hexanes:EtOAc then EtOAc) gave **8** (60 mg, 62%) as a white solid: mp ($Et_2O$ triturate) 252-254 °C; $^1H$ NMR [$(CD_3)_2SO$] δ 9.03 (br s, 1 H), 8.27 (dd, *J*= 8.5, 2.1 Hz, 1 H), 8.18 (d, *J* = 8.4 Hz, 1 H), 8.15 (d, *J* = 8.3 Hz, 2 H), 8.03 (s, 1 H), 7.48 (d, *J* = 8.3 Hz, 2 H), 4.66-4.78 (m, 3 H), 4.49 (d, *J*= 11.8 Hz, 1 H), 4.23-4.33 (m, 3 H). Anal. ($C_{19}H_{15}F_3N_4O_4$) C, H, N.

**K. Synthesis of (6*S*)-2-nitro-6-({4-[6-(trifluoromethyl)-3-pyridinyl]benzyl}oxy)-6,7-dihydro-5*H*-imidazo[2,1-*b*][1,3]oxazine (9) by the method of Scheme 6.**

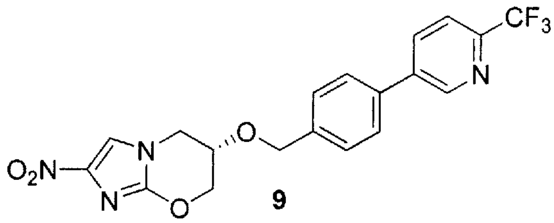

**9**

Reaction of boronate ester **62** (see Example 2J) (0.157 g, 0.391 mmol) and 5-bromo-2-(trifluoromethyl)pyridine (0.110 g, 0.487 mmol) as in Example 2J gave 9 (0.105 g, 64%) as a white solid: mp ($Et_2O$ triturate) 221-222 °C; $^1H$ NMR [$(CD_3)_2SO$] δ 9.08 (d, *J*= 2.1 Hz, 1 H), 8.35 (dd, J = 8.1, 1.9 Hz, 1 H), 8.03 (s, 1 H), 7.97 (d, *J* = 8.1 Hz, 1 H), 7.81 (d, *J* = 8.3 Hz, 2 H), 7.48 (d, *J* = 8.3 Hz, 2 H), 4.67-4.77 (m, 3 H), 4.49 (d, *J* = 11.8 Hz, 1 H), 4.22-4.33 (m, 3 H). Anal. ($C_{19}H_{15}F_3N_4O_4$) C, H, N.

**L. Synthesis of (6*S*)-2-nitro-6-({1-[4-(trifluoromethoxy)phenyl]-1*H*-pyrazol-3-yl}methoxy)-6,7-dihydro-5*H*-imidazo[2,1-A][1,3]oxazine (10) by the method of Scheme 7.**

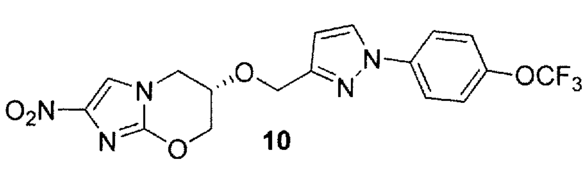

**10**

4-Trifluoromethoxybenzenediazonium tetrafluoroborate **(63)** (4.33 g, 15.7 mmol) was added to a solution of ethyl 2-chloroacetoacetate **(64)** (2.35 g, 14.3 mmol) in pyridine (6 mL) and water (6 mL) at -5 °C. The mixture was stirred at -5 °C for 0.5 h and the precipitate was filtered and washed with ice cold water. Recrystallisation from EtOH/water gave ethyl 2-chloro{[4-(trifluoromethoxy)phenyl]hydrazono}ethanoate (65) (3.977 g, 82%) as pale orange needles: mp 128-130 °C; $^1H$ NMR [$(CD_3)_2SO$] δ 10.68 (s, 1 H), 7.43 (d, *J* = 9.2 Hz, 2 H), 7.34 (d, *J* = 9.2 Hz, 2 H), 4.30 (t, *J* = 7.1 Hz, 2 H), 1.30 (q, *J* = 7.1 Hz, 3 H). APCI MS *m*/*z* 309, 311 [M - H]$^-$.

A stirred mixture of hydrazonoyl chloride 65 (1.55 g, 4.99 mmol), bicyclo[2.2.1]hepta-2,5-diene (1.25 mL, 24.6 mmol) and $Et_3N$ (2.0 mL, 14.3 mmol) in toluene (10 mL) was heated to 70 °C for 1 h. The mixture was cooled and filtered, the filter cake was washed with toluene (10 mL) and the organic fractions were combined and evaporated. The residue

was refluxed in xylenes (30 mL) for 2 h. Column chromatography on silica gel, eluting with hexanes, firstly gave xylenes, and then further elution with $CH_2Cl_2$ gave ethyl 1-[4-(trifluoromethoxy)phenyl]-1*H*-pyrazole-3-carboxylate **(66)** (1.176 g, 79%) as a white solid: mp 76-78 °C; $^1$H NMR ($CDCl_3$) δ 7.91 (d, *J* = 2.5 Hz, 1 H), 7.79 (d, *J* = 8.9 Hz, 2 H), 7.33 (d, *J* = 8.9 Hz, 2 H), 7.00 (d, *J* = 2.5 Hz, 1 H), 4.44 (q, *J* = 7.1 Hz, 2 H), 1.43 (t, *J* = 7.1 Hz, 3 H). APCI MS *m*/*z* 301 [M + H]$^+$.

$LiAlH_4$ (0.137 g, 3.61 mmol) was added to a solution of ester **66** (1.081 g, 3.60 mmol) in $Et_2O$ (20 mL) at 0 °C and the stirred mixture was warmed to room temperature for 1 h, then cooled to 0 °C and quenched with ice. The mixture was diluted with $Et_2O$ (100 mL) and saturated aqueous sodium potassium tartrate (100 mL) and then filtered through Celite. The organic layer was dried and chromatographed on silica gel, eluting with $CH_2Cl_2$:EtOAc (95:5), to give {1-[4-(trifluoromethoxy)phenyl]-1*H*-pyrazol-3-yl}methanol (67) (0.888 g, 96%) as a white solid: mp 53-54 °C; $^1$H NMR [$(CD_3)_2SO$] δ 8.44 (d, *J* = 2.5 Hz, 1 H), 7.92 (d, *J* = 8.5 Hz, 2 H), 7.48 (d, *J* = 8.5 Hz, 2 H), 6.50 (d, *J* = 2.5 Hz, 1 H), 5.15 (t, *J* = 5.8 Hz, 1 H), 4.51 (d, *J* = 5.8 Hz, 2 H). APCI MS *m*/*z* 259 [M + H]$^+$.

$PBr_3$ (0.312 mL, 3.32 mmol) was added to a solution of alcohol **67** (0.858 g, 3.32 mmol) in ether (15 mL) at 0 °C. The mixture was stirred at room temperature for 17 h, then cooled to 0 °C, quenched with ice, and partitioned between $CH_2Cl_2$ and water. Column chromatography of the organic portion on silica gel (eluting with $CH_2Cl_2$) gave 3-(bromomethyl)-1-[4-(trifluoromethoxy)phenyl]-1*H*-pyrazole **(68)** (0.952 g, 89%) as a white solid: mp 71-73 °C; $^1$H NMR ($CDCl_3$) δ 7.84 (d, *J* = 2.5 Hz, 1 H), 7.69 *(d, J* = 9.1 Hz, 2 H), 7.31 (d, *J* = 9.1 Hz, 2 H), 6.54 (d, *J* = 2.5 Hz, 1 H), 4.56 (s, 2 H). APCI MS *m*/*z* 321, 323 [M + H]$^+$.

NaH (60% w/w, 160 mg, 4.00 mmol) was added to a solution of oxazine alcohol **41** (0.473 g, 2.55 mmol) and bromide **68** (0.913 g, 2.84 mmol) in DMF (50 mL) at 0 °C. The mixture was stirred at 0 °C for 2 h and then quenched with ice and partitioned between EtOAc and water. The organic fraction was dried and evaporated, and then column chromatography on silica gel, eluting with a gradient of 1:1 hexanes:EtOAc to EtOAc, gave 10 (0.844 g, 78%) as a white solid: mp 103-105 °C; $^1$H NMR [$(CD_3)_2SO$] δ 8.50 (d, *J* = 2.5 Hz, 1 H), 8.01 (s, 1 H), 7.93 (d, *J* = 9.1 Hz, 2 H), 7.49 (d, *J* = 9.1 Hz, 2 H), 6.54 (d, *J* = 2.5 Hz, 1 H), 4.68-4.74 (m, 2 H), 4.65 (dt, *J* = 12.3, 2.4 Hz, 1 H), 4.47 (d, *J* = 11.8 Hz, 1 H), 4.20-4.31 (m, 3 H). Anal. ($C_{17}H_{14}F_3N_5O_5$) C, H, N.

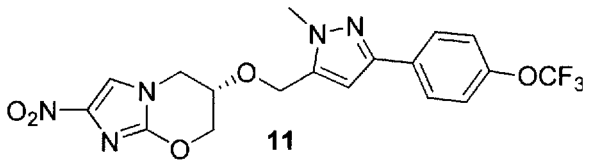

11

**N. Synthesis of (6*S*)-6-{[3-fluoro-4-(trifluoromethoxy)[1,1'-biphenyl]-4-yl)methoxy}-2-nitro-6,7-dihydro-5*H*-imidazo[2,1-b][1,3]oxazine (12) by the method of Scheme 9.**

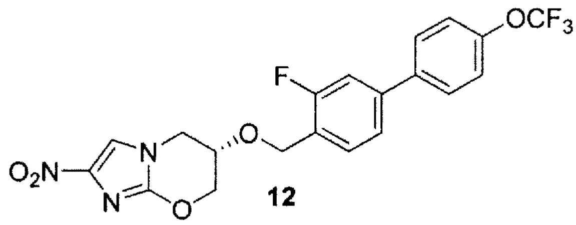

12

A solution of alcohol **41** (1.403 g, 7.58 mmol) and 4-bromo-1-(bromomethyl)-2-fluorobenzene **(74)** (2.66 g, 9.93 mmol) in anhydrous DMF (30 mL) under $N_2$ at 0 °C was treated with 60% NaH (427 mg, 10.7 mmol), then quickly degassed and resealed under $N_2$. After stirring at room temperature for 3 h, the reaction was cooled ($CO_2$/acetone), quenched with ice/aqueous $NaHCO_3$ (20 mL), added to brine (150 mL) and extracted with $CH_2Cl_2$ (4x 80 mL). The combined extracts were evaporated to dryness and the residue was chromatographed on silica gel. Elution with 0-2% EtOAc/$CH_2Cl_2$ firstly gave foreruns, and then elution with 3-5% EtOAc/$CH_2Cl_2$ gave (6*S*)-6-[(4-bromo-2-fluorobenzyl)oxy]-2-nitro-6,7-dihydro-5*H*-imidazo[2,1-*b*][1,3]oxazine **(75)** (2.633 g, 93%) as a pale yellow solid: mp (MeOH/$CH_2Cl_2$/hexane) 171-173 °C; $^1$H NMR [$(CD_3)_2SO$] δ 8.01 (s, 1 H), 7.54 (dd, *J* = 9.7, 1.8 Hz, 1 H), 7.42 (dd, *J* = 8.2, 1.8 Hz, 1 H), 7.37 (dd, *J* = 8.1, 7.7 Hz, 1 H), 4.72-4.62 (m, 3 H), 4.47 (br d, *J* = 11.9 Hz, 1 H), 4.30-4.19 (m, 3 H). Anal. ($C_{13}H_{11}BrFN_3O_4$) C, H, N.

A stirred mixture of bromide **75** (475 mg, 1.28 mmol), 4-(trifluoromethoxy)phenylboronic acid **(44)** (395 mg, 1.92 mmol) and $Pd(dppf)Cl_2$ (143 mg, 0.195 mmol) in toluene (18 mL) and EtOH (7 mL) was degassed for 8 min (vacuum

pump) and then $N_2$ was added. An aqueous solution of 2M $Na_2CO_3$ (3.5 mL, 7.0 mmol) was added by syringe and the stirred mixture was again degassed for 8 min, and then $N_2$ was added. The resulting mixture was stirred at 85 °C for 70 min, and then cooled, diluted with aqueous $NaHCO_3$ (50 mL) and extracted with $CH_2Cl_2$ (6x 50 mL). The extracts were evaporated to dryness and the residue was chromatographed on silica gel. Elution with 0-1% EtOAc/$CH_2Cl_2$ firstly gave foreruns, and then further elution with 1-2% EtOAc/$CH_2Cl_2$ gave **12** (539 mg, 93%) as a pale yellow solid: mp ($CH_2Cl_2$/pentane) 160-162 °C; $^1$H NMR ($CDCl_3$) δ 7.57 (dt, *J* = 8.8, 2.5 Hz, 2 H), 7.42 (t, *J* = 7.7 Hz, 1 H), 7.39 (s, 1 H), 7.35 (dd, *J* = 7.9, 1.7 Hz, 1 H), 7.33-7.23 (m, 3 H), 4.81-4.73 (m, 2 H), 4.65 (ddd, *J* = 12.2, 3.6, 2.0 Hz, 1 H), 4.38 (br d, *J* = 12.1 Hz, 1 H), 4.25-4.13 (m, 3 H). Anal. ($C_{20}H_{15}F_4N_3O_5$) C, H, N.

**O. Synthesis of (6*S*)-6-({2-fluoro-4-[6-(trifluoromethyl)-3-pyridinyl)benzyl}oxy)-2-nitro-6,7-dihydro-5*H*-imidazo[2,1-*b*][1,3]oxazine (23) by the method of Scheme 9.**

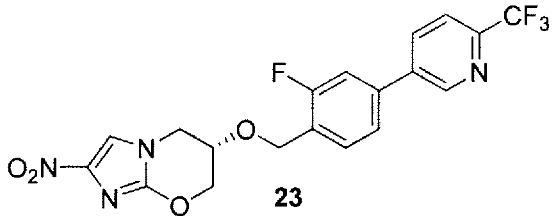

**23**

A stirred mixture of bromide **75** (see Example 2N) (503 mg, 1.35 mmol), 6-(trifluoromethyl)-3-pyridinylboronic acid (386 mg, 2.02 mmol) and Pd(dppf)$Cl_2$ (148 mg, 0.202 mmol) in toluene (20 mL) and EtOH (10 mL) was degassed for 12 min (vacuum pump) and then $N_2$ was added. An aqueous solution of 2M $Na_2CO_3$ (3.5 mL, 7.0 mmol) was added by syringe and the stirred mixture was again degassed for 12 min, and then $N_2$ was added. The resulting mixture was stirred at 90 °C for 3 h, and then cooled, diluted with aqueous $NaHCO_3$ (100 mL) and extracted with $CH_2Cl_2$ (6x 100 mL). The extracts were evaporated to dryness and the residue was chromatographed on silica gel. Elution with 0-3% EtOAc/$CH_2Cl_2$ firstly gave foreruns, and then further elution with 3-4% EtOAc/$CH_2Cl_2$ gave **23** (530 mg, 90%) as a cream solid: mp (MeOH/$CH_2Cl_2$/pentane) 195-198 °C; $^1$H NMR [$(CD_3)_2SO$] δ 9.12 (d, *J* = 2.1 Hz, 1 H), 8.40 (dd, *J* = 8.1, 1.9 Hz, 1 H), 8.03 (s, 1 H), 7.99 (d, *J* = 8.1 Hz, 1 H), 7.75 (dd, *J* = 11.3, 1.7 Hz, 1 H), 7.68 (dd, *J* = 7.9, 1.8 Hz, 1 H), 7.57 (t, *J* = 7.8 Hz, 1 H), 4.80 (br d, *J* = 13.0 Hz, 1 H), 4.76 (br d, *J* = 13.3 Hz, 1 H), 4.69 (dt, *J* = 12.0, 2.5 Hz, 1 H), 4.50 (br d, *J* = 11.7 Hz, 1 H), 4.35-4.22 (m, 3 H). Anal. ($C_{19}H_{14}F_4N_4O_4$) C, H, N.

**P. Synthesis of (6*S*)-6-({2-fluoro-4-[5-(trifluoromethyl)-2-pyridinyl]benzyl}oxy)-2-nitro-6,7-dihydro-5*H*-imidazo[2,1-*b*][1,3]oxazine (14) by the method of Scheme 9.**

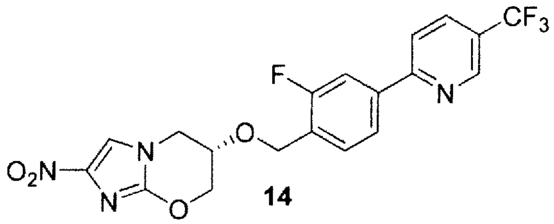

**14**

A stirred mixture of bromide **75** (see Example 2N) (1.601 g, 4.30 mmol), bis(pinacolato)diboron (1.179 g, 4.64 mmol), Pd(dppf)$Cl_2$ (0.473 g, 0.646 mmol) and KOAc (1.497 g, 15.3 mmol) in anhydrous DMSO (24 mL) was degassed for 35 min (vacuum pump) and then $N_2$ was added. The mixture was stirred at 89 °C for 5 h, and then cooled, added to ice-water (150 mL) and extracted with EtOAc (5x 100 mL). The extracts were washed with water (2x 100 mL), evaporated to dryness and the residue was chromatographed on silica gel. Elution with 50% EtOAc/petroleum ether firstly gave foreruns, and then further elution with 50-67% EtOAc/petroleum ether gave (6*S*)-6-{[2-fluoro-4-(4,4,5,5-tetramethyl-1,3,2-dioxaborolan-2-yl)benzyl]oxy}-2-nitro-6,7-dihydro-5*H*-imidazo[2,1-*b*][1,3]oxazine **(76)** (1.186 g, 66%) as a cream solid: mp ($CH_2Cl_2$/$Et_2O$/pentane) 147-149 °C; $^1$H NMR ($CDCl_3$) δ 7.58 (dd, *J* = 7.5, 0.9 Hz, 1 H), 7.49 (br d, *J* = 10.3 Hz, 1 H), 7.38 (s, 1 H), 7.35 (t, *J* = 7.3 Hz, 1 H), 4.76 *(d, J* = 12.9 Hz, 1 H), 4.73 (d, *J* = 12.7 Hz, 1 H), 4.59 (ddd, *J* = 12.1,

3.8, 2.0 Hz, 1 H), 4.34 (dd, *J* = 12.0, 1.5 Hz, 1 H), 4.20-4.07 (m, 3 H), 1.34 (s, 12 H); HRFABMS calcd for $C_{19}H_{23}BFN_3O_6$ *m*/*z* [M + H]$^+$ 420.1742, 419.1779, found 420.1733, 419.1763.

A stirred mixture of boronate ester **76** (602 mg, 1.43 mmol), 2-chloro-5-trifluoromethylpyridine (1.08 g, 5.96 mmol) and Pd(dppf)$Cl_2$ (0.232 g, 0.317 mmol) in toluene (18 mL) and EtOH (9 mL) was degassed for 12 min (vacuum pump) and then $N_2$ was added. An aqueous solution of 2M $Na_2CO_3$ (3.8 mL, 7.6 mmol) was added by syringe and the stirred mixture was again degassed for 12 min, and then $N_2$ was added. The resulting mixture was stirred at 90 °C for 120 min, and then cooled, diluted with aqueous $NaHCO_3$ (100 mL) and extracted with $CH_2Cl_2$ (6x 100 mL). The extracts were evaporated to dryness and the residue was chromatographed on silica gel. Elution with 0-2% EtOAc/$CH_2Cl_2$ firstly gave foreruns, and then further elution with 2-6% EtOAc/$CH_2Cl_2$ gave **14** (523 mg, 83%) as a pale yellow solid: mp ($CH_2Cl_2$/hexane) 233-235 °C; $^1$H NMR ($CDCl_3$) δ 8.95 (m, 1 H), 8.01 (dd, *J* = 8.3, 2.3 Hz, 1 H), 7.86-7.79 (m, 3 H), 7.49 (t, *J* = 7.8 Hz, 1 H), 7.40 (s, 1 H), 4.82 (br d, *J* = 13.1 Hz, I H), 4.78 (br d, *J* = 13.3 Hz, 1 H), 4.66 (ddd, *J* = 12.2, 3.5, 2.0 Hz, 1 H), 4.39 (dd, *J* = 12.1, 1.4 Hz, 1 H), 4.26-4.14 (m, 3 H). Anal. ($C_{19}H_{14}F_4N_4O_4$) C, H, N.

**Q. Synthesis of (6*S*)-2-nitro-6-{[4'-(trifluoromethoxy)[1,1'-biphenyl]-4-yl]oxy}-6,7-dihydro-5*H*-imidazo[2,1-*b*][1,3]oxazine (13) by the method of Scheme 10.**

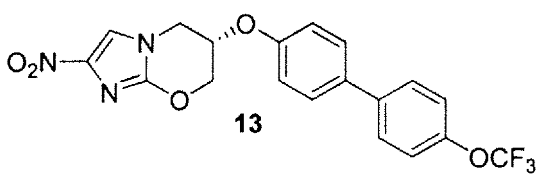

**13**

A mixture of (*S*)-glycidol (77) (20 g, 0.27 mol), benzyl alcohol (27.9 mL, 0.27 mol) and CsF (0.82 g, 5.40 mmol) was heated with stirring at 120 °C for 16 h. Unreacted benzyl alcohol was removed using a rotary evaporator attached to a high vacuum line. The product was partitioned between EtOAc and water, and the organic extract was evaporated and chromatographed on silica. Elution with petroleum ether gave fore fractions, and then further elution with EtOAc/petroleum ether (3:7) gave (2*S*)-3-(benzyloxy)-1,2-propanediol (78) (9.52 g, 19%) as a viscous oil: [α]$^{19}$ -3.64° *(c,* 6.59, $CHCl_3$); $^1$H NMR ($CDCl_3$) δ 7.38-7.28 (m, 5 H), 4.56 (s, 2 H), 3.92-3.87 (m, 1 H), 3.71 (dd, *J* = 11.4, 3.9 Hz, 1 H), 3.64 (dd, *J* = 11.4, 5.4 Hz, 1 H), 3.61-3.57 (m, 2 H), 2.60 (br, 1 H), 2.22 (br, 1 H). APCI MS *m*/*z* 183 [M + H]$^+$.

Chloro(triisopropyl)silane (12.2 mL, 0.057 mol) was added dropwise at 20 °C to a stirred solution of diol **78** (9.52 g, 0.052 mol) and imidazole (5.33 g, 0.078 mol) in DMF (150 mL) and stirring was continued for 16 h. Most of the DMF was removed under reduced pressure and the residue was partitioned between EtOAc and water. The organic extract was washed well with water, then brine, and was evaporated to give an oil, which was chromatographed on silica. Elution with petroleum ether gave fore fractions, and then further elution with EtOAc/petroleum ether (1:19) gave (2*R*)-1-(benzyloxy)-3-[(triisopropylsilyl)oxy]-2-propanol (79) (13.80 g, 78%) as a colourless oil: [α]$^{19}$ -0.78° (c, 8.93, $CHCl_3$); $^1$H NMR ($CDCl_3$) δ 7.41-7.27 (m, 5 H), 4.55 (s, 2 H), 3.90-3.84 (m, 1 H), 3.79-3.72 (m, 2 H), 3.59-3.51 (m, 2 H), 2.52 (d, *J* = 5.1 Hz, 1 H), 1.13-1.03 (m, 21 H). APCI MS *m*/*z* 339 [M+H]$^+$.

1,1'-Diisopropyl azodicarboxylate (7.70 mL, 0.039 mol) was added dropwise at 5 °C to a solution of the alcohol **79** (12.40 g, 0.037 mol), 4'-(trifluoromethoxy)[1,1'-biphenyl]-4-ol (reported by Edsall et al., 2003, via Suzuki coupling of 4-bromophenol and boronic acid 44) (8.29 g, 0.033 mol) and triphenylphosphine (10.26 g, 0.039 mol) in anhydrous benzene (25 mL) and the solution was stirred at 20 °C for 18 h. The product was adsorbed directly onto silica by concentration under reduced pressure, and chromatography on silica gel, eluting with EtOAc/petroleum ether (1:19), gave 4-[((1S)-2-(benzyloxy)-1-{[(triisopropylsilyl)oxy]methyl}ethyl)oxy]-4'-(trifluoromethoxy)-1,1'-biphenyl **(80)** (14.30 g, 69%) as a colourless oil: [α]$^{19}$ +5.9° (c, 6.95, $CHCl_3$); $^1$H NMR ($CDCl_3$) δ 7.72 (d, *J* = 8.8 Hz, 2 H), 7.58 (d, *J* = 8.8 Hz, 2 H), 7.40 (d, *J* = 8.8 Hz, 2 H), 7.35-7.24 (m, 5 H), 7.06 (d, *J* = 8.8 Hz, 2 H), 4.64-4.57 (m, 1 H), 4.52 (s, 2 H), 3.98-3.87 (m, 2 H), 3.76-3.65 (m, 2 H), 1.08-0.98 (m, 21 H). APCI MS *m*/*z* 576 [M + H]$^+$.

A mixture of the benzyl ether **80** (10.79 g, 0.019 mol) and 5% Pd-C (500 mg) in 1:1 EtOAc/EtOH (250 mL) was hydrogenated at 60 psi for 4 h. The catalyst was removed by filtration through Celite and the filtrate was concentrated under reduced pressure to give (2S)-2-{[4'-(trifluoromethoxy)[1,1'-biphenyl]-4-yl]oxy}-3-[(triisopropylsilyl)oxy]-1-propanol **(81)** as a viscous oil, sufficiently pure for use in the next step. Iodine (6.03 g, 0.024 mol) was added in portions at 20 °C to a vigorously stirred solution of the crude alcohol **81,** triphenylphosphine (6.23 g, 0.024 mol) and imidazole (2.49 g, 0.036 mol) in benzene (100 mL) and stirring was continued for 1 h. After dilution with EtOAc the mixture was washed with water, 2N $Na_2SO_3$ and water again. The extract was evaporated and chromatographed on silica gel, eluting with EtOAc/petroleum ether (1:19), to give 4-[((1*R*)-2-iodo-1-{[(triisopropylsilyl)oxy]methyl}ethyl)oxy]-4'-(trifluoromethoxy)-1,1'-biphenyl (82) (9.08 g, 81% overall) as a colourless oil; $^1$H NMR ($CDCl_3$) δ 7.54 (d, *J* = 8.8 Hz, 2 H), 7.48 *(d, J* = 8.8

Hz, 2 H), 7.26 (br d, J = 8.8 Hz, 2 H), 7.02 (d, *J* = 8.8 Hz, 2 H), 4.31-4.25 (m, 1 H), 4.03 (dd, *J* = 10.4, 4.8 Hz, 1 H), 3.93 *(dd, J* = 10.4, 5.6 Hz, 1 H), 3.55 (dd, *J* = 10.5, 5.6 Hz, 1 H), 3.45 (dd, *J* = 10.5, 4.8 Hz, 1 H), 1.15-1.06 (m, 21 H). APCI MS *m*/*z* 595 $[M + H]^+$.

A mixture of 2-bromo-4(5)-nitroimidazole (0.73 g, 3.82 mmol), the iodide **82** (2.50 g, 4.20 mmol) and $K_2CO_3$ (0.63 g, 4.58 mmol) in DMF (30 mL) was stirred at 87 °C for 20 h. The resulting mixture was partitioned between EtOAc and brine and the extract was washed well with brine. Evaporation gave an oil, which was chromatographed on silica gel, eluting with EtOAc/petroleum ether (1:9), to give 2-bromo-4-nitro-1-{(2S)-2-{[4'-(trifluoromethoxy)[1,1'-biphenyl]-4-yl]oxy}-3-[(triisopropylsilyl)oxy]propyl}-1H-imidazole **(83)** (1.05 g, 42%) as an oil; $^1H$ NMR ($CDCl_3$) δ 7.95 (s, 1 H), 7.51 (d, *J* = 8.8 Hz, 2 H), 7.45 (d, *J* = 8.8 Hz, 2 H), 7.25 (br d, *J* = 8.8 Hz, 2 H), 6.89 (d, *J* = 8.8 Hz, 2 H), 4.63-4.56 (m, 2 H), 4.37-4.29 (m, 1 H), 4.02 (dd, *J* = 10.7, 3.4 Hz, 1 H), 3.84 (dd, *J*= 10.7, 6.8 Hz, 1 H), 1.17-1.07 (m, 21 H). APCI MS *m*/*z* 660, 658 $[M + H]^+$.

Tetra-*n*-butylammonium fluoride (3.18 mL of a 1M solution in THF, 3.18 mmol) was added at 20 °C to a solution of silyl ether 83 (1.05 g, 1.59 mmol) in THF (40 mL) and the solution was stirred at room temperature for 1 h. After dilution with EtOAc, the solution was washed with saturated aqueous $NaHCO_3$ solution, then water, and then evaporated to give an oil, which was chromatographed on silica. Elution with EtOAc/petroleum ether (1:1) gave fore fractions, and then further elution with EtOAc gave the deprotected alcohol. This material was immediately dissolved in DMF (20 mL) and the solution was cooled to 5 °C and treated with NaH (0.19 g of a 60% dispersion in mineral oil, 4.77 mmol). The cooling bath was removed and the mixture was stirred at 20 °C for 30 min. Water was added, the mixture was extracted with EtOAc and the extract was evaporated to give an oil, which was chromatographed on silica. Elution with EtOAc/petroleum ether (1:1) gave fore fractions, and then further elution with EtOAc/petroleum ether (2:1) gave **13** (175 mg, 26%) as a white solid: mp 210 °C; $[\alpha]^{19}$ -9.5° (c, 0.84, acetone); $^1H$ NMR $[(CD_3)_2SO]$ δ 8.07 (s, 1 H), 7.75 (d, *J* = 8.8 Hz, 2 H), 7.66 (d, *J* = 8.8 Hz, 2 H), 7.41 (br d, *J* = 8.8 Hz, 2 H), 7.15 (d, *J* = 8.8 Hz, 2 H), 5.31-5.27 (m, 1 H), 4.71-4.63 (m, 2 H), 4.42 (dd, *J* = 13.8, 3.2 Hz, 1 H), 4.34 (br d, *J* = 13.8 Hz, 1 H). Anal. ($C_{19}H_{14}F_3N_3O_5$) C, H, N. Chiral HPLC analysis revealed this product to have an ee of 70%.

**R. Synthesis of (6S)-6-{[2-fluoro-4'-(trifluoromethoxy)[1,1'-biphenyl]-4-yl]methoxy}-2-nitro-6,7-dihydro-5*H*-imidazo[2,1-*b*][1,3]oxazine (15) by the method of Scheme 11.**

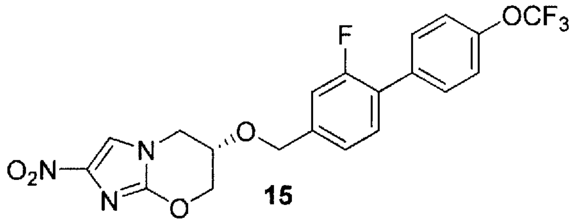

**15**

A suspension of 4-bromo-3-fluorobenzoic acid **(84)** (1.61 g, 7.35 mmol) in anhydrous THF (10 mL, then 4x 3 mL to rinse) under $N_2$ was added drop-wise (over 40 min) to a suspension of sodium borohydride (400 mg, 10.6 mmol) in anhydrous THF (15 mL) under $N_2$, and then the mixture was cooled in an ice bath. A solution of iodine (1.008 g, 3.97 mmol) in anhydrous THF (10 mL, then 2x 3 mL) was added drop-wise (over 35 min) to the stirred solution and then the mixture was stirred at room temperature for 14 h. The mixture was concentrated under reduced pressure and then treated successively with water (20 mL), 10% HCl (3.4 mL) and water (20 mL), and extracted with $CH_2Cl_2$ (4x 50 mL). The extracts were evaporated to dryness and the residue was chromatographed on silica gel, eluting with 50% $CH_2Cl_2$/petroleum ether, to give (4-bromo-3-fluorophenyl)methanol (85) (1.096 g, 73%) as a white solid: mp ($CH_2Cl_2$/petroleum ether) 39-40 °C; $^1H$ NMR ($CDCl_3$) δ 7.52 (dd, *J* = 8.0, 7.2 Hz, 1 H), 7.16 (dd, *J* = 9.3, 1.8 Hz, 1 H), 7.02 (dd, *J* = 8.2, 1.8 Hz, 1 H), 4.67 (d, *J* = 5.9 Hz, 2 H), 1.75 (t, *J* = 5.9 Hz, 1 H); HREIMS calcd for $C_7H_6BrFO$ *m*/*z* ($M^+$) 205.9567, 203.9586, found 205.9566, 203.9580.

Bromination of alcohol 85 as in Example 2A for 20 h gave 1-bromo-4-(bromomethyl)-2-fluorobenzene (86) (100%) as a white solid: mp (pentane) 39-41 °C; $^1H$ NMR ($CDCl_3$) δ 7.52 (dd, *J* = 8.1, 7.1 Hz, 1 H), 7.17 (dd, *J* = 9.0, 2.0 Hz, 1 H), 7.06 (dd, *J* = 8.2, 1.8 Hz, 1 H), 4.41 (d, 2 H); HREIMS calcd for $C_7H_5Br_2F$ *m*/*z* ($M^+$) 269.8701, 267.8722, 265.8742, found 269.8692, 267.8713, 265.8726.

Reaction of bromide **86** (1.29 equiv.) with oxazine alcohol **41** as in Example 2N, followed by chromatography of the product on silica gel, eluting with 0-2% EtOAc/$CH_2Cl_2$ (foreruns) and then 2-4% EtOAc/$CH_2Cl_2$ gave (6*S*)-6-[(4-bromo-3-fluorobenzyl)oxy]-2-nitro-6,7-dihydro-5*H*-imidazo[2,1-*b*][1,3]oxazine **(87)** (89%) as a pale yellow solid: mp (MeOH/$CH_2Cl_2$/hexane) 181-183 °C; $^1H$ NMR $[(CD_3)_2SO]$ δ 8.01 (s, 1 H), 7.68 *(dd, J* = 8.0, 7.5 Hz, 1 H), 7.30 (dd, *J* = 9.8,

1.9 Hz, 1 H), 7.12 (dd, *J* = 8.2, 1.5 Hz, 1 H), 4.70-4.60 (m, 3 H), 4.47 (br d, *J* = 11.7 Hz, 1 H), 4.31-4.18 (m, 3 H). Anal. ($C_{13}H_{11}BrFN_3O_4$) C, H, N.

A stirred mixture of bromide **87** (503 mg, 1.35 mmol), 4-(trifluoromethoxy)phenylboronic acid **(44)** (500 mg, 2.43 mmol) and Pd(dppf)$Cl_2$ (304 mg, 0.415 mmol) in toluene (16 mL) and EtOH (8 mL) was degassed for 12 min (vacuum pump) and then $N_2$ was added. An aqueous solution of 2M $Na_2CO_3$ (3.6 mL, 7.2 mmol) was added by syringe and the stirred mixture was again degassed for 12 min, and then $N_2$ was added. The resulting mixture was stirred at 90 °C for 6 h, and then cooled, diluted with aqueous $NaHCO_3$ (100 mL) and extracted with $CH_2Cl_2$ (6x 100 mL). The extracts were evaporated to dryness and the residue was chromatographed on silica gel. Elution with 0-1% EtOAc/$CH_2Cl_2$ firstly gave foreruns, and then further elution with 2% EtOAc/$CH_2Cl_2$ gave **15** (478 mg, 78%) as a pale yellow solid: mp ($CH_2Cl_2$/pentane) 181-183 °C; $^1H$ NMR ($CDCl_3$) δ 7.55 (dtd, *J* = 8.8, 2.4, 1.5 Hz, 2 H), 7.42 (t, *J* = 7.9 Hz, 1 H), 7.40 (s, 1 H), 7.29 (br dd, *J* = 8.8, 0.9 Hz, 2 H), 7.17 (dd, *J* = 7.8, 1.6 Hz, 1 H), 7.13 (dd, *J* = 11.0, 1.4 Hz, 1 H), 4.77 (d, *J* = 12.2 Hz, 1 H), 4.68-4.62 (m, 2 H), 4.38 (dd, *J* = 12.2, 1.5 Hz, 1 H), 4.26-4.14 (m, 3 H). Anal. ($C_{20}H_{15}F_4N_3O_5$) C, H, N.

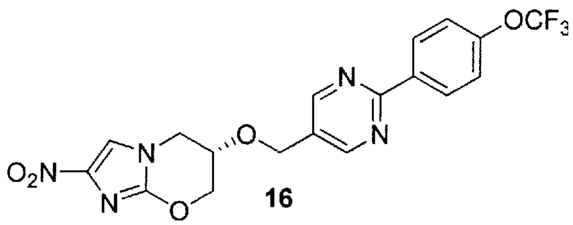

16

**T. Synthesis of (6*S*)-2-nitro-6-({4-[4-(trifluoromethoxy)benzyl]benzyl}oxy)-6,7-dihydro-5*H*-imidazo[2,1-*b*][1,3]oxazine (17) by the method of Scheme 13.**

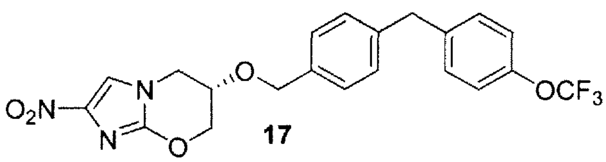

17

A solution of methyl 4-(bromomethyl)benzoate **(93)** (0.23 mL, 1.0 mmol) and 4-(trifluoromethoxy)phenylboronic acid **(44)** (0.24 g, 1.1 mmol) in DME (3 mL) and 2M aqueous $K_2CO_3$ (1 mL) was degassed, then treated with tetrakis(triphenylphosphine) palladium (58 mg, 50 μmol). The reaction mixture was stirred under $N_2$ at 105 °C for 24 h, and then EtOAc (250 mL) was added. The organic layer was washed with water, the aqueous layer was re-extracted with EtOAc (100 mL), and the combined organic layers were washed with brine, dried ($Na_2SO_4$) and concentrated under reduced pressure. The residue was chromatographed on silica gel, eluting with petroleum ether/EtOAc (9:1), to give methyl 4-[4-(trifluoromethoxy)benzyl]benzoate (94) (215 mg, 68%) as a colourless oil; $^1H$ NMR ($CDCl_3$) δ 7.98-7.94 (m, 2 H), 7.26-7.22 (m, 2 H), 7.20-7.12 (m, 4 H), 4.03 (s, 2 H), 3.90 (s, 3 H); HREIMS calcd for $C_{16}H_{13}F_3O_3$ *m/z* ($M^+$) 310.0817, found 310.0815.

$LiAlH_4$ (55 mg, 1.45 mmol) was added to a solution of ester 94 (203 mg, 0.65 mmol) in ether (5 mL) and the mixture was stirred at room temperature for 3 h, then EtOAc (150 mL) was added. The organic layer was washed with water, the aqueous layer was re-extracted with EtOAc (100 mL), and the combined organic layers were washed with brine, dried ($Na_2SO_4$) and concentrated under reduced pressure to give {4-[4-(trifluoromethoxy)benzyl]phenyl}methanol **(95)** (185 mg, quant.) as a white solid: mp (EtOAc/hexane) 60-61 °C; $^1H$ NMR ($CDCl_3$) δ 7.32-7.27 (m, 2 H), 7.20-7.14 (m, 4 H), 7.13-7.09 (m, 2 H), 4.67 (s, 2 H), 3.98 (s, 2 H); HREIMS calcd for $C_{15}H_{13}F_3O_2$ *m/z* ($M^+$) 282.0868, found 282.0866.

A solution of alcohol **95** (0.18 g, 0.64 mmol) in $CH_2Cl_2$ (4 mL) was treated with $PBr_3$ (115 μL, 1.2 mmol). The reaction mixture was stirred at room temperature for 2 h, then EtOAc (150 mL) was added. The organic layer was washed with water (100 mL), the aqueous layer was re-extracted with EtOAc (100 mL), and the combined organic layers were washed with brine (100 mL), dried ($Na_2SO_4$) and concentrated under reduced pressure. The residue was chromatographed on silica gel, eluting with petroleum ether/EtOAc (9:1), to give 1-(bromomethyl)-4-[4-(trifluoromethoxy)benzyl]benzene **(96)** (0.14 g, 64%) as a white solid: mp (EtOAc/hexane) 33-35 °C; $^1H$ NMR ($CDCl_3$) δ 7.34-7.30 (m, 2 H), 7.20-7.10 (m, 6 H), 4.48 (s, 2 H), 3.97 (s, 2 H); HREIMS calcd for $C_{15}H_{12}{}^{79}BrF_3O$ *m/z* ($M^+$) 344.0024, found 344.0033; calcd for $C_{15}H_{12}{}^{81}BrF_3O$ *m/z* ($M^+$) 346.0003, found 346.0011.

A solution of bromide **96** (0.12 g, 0.35 mmol) and alcohol **41** (54 mg, 0.29 mmol) in DMF (2 mL) was treated with NaH (60% in oil, 17 mg, 0.43 mmol) and the mixture was stirred at room temperature for 3 h, then EtOAc (150 mL) was added. The organic layer was washed with water (100 mL), the aqueous layer was re-extracted with EtOAc (100

mL), and the combined organic layers were washed with brine (100 mL), dried ($Na_2SO_4$) and concentrated under reduced pressure. The residue was chromatographed on silica gel, eluting with 0-3% $MeOH/CH_2Cl_2$, to give 17 (95 mg, 73%) as a light yellow solid: mp ($CH_2Cl_2$/MeOH) 132-133 °C; $^1$H NMR [$(CD_3)_2SO$] δ 8.00 (s, 1 H), 7.35-7.30 (m, 2 H), 7.28-7.19 (m, 6 H), 4.63 (dt, *J* = 11.9, 2.3 Hz, 1 H), 4.61 *(d, J* = 11.8 Hz, 1 H), 4.57 (d, *J* = 11.8 Hz, 1 H), 4.45 (d, *J* = 11.9 Hz, 1 H), 4.27-4.17 (m, 3 H), 3.96 (s, 2 H). Anal. ($C_{21}H_{18}F_3N_3O_5$) C, H, N.

**U. Synthesis of (6*S*)-2-nitro-6-[(5-{[4-(trifluoromethoxy)phenyl]ethynyl}-2-pyridinyl)methoxy]-6,7-dihydro-5*H*-imidazo[2,1-*b*][1,3]oxazine (18) by the method of Scheme 14.**

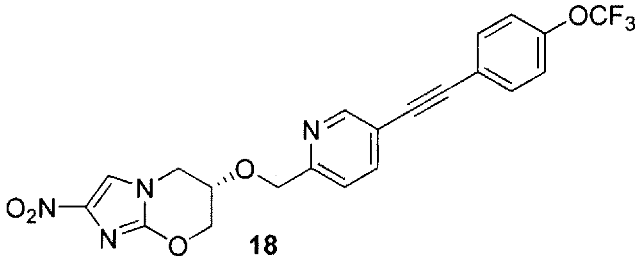

**18**

A mixture of bromide 59 (see Example 2I) (0.310 g, 0.873 mmol), $PdCl_2(PPh_3)_2$ (33 mg, 0.047 mmol) and copper iodide (18 mg, 0.095 mmol) in DMF (4 mL) and $Et_3N$ (4 mL) was purged with $N_2$. Ethynyltrimethylsilane (0.61 mL, 4.3 mmol) was added and the mixture was stirred in a sealed tube at 50 °C for 18 h, and then partitioned between EtOAc and water. The residue was dissolved in THF (20 mL), cooled to 0 °C and treated with TBAF (1M in THF, 1.8 mL), and then the solution was stirred for 2 h. Removal of the solvent gave a residue which was partitioned between EtOAc and water. Column chromatography of the organic portion on silica gel using gradient elution (0-5% MeOH:EtOAc) gave (6*S*)-6-[(5-ethynyl-2-pyridinyl)methoxy]-2-nitro-6,7-dihydro-5*H*-imidazo[2,1-*b*][1,3]oxazine **(97)** (0.178 g, 68%) as a tan solid: mp 135-136 °C; $^1$H NMR [$(CD_3)_2SO$] δ 8.61 (d, *J* = 1.6 Hz, 1 H), 8.02 (s, 1 H), 7.90 (dd, *J* = 8.0, 2.2 Hz, 1 H), 7.38 (d, *J* = 8.0 Hz, 1 H), 4.78 (d, *J* = 13.8 Hz, 1 H), 4.74 (d, *J* = 13.8 Hz, 1 H), 4.69 (dt, *J* = 12.0, 2.6 Hz, 1 H), 4.49 (d, *J* = 12.0 Hz, 1 H), 4.40 (s, 1 H), 4.30-4.35 (m, 2 H), 4.25 (dd, *J* = 13.7, 3.5 Hz, 1 H). Anal. ($C_{14}H_{12}N_4O_4$) C, H, N.

A mixture of alkyne **97** (0.075 g, 0.25 mmol), 1-iodo-4-(trifluoromethoxy)benzene **(70)** (0.088 g, 0.30 mmol) and copper iodide (5 mg, 0.03 mmol) in DMF (2 mL) and $Et_3N$ (2 mL) was purged with $N_2$. $PdCl_2(PPh_3)_2$ (9 mg, 0.01 mmol) was added and the mixture was stirred at room temperature for 0.5 h, and then partitioned between EtOAc and water. Column chromatography of the organic portion on silica gel using gradient elution (0-5% MeOH:EtOAc) gave **18** (0.084 g, 73%) as a white solid: mp 207-208 °C; $^1$H NMR [$(CD_3)_2SO$] δ 8.71 (d, *J*= 1.7 Hz, 1 H), 8.03 (s, 1 H), 7.99 (dd, *J*= 8.1, 2.2 Hz, 1 H), 7.73 (d, *J*= 8.9 Hz, 2 H), 7.42-7.47 (m, 3 H), 4.81 (d, *J* = 13.9 Hz, 1 H), 4.77 (d, *J* = 13.9 Hz, 1 H), 4.71 (dt, *J* = 12.0, 2.5 Hz, 1 H), 4.51 (d, *J* = 11.9 Hz, 1 H), 4.31-4.37 (m, 2 H), 4.26 *(dd, J* = 13.7, 3.5 Hz, 1 H). Anal. ($C_{21}H_{15}F_3N_4O_5$) C, H, N.

**V. Synthesis of (6*S*)-2-nitro-6-[(5-{[6-(trifluoromethyl)-3-pyridinyl]ethynyl}-2-pyridinyl)methoxy)-6,7-dihydro-5*H*-imidazo[2,1-*b*][1,3]oxazine (25) by the method of Scheme 14.**

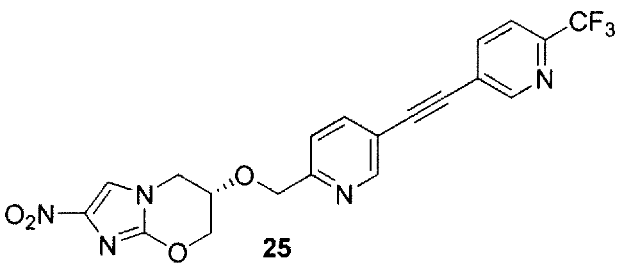

**25**

Sonogashira coupling of alkyne **97** (see Example 2U) (0.075 g, 0.25 mmol) and 5-bromo-2-(trifluoromethyl)pyridine **(98)** (0.068 g, 0.30 mmol) as in Example 2U, at 50 °C for 0.5 h, gave **25** (0.086 g, 77%) as a white solid: mp 226-227 °C; $^1$H NMR [$(CD_3)_2SO$] δ 8.97 *(d, J* = 1.3 Hz, 1 H), 8.78 (d, *J* = 1.4 Hz, 1 H), 8.30 (dd, *J* =8.0, 1.4 Hz, 1 H),

8.06 (dd, *J* = 8.1, 2.2 Hz, 1 H), 8.04 (s, 1 H), 8.00 (d, *J* = 8.2 Hz, 1 H), 7.43 (d, *J* = 8.2 Hz, 1 H), 4.83 (d, *J* = 13.9 Hz, 1 H), 4.79 *(d, J* = 13.9 Hz, 1 H), 4.72 (dt, *J* = 12.0, 2.5 Hz, 1 H), 4.51 *(d, J* = 11.9 Hz, 1 H), 4.32-4.38 (m, 2 H), 4.26 (dd, *J*= 13.8, 3.5 Hz, 1 H). Anal. ($C_{20}H_{14}F_3N_5O_4$) C, H, N.

**W. Synthesis of (6*S*)-2-nitro-6-({(2*E*)-3-[4'-(trifluoromethoxy)[1,1'-biphenyl]-4-yl]-2-propenyl}oxy)-6,7-dihydro-5*H*-imidazo[2,1-*b*][1,3]oxazine (19) by the method of Scheme 15.**

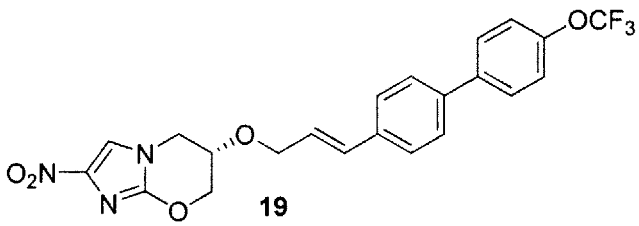

**19**

A solution of methyl (*E*)-3-(4-bromophenyl)-2-propenoate **(99)** (0.500 g, 2.07 mmol) and 4-(trifluoromethoxy)phenylboronic acid (44) (0.612 g, 2.97 mmol) in dioxane (40 mL) and aqueous $K_2CO_3$ (2M, 10 mL, 20 mmol) was purged with $N_2$. Pd(dppf)$Cl_2$ (0.050 g, 0.06 mmol) was added and the solution was refluxed under $N_2$ for 1 h. The dioxane was removed and the residue was extracted with EtOAc, the organic fraction was dried and the solvent was removed. Column chromatography of the residue on silica gel using gradient elution (hexanes to $CH_2Cl_2$) gave methyl (2*E*)-3-[4'-(trifluoromethoxy)[1,1'-biphenyl]-4-yl]-2-propenoate (100) (0.567 g, 85%) as a white solid: mp 98-100 °C; $^1$H NMR ($CDCl_3$) $\delta$ 7.73 (d, *J* = 16.0 Hz, 1 H), 7.56-7.63 (m, 6 H), 7.30 (dd, *J* = 8.8, 0.9 Hz, 2 H), 6.48 (d, *J* = 16.0 Hz, 1 H), 3.82 (s, 3 H). APCI MS *m*/*z* 323 [M + H]$^+$.

DIBAL-H (20% w/w in toluene, 2 mL, 2.39 mmol) was added to a slurry of ester **100** (0.396 g, 1.23 mmol) in toluene (12 mL) at -78 °C. The mixture was warmed to room temperature, stirred for 1 h, and then poured onto ice cold $NH_4Cl$ solution (50 mL). The mixture was diluted with $CH_2Cl_2$ (100 mL), filtered through Celite and the organic layer was dried and evaporated. Column chromatography of the residue on silica gel using gradient elution ($CH_2Cl_2$ to 95:5 $CH_2Cl_2$:EtOAc) gave (2*E*)-3-[4'-(trifluoromethoxy)[1,1'-biphenyl]-4-yl]-2-propen-1-ol **(101)** (0.195 g, 54%) as a white solid: mp 121-123 °C; $^1$H NMR ($CDCl_3$) $\delta$ 7.60 (d, *J*= 8.7 Hz, 2 H), 7.53 (d, J = 8.4 Hz, 2 H), 7.47 *(d, J* = 8.4 Hz, 2 H), 7.28 (d, *J* = 8.1 Hz, 2 H), 6.66 (d, *J* = 15.9 Hz, 1 H), 6.42 (dt, *J* = 15.9, 5.7 Hz, 1 H), 4.36 (dd, *J* = 5.9, 5.7 Hz, 2 H), 1.44 (t, *J* = 5.9 Hz, 1 H). APCI MS *m*/*z* 307 [M - H - $H_2O$ + MeOH]$^-$.

$PBr_3$ (26 $\mu$L, 0.28 mmol) was added to a solution of alcohol **101** (0.159 g, 0.540 mmol) in $Et_2O$ (10 mL) at 0 °C. The mixture was warmed to room temperature and stirred for 1 h, then quenched with ice and extracted with $Et_2O$. The organic fraction was dried and evaporated, and then column chromatography of the residue on silica gel (eluting with $CH_2Cl_2$) gave 4-[(1*E*)-3-bromo-1-propenyl]-4'-(trifluoromethoxy)-1,1'-biphenyl **(102)** (0.123 g, 71%) as a white solid: mp 121-123 °C; $^1$H NMR ($CDCl_3$) $\delta$ 7.60 (d, *J* = 8.8 Hz, 2 H), 7.53 (d, *J* = 8.4 Hz, 2 H), 7.46 (d, *J* = 8.4 Hz, 2 H), 7.28 *(d, J* = 8.8 Hz, 2 H), 6.69 (d, *J* = 15.6 Hz, 1 H), 6.45 (dt, *J* = 15.6, 7.8 Hz, 1 H), 4.18 (dd, *J* = 7.8, 0.9 Hz, 2 H). APCI MS *m*/*z* 277 [M + H - HBr]$^+$.

NaH (60% w/w, 0.016 g, 0.40 mmol) was added to a solution of oxazine alcohol **41** (0.050 g, 0.27 mmol) and bromide **102** (0.100 g, 0.28 mmol) in DMF (6 mL) at -78 °C. The mixture was stirred at 0 °C for 1 h, then quenched with ice and partitioned between EtOAc and water. The organic fraction was dried and evaporated, and then column chromatography of the residue on silica gel using gradient elution (1:1 hexanes:EtOAc to EtOAc) gave **19** (0.079 g, 63%) as a white solid: mp 220-221 °C; $^1$H NMR [$(CD_3)_2SO$] $\delta$ 8.04 (s, 1 H), 7.80 (d, *J* = 8.8 Hz, 2 H), 7.66 (d, *J* = 8.4 Hz, 2 H), 7.55 (d, *J* = 8.4 Hz, 2 H), 7.44 (d, *J* = 8.8 Hz, 2 H), 6.66 *(d, J* = 16.0 Hz, 1 H), 6.43 (dt, *J* = 16.0, 5.9 Hz, 1 H), 4.65 *(d, J* = 11.9 Hz, 1 H), 4.48 (d, *J* = 11.9 Hz, 1 H), 4.21-4.35 (m, 5 H). Anal. ($C_{22}H_{18}F_3N_3O_5$) C, H, N.

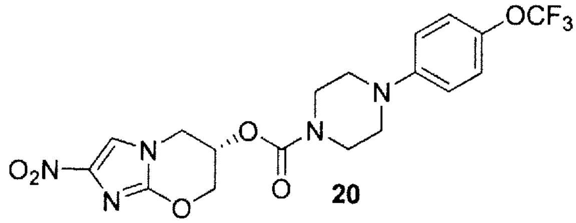

**20**

**Y. Synthesis of (6*S*)-6-({5-[3-fluoro-4-(trifluoromethoxy)phenyl]-2-pyridinyl}methoxy)-2-nitro-6,7-dihydro-5*H*-imidazo[2,1-*b*][1,3]oxazine (22) by the method of Scheme 17.**

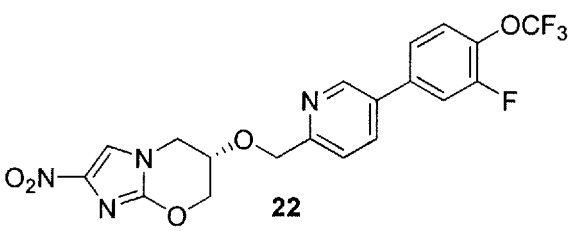

**22**

A stirred mixture of (5-bromo-2-pyridinyl)methanol **(105)** (753 mg, 4.00 mmol), 3-fluoro-4-(trifluoromethoxy)phenylboronic acid **(56)** (see Example 2G) (1.165 g, 5.20 mmol) and Pd(dppf)$Cl_2$ (366 mg, 0.50 mmol) in toluene (40 mL) and EtOH (20 mL) was degassed for 15 min (vacuum pump) and then $N_2$ was added. An aqueous solution of 2M $Na_2CO_3$ (10 mL, 20.0 mmol) was added by syringe and the stirred mixture was again degassed for 15 min, and then $N_2$ was added. The resulting mixture was stirred at 89 °C for 2 h, and then cooled, diluted with aqueous $NaHCO_3$ (120 mL) and extracted with $CH_2Cl_2$ (6x 100 mL). The extracts were evaporated to dryness and the residue was chromatographed on silica gel. Elution with 50-75% $CH_2Cl_2$/petroleum ether firstly gave foreruns, and then further elution with 75% $CH_2Cl_2$/petroleum ether and 0-0.5% MeOH/$CH_2Cl_2$ gave {5-[3-fluoro-4-(trifluoromethoxy)phenyl]-2-pyridinyl}methanol **(106)** (687 mg, 60%) as a light yellow-brown solid: mp 51-53 °C; $^1$H NMR ($CDCl_3$) δ 8.76 (d, *J* = 1.9 Hz, 1 H), 7.84 (dd, *J* = 8.1, 2.3 Hz, 1 H), 7.46-7.34 (m, 4 H), 4.83 (d, *J* = 5.1 Hz, 2 H), 3.47 (t, *J* = 5.2 Hz, 1 H); HRESIMS calcd for $C_{13}H_{10}F_4NO_2$ *m/z* $[M + H]^+$ 288.0642, found 288.0641.

A solution of alcohol **106** (678 mg, 2.36 mmol) and triphenylphosphine (746 mg, 2.84 mmol) in anhydrous $CH_2Cl_2$ (30 mL) was carefully treated with recrystallized N-bromosuccinimide (507 mg, 2.85 mmol) (water bath cooling), and the mixture was stirred at room temperature for 3 h. The resulting solution was concentrated, and then added to excess petroleum ether at the top of a silica gel column (25 g in petroleum ether), rinsing on with minimal extra $CH_2Cl_2$. Elution with petroleum ether firstly gave foreruns, and then further elution with 10-20% $Et_2O$/pentane gave 2-(bromomethyl)-5-[3-fluoro-4-(trifluoromethoxy)phenyl]pyridine (107) (616 mg, 75%) as a white solid that was used directly in the next step; $^1$H NMR ($CDCl_3$) δ 8.76 (dd, *J* = 2.4, 0.6 Hz, 1 H), 7.84 (dd, *J* = 8.1, 2.4 Hz, 1 H), 7.54 (dd, *J* = 8.0, 0.6 Hz, 1 H), 7.46-7.33 (m, 3 H), 4.60 (s, 2 H); HRESIMS calcd for $C_{13}H_9BrF_4NO$ *m/z* $[M + H]^+$ 351.9778, 349.9798, found 351.9778, 349.9798.

A solution of oxazine alcohol **41** (311 mg, 1.68 mmol) and bromide **107** (614 mg, 1.75 mmol) in anhydrous DMF (6.5 mL) under $N_2$ at 0 °C was treated with 60% NaH (88.5 mg, 2.21 mmol), then quickly degassed and resealed under $N_2$. After stirring at room temperature for 2.5 h, the reaction was cooled ($CO_2$/acetone), quenched with ice/aqueous $NaHCO_3$ (20 mL), added to brine (40 mL) and extracted with $CH_2Cl_2$ (8x 50 mL). The combined extracts were evaporated to dryness and the residue was chromatographed on silica gel. Elution with 0-0.75% MeOH/$CH_2Cl_2$ firstly gave foreruns, and then further elution with 0.75-1.5% MeOH/$CH_2Cl_2$ gave **22** (676 mg, 89%) as a light yellow solid: mp ($CH_2Cl_2$/pentane) 182-184 °C; $^1$H NMR ($CDCl_3$) δ 8.74 (dd, *J* = 2.3, 0.7 Hz, 1 H), 7.86 (dd, *J* = 8.1, 2.4 Hz, 1 H), 7.48-7.38 (m, 4 H), 7.35 (ddd, *J* = 8.4, 2.2, 1.0 Hz, 1 H), 4.87 (d, *J* = 13.0 Hz, 1 H), 4.81 (d, *J* = 13.0 Hz, 1 H), 4.70 (ddd, *J* = 12.2, 3.5, 1.5 Hz, 1 H), 4.40 (dd, *J* = 12.2, 1.4 Hz, 1 H), 4.33-4.20 (m, 3 H). Anal. ($C_{19}H_{14}F_4N_4O_5$) C, H, N.

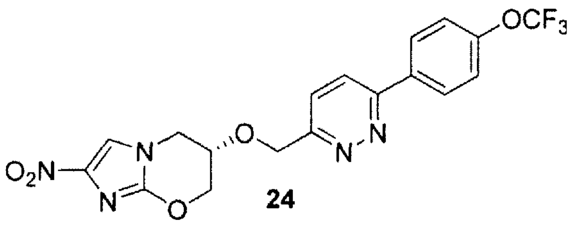

**24**

**AA. Synthesis of (6*S*)-6-{[4-(5-fluoro-2-pyridinyl)benzyl)oxy}-2-nitro-6,7-dihydro-5*H*-imidazo[2,1-*b*][1,3]oxazine (26) by the method of Scheme 19.**

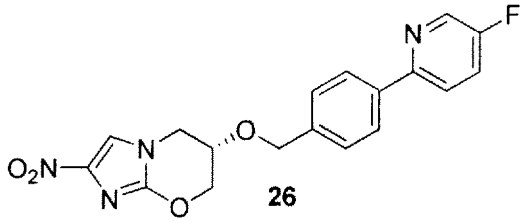

26

A stirred mixture of 4-(hydroxymethyl)phenylboronic acid **(34)** (501 mg, 3.30 mmol) and Pd(dppf)$Cl_2$ (338 mg, 0.462 mmol) in toluene (36 mL) and EtOH (18 mL) was degassed for 15 min (vacuum pump) and then $N_2$ was added. An aqueous solution of 2M $Na_2CO_3$ (9 mL, 18 mmol) was added by syringe and the stirred mixture was again degassed for 15 min, and then $N_2$ was added. 2-Bromo-5-fluoropyridine **(110)** (1.44 g, 8.18 mmol) was added by syringe and the resulting mixture was stirred at 89 °C for 200 min. The cooled mixture was then diluted with aqueous $NaHCO_3$ (100 mL) and extracted with $CH_2Cl_2$ (5x 100 mL). The extracts were evaporated to dryness and the residue was chromatographed on silica gel. Elution with $CH_2Cl_2$ and 0-25% $Et_2O$/petroleum ether firstly gave foreruns, and then further elution with 33-50% $Et_2O$/petroleum ether gave [4-(5-fluoro-2-pyridinyl)phenyl]methanol **(111)** (307 mg, 46%) as a cream solid (following pentane trituration): mp 100-101 °C; $^1$H NMR ($CDCl_3$) $\delta$ 8.54 (d, *J* = 2.9 Hz, 1 H), 7.94 (dt, *J* = 8.4, 1.9 Hz, 2 H), 7.72 (ddd, *J* = 8.8, 4.2, 0.5 Hz, 1 H), 7.50-7.43 (m, 3 H), 4.76 (d, *J* = 6.0 Hz, 2 H), 1.69 (t, *J* = 6.0 Hz, 1 H); HRESIMS calcd for $C_{12}H_{11}FNO$*m/z* $[M + H]^+$ 204.0819, found 204.0824.

A solution of alcohol **111** (305 mg, 1.50 mmol) and triphenylphosphine (474 mg, 1.81 mmol) in anhydrous $CH_2Cl_2$ (12 mL) was carefully treated with recrystallized N-bromosuccinimide (322 mg, 1.81 mmol) (water bath cooling), and the mixture was stirred at room temperature for 3 h. The resulting solution was concentrated, and then added to excess pentane at the top of a silica gel column (20 g in pentane), rinsing on with minimal extra $CH_2Cl_2$. Elution with pentane firstly gave foreruns, and then further elution with 20-50% $Et_2O$/pentane gave 2-[4-(bromomethyl)phenyl]-5-fluoropyridine **(112)** (348 mg, 87%) as a white solid that was used directly in the next step; $^1$H NMR ($CDCl_3$) $\delta$ 8.54 (d, *J* = 2.9 Hz, 1 H), 7.92 (dt, *J* = 8.4, 1.9 Hz, 2 H), 7.72 (ddd, *J* = 8.7, 4.3, 0.4 Hz, 1 H), 7.52-7.43 (m, 3 H), 4.54 (s, 2 H); HRESIMS calcd for $C_{12}H_{10}BrFN$ *m/z* $[M + H]^+$ 267.9955, 265.9975, found 267.9959, 265.9979.

A solution of oxazine alcohol **41** (242 mg, 1.31 mmol) and bromide **112** (346 mg, 1.30 mmol) in anhydrous DMF (5 mL) under $N_2$ at 0 °C was treated with 60% NaH (70 mg, 1.75 mmol), then quickly degassed and resealed under $N_2$. After stirring at room temperature for 135 min, the reaction was cooled ($CO_2$/acetone), quenched with ice/aqueous $NaHCO_3$ (20 mL), added to brine (100 mL) and extracted with $CH_2Cl_2$ (9x 100 mL). The combined extracts were evaporated to dryness and the residue was chromatographed on silica gel. Elution with 0-6% EtOAc/$CH_2Cl_2$ firstly gave foreruns, and then further elution with 7-10% EtOAc/$CH_2Cl_2$ gave the crude product, which was further chromatographed on silica gel. Elution with petroleum ether and 50-67% EtOAc/petroleum ether firstly gave foreruns, and then further elution with 30% EtOAc/$CH_2Cl_2$ gave **26** (357 mg, 74%) as a cream solid: mp ($CH_2Cl_2$/pentane) 180-181 °C; $^1$H NMR ($CDCl_3$) $\delta$ 8.54 (d, *J* = 2.9 Hz, 1 H), 7.95 (dt, *J* = 8.4, 1.9 Hz, 2 H), 7.72 (ddd, *J* = 8.8, 4.2, 0.4 Hz, 1H), 7.48 (ddd, *J* = 8.7, 8.1, 2.9 Hz, 1 H), 7.41 (br d, *J* = 8.4 Hz, 2 H), 7.37 (s, 1 H), 4.79 (d, *J* = 12.2 Hz, I H), 4.68 (d, *J* = 12.2 Hz, 1 H), 4.61 (ddd, *J* = 12.1, 3.7, 1.9 Hz, 1 H), 4.35 (dd, *J* = 12.1, 1.5 Hz, 1 H), 4.20-4.09 (m, 3 H). Anal. ($C_{18}H_{15}FN_4O_4$) C, H, N.

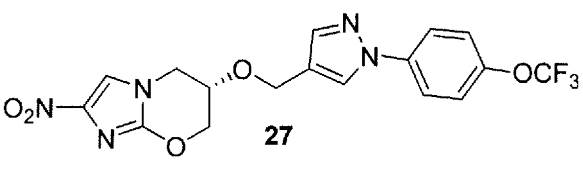

27

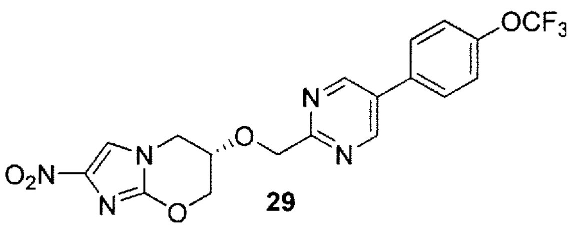

29

**DD. Synthesis of (6*S*)-2-nitro-6-({3-[4'-(trifluoromethoxy)[1,1'-biphenyl]-4-yl]-2-propynyl}oxy)-6,7-dihydro-5*H*-imidazo[2,1-*b*][1,3]oxazine (30) by the method of Scheme 22.**

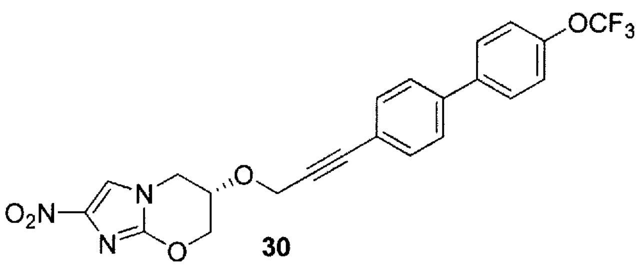

**30**

NaH (60% w/w, 0.280 g, 7.0 mmol) was added to a solution of oxazine alcohol 41 (1.00 g, 5.40 mmol) and 1-bromo-4-(3-bromo-1-propynyl)benzene **(122)** (prepared in two steps from 1-bromo-4-iodobenzene and propargyl alcohol, as described in WO 9524400) (1.57 g, 5.73 mmol) in DMF (25 mL) at 0 °C. The mixture was stirred at 0 °C for 1 h, and then quenched with water and extracted with EtOAc. The organic fraction was dried and the solvent was removed, and then column chromatography of the residue on silica gel using gradient elution (1:1 hexanes:EtOAc to EtOAc) gave (6*S*)-6-{[3-(4-bromophenyl)-2-propynyl]oxy}-2-nitro-6,7-dihydro-5*H*-imidazo[2,1-*b*][1,3]oxazine **(123)** (1.58 g, 77%) as a white solid: mp 160-162 °C; $^1$H NMR [$(CD_3)_2SO$] δ 8.03 (s, 1 H), 7.60 (d, *J* = 8.6 Hz, 2 H), 7.42 (d, *J* = 8.6 Hz, 2 H), 4.66 (dt, *J*= 12.1, 2.4 Hz, 1 H), 4.57 (s, 2 H), 4.49 (d, *J* = 12.1 Hz, 1 H), 4.37-4.40 (m, 1 H), 4.30 (dt, *J* = 13.7, 2.0 Hz, 1 H), 4.25 (dd, *J* = 13.7, 3.2 Hz, 1 H). Anal. ($C_{15}H_{12}BrN_3O_4$) C, H, N.

Suzuki coupling of bromide **123** and 4-(trifluoromethoxy)phenylboronic acid (44) as in Example 2D followed by column chromatography of the product on silica gel using gradient elution (1:1 hexanes: EtOAc to EtOAc) gave **30** (72%) as a white solid: mp 192-194 °C; $^1$H NMR [$(CD_3)_2SO$] δ 8.04 (s, 1 H), 7.83 (d, *J* = 8.8 Hz, 2 H), 7.72 (d, *J* = 8.4 Hz, 2 H), 7.58 (d, *J* = 8.4 Hz, 2 H), 7.46 *(d, J* = 8.8 Hz, 2 H), 4.68 (dt, *J* = 12.1, 2.4 Hz, 1 H), 4.61 (s, 2 H), 4.51 (d, *J* = 12.0 Hz, 1 H), 4.39-4.42 (m, 1 H), 4.32 (dt, *J* = 13.6, 2.0 Hz, 1 H), 4.27 (dd, *J* = 13.6, 3.2 Hz, 1 H). Anal. ($C_{22}H_{16}F_3N_3O_5$) C, H, N.

**EE. Synthesis of (6*S*)-2-nitro-6-[(4-{(*E*)-2-[4-(tritluoromethoxy)phenyl]ethenyl}benzyl)oxy]-6,7-dihydro-5*H*-imidazo[2,1-*b*][1,3]oxazine (31) by the method of Scheme 23.**

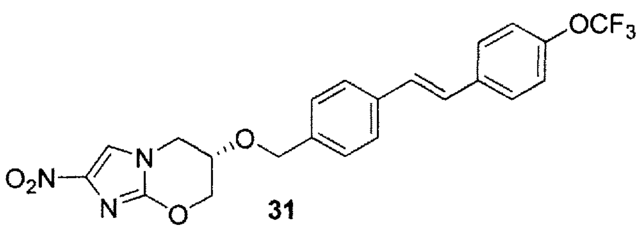

**31**

4-(Trifluoromethoxy)benzaldehyde **(125)** (0.928 g, 4.88 mmol), $K_2CO_3$ (2.8 g, 20 mmol) and 18-crown-6 (0.04 g, 0.15 mmol) were added to a solution of [4-(methoxycarbonyl)benzyl]triphenylphosphonium bromide **(124)** (2.00 g, 4.07 mmol) in THF (60 mL) and $CH_2Cl_2$ (40 mL). The mixture was refluxed under $N_2$ for 18 h, and then partitioned between EtOAc and water. The organic layer was washed with brine and the solvent was removed. Column chromatography of the residue on silica gel, eluting with 19:1 hexanes:EtOAc, gave a crude product which was recrystallised from hexanes to give methyl 4-{(*E*)-2-[4-(trifluoromethoxy)phenyl]ethenyl}benzoate **(126)** (0.549 g, 42%) as white flakes: mp (hexanes) 120-122 °C; $^1$H NMR ($CDCl_3$) δ 8.03 (d, *J* = 8.4 Hz, 2 H), 7.51-7.58 (m, 4 H), 7.16-7.24 (m, 3 H), 7.09 (d, J= 16.3 Hz, 1 H), 3.93 (s, 3 H). APCI MS *m*/*z* 323 $[M + H]^+$.

$LiAH_4$ (0.039 g, 1.03 mmol) was added to a solution of ester **126** (0.166 g, 0.515 mmol) in $Et_2O$ (10 mL) at 0 °C. The mixture was stirred at room temperature for 0.5 h, and then cooled to 0 °C, quenched with ice, and filtered through Celite. The organic fraction was dried and evaporated, and then column chromatography of the residue, eluting with 95:5 $CH_2Cl_2$:MeOH, gave (4-{(*E*)-2-[4-(trifluoromethoxy)phenyl]ethenyl}phenyl)methanol **(127)** (0.182 g, 82%) as a white solid: mp 161-163 °C; $^1$H NMR [$(CD_3)_2SO$] δ 7.78 (d, *J* = 8.8 Hz, 2 H), 7.57 (d, *J* = 8.2 Hz, 2 H), 7.31-7.38 (m, 4 H), 7.27 (s, 2 H), 5.16 (t, *J* = 5.7 Hz, I H), 4.51 (d, *J* = 5.7 Hz, 2 H). APCI MS *m*/*z* 277 $[M + H - H_2O]^+$.

$PBr_3$ (56 μL, 0.60 mmol) was added to a solution of alcohol **127** (0.177 g, 0.601 mmol) in anhydrous $CH_2Cl_2$ (10 mL) at 0 °C. The mixture was stirred at room temperature for 1 h, and then cooled to 0 °C, quenched with ice, and extracted with $CH_2Cl_2$. The organic fraction was dried, and evaporated, and then column chromatography of the residue, eluting with $CH_2Cl_2$, gave 1-{(*E*)-2-[4-(bromomethyl)phenyl]ethenyl}-4-(trifluoromethoxy)benzene **(128)** (0.125 g, 58%) as a white solid: mp 100-102 °C; $^1H$ NMR ($CDCl_3$) δ 7.52 (d, *J* = 8.6 Hz, 2 H), 7.48 (d, *J* = 8.3 Hz, 2 H), 7.39 (d, *J* = 8.3 Hz, 2 H), 7.20 (br d, *J* = 8.1 Hz, 2 H), 7.10 (d, *J* = 16.3 Hz, 1 H), 7.05 (d, *J*= 16.3 Hz, 1 H), 4.51 (s, 2 H). APCI MS *m/z* 277 $[M + H - HBr]^+$.

NaH (60% w/w, 0.010 g, 0.25 mmol) was added to a solution of alcohol **41** (0.024 g, 0.13 mmol) and bromide **128** (0.056 g, 0.16 mmol) in anhydrous DMF (5 mL) at -78 °C. The mixture was then stirred at 0 °C for 1 h, quenched with water, and extracted with EtOAc. The organic fraction was dried and evaporated, and then column chromatography using gradient elution (1:1 hexanes:EtOAc to EtOAc) gave **31** (0.043 g, 72%) as a white solid: mp 228-230 °C; $^1H$ NMR $[(CD_3)_2SO]$ δ 8.02 (s, 1 H), 7.72 (d, *J* = 8.8 Hz, 2 H), 7.59 (d, *J* = 8.2 Hz, 2 H), 7.27-7.38 (m, 6 H), 4.61-4.70 (m, 3 H), 4.47 (d, *J* = 11.9 Hz, 1 H), 4.20-4.31 (m, 3 H). Anal. ($C_{22}H_{18}F_3N_3O_5$) C, H, N.

**FF. Synthesis of (6*S*)-2-nitro-6-[(4-{[4-(trifluoromethoxy)phenyl]ethynyl}benzyl)oxy]-6,7-dihydro-5*H*-imidazo[2,1-*b*][1,3]oxazine (32) by the method of Scheme 24.**

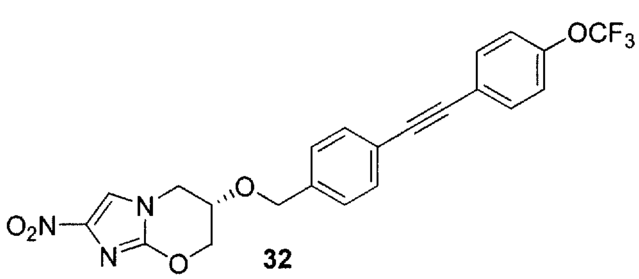

**32**

A mixture of iodide **43** (see Example 2C) (1.00 g, 2.49 mmol) and copper iodide (51 mg, 0.27 mmol) in DMF (10 mL) and $Et_3N$ (10 mL) was purged with $N_2$. Ethynyltrimethylsilane (1.0 mL, 7.1 mmol) and $PdCl_2(PPh_3)_2$ (93 mg, 0.13 mmol) were added and the mixture was stirred under $N_2$ for 0.5 h. The resulting mixture was partitioned between EtOAc and water, the organic fraction was dried, and the solvent was removed. The residue was dissolved in THF (50 mL) and tetra-*n*-butylammonium fluoride (5 mL of a 1M solution in THF, 5 mmol) was added. The solution was stirred for 2 h and then concentrated. The residue was partitioned between EtOAc and water, and the organic fraction was dried and concentrated. Column chromatography of the residue on silca gel using gradient elution (1:1 hexanes EtOAc to EtOAc) gave (6*S*)-6-[(4-ethynylbenzyl)oxy]-2-nitro-6,7-dihydro-5*H*-imidazo[2,1-*b*][1,3]oxazine **(129)** (0.530 g, 71%) as a white solid: mp 162-164 °C; $^1H$ NMR $[(CD_3)_2SO]$ δ 8.01 (s, 1 H), 7.45 (d, *J* = 8.3 Hz, 2 H), 7.32 (d, *J* = 8.3 Hz, 2 H), 4.62-4.71 (m, 3 H), 4.47 (d, *J* = 11.9 Hz, 1 H), 4.20-4.30 (m, 3 H), 4.14 (s, 1 H). Anal. ($C_{15}H_{13}N_3O_4$) C, H, N.

Sonogashira coupling of alkyne **129** and 1-iodo-4-(trifluoromethoxy)benzene (70) as in Example 2U, followed by column chromatography of the product on silica gel using gradient elution (1:1 hexanes: EtOAc to EtOAc), gave **32** (72%) as a white solid: mp 233-236 °C; $^1H$ NMR $[(CD_3)_2SO]$ δ 8.03 (s, 1 H), 7.69 (d, *J*= 8.9 Hz, 2 H), 7.55 (d, *J*= 8.3 Hz, 2 H), 7.42 (d, *J* = 8.9 Hz, 2 H), 7.37 (d, *J* = 8.3 Hz, 2 H), 4.62-4.73 (m, 3 H), 4.48 (d, *J* = 11.9 Hz, 1 H), 4.22-4.32 (m, 3 H). Anal. ($C_{22}H_{16}F_3N_3O_5$) C, H, N.

**GG. Synthesis of (6*S*)-2-nitro-6-[(6-{[4-(trifluoromethoxy)phenyl]ethynyl}-3-pyridinyl)methoxy]-6,7-dihydro-5*H*-imidazo[2,1-*b*][1,3]oxazine (33) by the method of Scheme 24.**

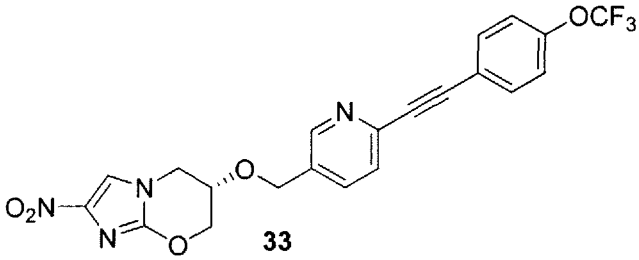

33

Sonogashira coupling of bromide **52** (see Example 2F) (0.310 g, 0.873 mmol) and ethynyltrimethylsilane (0.61 mL, 4.3 mmol) at room temperature for 18 h, followed by desilylation with TBAF, as in Example 2U, gave (6*S*)-6-[(6-ethynyl-3-pyridinyl)methoxy]-2-nitro-6,7-dihydro-5*H*-imidazo[2,1-*b*][1,3]oxazine (130) (0.150 g, 57%) as a white solid: mp 168-170 °C; $^1$H NMR [$(CD_3)_2SO$] δ 8.51 (d, *J* = 1.6 Hz, 1 H), 8.02 (s, 1 H), 7.74 (dd, *J* = 8.0, 2.2 Hz, 1 H), 7.54 (dd, *J* = 8.0, 0.6 Hz, 1 H), 4.73 *(d, J* = 12.6 Hz, 1 H), 4.70 (d, *J* = 12.6 Hz, 1 H), 4.67 (dt, *J* = 12.3, 2.3 Hz, 1 H), 4.47 (d, *J* = 11.9 Hz, 1 H), 4.29 (s, 1 H), 4.20-4.28 (m, 3 H). APCI MS *m*/*z* 301 [M + H]$^+$.

Sonogashira coupling of alkyne 130 and 1-iodo-4-(trifluoromethoxy)benzene **(70)** as in Example 2U gave **33** (55%) as a white solid: mp 235-238 °C; $^1$H NMR [$(CD_3)_2SO$] δ 8.56 (d, *J* = 1.7 Hz, 1 H), 8.03 (s, 1 H), 7.72-7.82 (m, 3 H), 7.65 (d, *J* = 7.9 Hz, 1 H), 7.45 (d, *J* = 8.0 Hz, 2 H), 4.76 (d, *J* = 12.7 Hz, 1 H), 4.72 (d, *J* = 12.7 Hz, 1 H), 4.69 (dt, *J* = 12.0, 2.3 Hz, 1 H), 4.49 (d, *J* = 11.9 Hz, 1 H), 4.22-4.32 (m, 3 H). Anal. ($C_{21}H_{15}F_3N_4O_5$) C, H, N.

**EXAMPLE 3. PHYSICOCHEMICAL PROPERTIES, STABILITY, AND BIOLOGICAL ACTIVITIES**

The physicochemical properties of the compounds of the invention were evaluated as follows. Results are shown below in Table 2.

(a) Calculated lipophilicity (CLOGP). These were calculated using LogP/log D prediction software from ACD/Labs (version 8.0, Advanced Chemistry Development, Inc., Toronto, Ontario, Canada).

(b) Water solubility. The solid compound sample was mixed with water (enough to make a 2 mM solution) in an Eppendorf tube and the suspension was sonicated for 15 min, and then centrifuged at 13,000 rpm for 6 min. An aliquot of the clear supernatant was diluted 2-fold with water, and then HPLC was conducted. The solubility was calculated by comparing the peak area obtained with that from a standard solution of the compound in DMSO (after allowing for varying dilution factors and injection volumes).

The microsomal stability and in vitro biological activity of the compounds of the invention was also evaluated, with results shown in Table 2.

(a) Minimum inhibitory concentrations (MICs). Compounds were evaluated for their activity against replicating *Mycobacterium tuberculosis* in an 8 day microplate-based assay using Alamar blue reagent (added on day 7) for determination of growth (MABA) (Collins et al., 1997; Falzari et al., 2005). The lowest compound concentration effecting an inhibition of >90% was considered the MIC. Screening for the activity of the compounds against bacteria in the non-replicating state that models clinical persistence used an 11 day high-throughput, luminescence-based low-oxygen-recovery assay (LORA), where *M. tuberculosis* bacteria containing a plasmid with an acetamidase promoter driving a bacterial luciferase gene were first adapted to low oxygen conditions by extended culture (Cho et al., 2007).

(b) Stability of the compounds to human and mouse microsomes. Test compounds (1 μM) were incubated at 37 °C with pooled human or CD-1 mouse liver microsome preparations (0.5 mg/mL final protein concentration) and an NADPH regenerating system ($MgCl_2$, 3.3 mM; G6P, 3.3 mM; G6PD, 0.4 U/mL; NADP+, 1.3 mM) in phosphate buffer (75 mM, pH 7.4), with a final volume of 200 μL. The compounds were dissolved in DMSO such that the final DMSO concentration was 0.5%. Reactions were stopped at 0 and 60 min by the addition of MeCN (100 μL) containing 0.2 μM metoprolol as an internal standard. Samples were diluted 10x and centrifuged prior to analysis by LC-MS/MS using electrospray ionization and SRM monitoring using a gradient LC method. LC peak areas were integrated and expressed as analyte/IS peak area ratios (PAR), and a mean value for each time point was calculated from the duplicates. The percent remaining value was calculated as:

$$\% \text{ remaining} = 100 \text{ x } (\text{Mean } PAR_{T60} / \text{Mean } PAR_{T0}).$$

**Table 2. Physicochemistry, microsomal stability and in vitro biological activity of the compounds of Table 1**

| No | Physicochemistry | | MIC (μM) | | Microsomes (% remaining, 1 h) | |
|---|---|---|---|---|---|---|
| | LOGP (calc) | Solubility (μg/mL) | MABA (aerobic) | LORA (anaerobic) | Human | Mouse |
| | | | | | | |
| **PA-824** | 2.70 | 19 | 0.50 | 2.6 | 82 | 94 |
| **1** | 5.07 | 0.66 | 0.04 | 0.78 | 91 | 86 |
| **2** | 4.33 | 0.1 | 0.03 | 0.34 | 93 | 86 |
| **3** | 4.36 | 1.2 | 0.035 | 1.3 | 97 | 96 |
| **4** | 2.19 | 2.6 | 0.023 | 1.0 | 98 | 91 |
| **5** | 2.10 | 3.8 | 0.06 | 2.9 | 88 | 80 |
| **6** | 3.01 | 2.3 | 0.05 | 0.54 | 83 | 87 |
| **7** | 3.04 | 2.5 | 0.065 | 3.7 | 97 | 97 |
| **8** | 3.54 | 0.20 | 0.06 | 1.0 | 93 | 90 |
| **9** | 3.57 | 1.0 | 0.03 | 2.1 | 86 | 91 |
| **10** | 2.60 | 2.7 | 0.05 | 0.61 | 87 | 67 |
| **11** | 2.46 | 5.3 | 0.06 | 0.58 | 86 | 81 |
| **12** | 4.38 | 1.4 | 0.017 | 1.2 | 93 | 85 |
| **13** | 3.98 | 3.0 | 0.05 | 1.3 | 99 | 97 |
| **14** | 3.56 | 1.2 | 0.055 | 2.3 | 90 | 77 |
| **15** | 4.87 | 0.33 | 0.055 | 0.51 | 97 | 91 |
| **16** | 3.05 | 2.1 | 0.027 | 1.8 | 96 | 87 |
| **17** | 4.69 | 0.16 | 0.02 | 1.1 | 85 | 70 |
| **18** | 3.77 | 0.36 | 0.02 | 1.4 | 98 | 97 |
| **19** | 4.83 | 0.50 | 0.063 | >64 | 100 | 95 |
| **20** | 1.56 | 17 | 0.13 | 1.1 | 82 | 85 |
| **21** | 2.99 | 3.0 | 0.025 | 0.93 | 100 | 90 |
| **22** | 3.02 | 30 | 0.05 | 1.3 | 97 | 86 |
| **23** | 3.59 | 5.7 | 0.13 | 0.68 | 96 | 78 |
| **24** | 1.52 | 6.1 | 0.075 | 1.7 | 94 | 92 |
| **25** | 2.30 | | 0.035 | 0.74 | 88 | 91 |
| **26** | 2.09 | 67 | 0.035 | 1.3 | 83 | 61 |
| **27** | 2.60 | | 0.15 | 1.8 | 87 | 64 |
| **28** | 3.53 | 0.18 | 0.017 | 1.0 | 87 | 77 |
| **29** | 2.63 | 8.1 | 0.11 | 1.9 | 92 | 87 |
| **30** | 5.60 | 0.07 | 0.16 | 0.99 | 93 | 85 |
| **31** | 5.35 | | 0.02 | 27 | 99 | 98 |
| **32** | 5.26 | | 0.017 | >128 | | |
| **33** | 3.77 | | 0.02 | 0.94 | | |

The in vivo biological activity of the compounds of the invention was evaluated in two assays, and pharmacokinetic parameters were also determined, with results shown below in Table 3.

(a) In vivo mouse acute TB infection assay. BALB/c mice were infected via aerosol with a suspension of ~2 x $10^6$ colony forming units (CFU) of *M. tuberculosis* Erdman/mL (Falzari et al., 2005). Each compound was given orally to a group of 7 or 8 mice at 100 mg/kg daily for 5 days a week for 3 weeks, beginning on day 11 post-infection. Compounds were administered as a suspension in 0.5% CMC/0.08% Tween 80 in water. Mice were sacrificed on day 31 and the numbers of CFU in the lungs were determined and compared with the CFU for vehicle alone-treated mice at this time. PA-824 was employed as a positive control in each experiment, and the results are recorded as the ratio of the average reduction in CFU in the compound-treated mice/the average CFU reduction in the mice treated with PA-824. In this assay, PA-824 caused up to 2.5-3 log reductions in CFU.

(b) In vivo mouse chronic TB infection assay. Compounds were given orally as in (a) but with treatment beginning ~70 days after infection. In this assay, PA-824 caused a ~2 log reduction in CFU.

(c) In vivo pharmacokinetics. Compounds were administered orally to CD-1 mice at a dose of 40 mg/kg, as a suspension in 0.5% carboxymethylcellulose/0.08% Tween 80 in water. Samples derived from plasma and lungs were analyzed by LC-MS/MS to generate the required pharmacokinetic parameters.

**Table 3. In vivo pharmacokinetics and biological activity of selected compounds of Table 1**

| No | In vivo pharmacokinetics | | | | In vivo efficacy vs PA-824 | |
|---|---|---|---|---|---|---|
| | t½ (h) plasma | Cmax plasma (μg/mL) | AUC lung (μg·h/mL) | AUC ratio (lung/plasma) | Acute | Chronic |
| | | | | | | |
| **PA-824** | 4.2 | 5.5 | 296 | 3.8 | 1.0 | 1.0 |
| **1** | 19.9 | 6.8 | 3363 | 17 | 23 | 2.1 |
| **2** | 20.5 | 17.1 | >513 | >1 | 419 | ND |
| **3** | 14.4 | 7.4 | 218 | 1.1 | >205 | 12 |
| **4** | 7.2 | 9.6 | >324 | >2.1 | 167 | ND |
| **5** | 2.7 | 5.3 | 136 | 2.2 | 7.8 | 20 |
| **6** | 24 | 12.2 | 81 | 0.27 | >89 | 15 |
| **7** | 5.4 | 25.9 | 427 | 1.0 | 27 | 0.9 |
| **8** | 8.8 | 1.1 | 67.3 | 3.3 | 33 | 1.7 |
| **9** | ND | 13.5 | 414 | 1.9 | 15 | 4.6 |
| **10** | 2.4 | 1.51 | 31.5 | 3.5 | 41 | ND |
| **11** | 6.6 | 1.66 | 92.4 | 4.7 | 12 | 0.9 |
| **12** | 22 | 6.7 | 549 | 2.7 | 33 | 16 |
| **13** | 38 | 1.86 | 347 | 3.4 | 8.1 | ND |
| **14** | 13.9 | 0.44 | 148 | 16 | 52 | ND |
| **15** | ND | ND | ND | ND | >933 | ND |
| **16** | 23.1 | 1.16 | 131 | 3.0 | >1120 | ND |
| **18** | 23.5 | 2.36 | 235 | 2.5 | >933 | ND |
| **20** | 10.5 | 2.5 | 169 | 3.2 | 5.2 | ND |
| **21** | 13.9 | 7.0 | 251 | 1.4 | >840 | ND |
| **22** | 8.4 | 7.6 | 155 | 1.1 | 233 | ND |

(continued)

| No | In vivo pharmacokinetics | | | | In vivo efficacy vs PA-824 | |
|---|---|---|---|---|---|---|
| | t½ (h) plasma | Cmax plasma (μg/mL) | AUC lung (μg·h/mL) | AUC ratio (lung/plasma) | Acute | Chronic |
| **23** | 4.9 | 1.56 | 55.4 | 2.5 | 8.8 | ND |
| **24** | 2.9 | 3.56 | 93.9 | 1.8 | 11 | ND |
| **28** | 21.7 | 2.89 | 284 | 3.2 | >933 | ND |
| **30** | 7.5 | 0.57 | 13.6 | 1.4 | 89 | 11 |
| **31** | ND | ND | ND | ND | 5.8 | ND |

**REFERENCES CITED**

The content of each of the documents listed below is hereby incorporated by reference.

U.S. Patent Documents

U.S. Patent No. 5,668,127
U.S. Patent No. 6,087,358

International Patent Documents

EP 1555259
WO 95/24400
WO 2007/075872

Non-Patent Publications

Anderson et al., Org. Biomol. Chem. 6, 1973-1980 (2008).
Cho et al., Antimicrob. Agents Chemother. 51, 1380-1385 (2007).
Collins et al., Antimicrob. Agents Chemother. 41, 1004-1009 (1997).
deSolms et al., J. Med. Chem. 46, 2973-2984 (2003).
Edsall et al., Bioorg. Med. Chem. 11, 3457-3474 (2003).
Falzari et al., Antimicrob. Agents Chemother. 49, 1447-1454 (2005).
Ferrara et al., Lancet 367, 1328-1334 (2006).
Kiener et al., Synlett 10, 814-816 (1994).
Kim et al., J. Med. Chem. 52, 1317-1328 and 1329-1344 (2009).
Li et al., Bioorg. Med. Chem. Lett. 18, 2256-2262 (2008).
Manjunatha et al., Proc, Natl. Acad. Sci. USA 103, 431-436 (2006).
Sasaki et al., J. Med. Chem. 49, 7854-7860 (2006).
Schubert et al., Synlett 3, 342-344 (1999).
Singh et al., Science 322, 1392-1395 (2008).
Stover et al., Nature 405, 962-966 (2000).
Tyagi et al., Antimicrob. Agents Chemother. 49, 2289-2293 (2005).
van den Heuvel et al., J. Org. Chem. 69, 250-262 (2004).

## Claims

1. A compound having a general structure of Formula I:

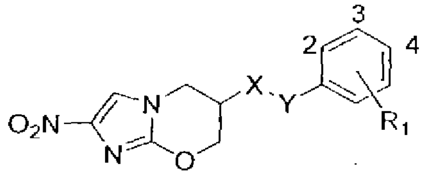

I

wherein X is O, $OCH_2$, $OCH_2CH=CH$ or $OCH_2C\equiv C$.
Y is any one of formulae IIa-IIc:

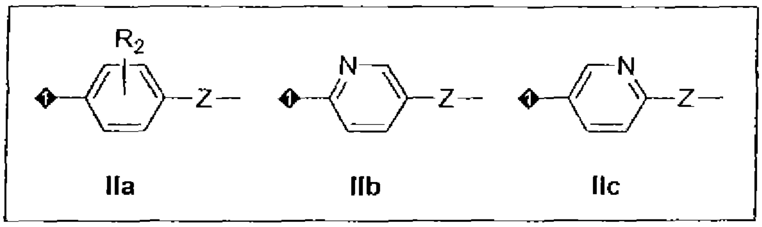

IIa IIb IIc

wherein ◆— is an attachment to X and Formula IIa comprises a ring having $R_2$ as a substituent, and Z in Formulae IIa-IIc is $CH_2$, CH=CH, C=C or a direct bond,

numbers 2, 3, and 4 are ring positions on a terminal ring having $R_1$ as a substituent, the terminal ring of Formula I comprises C, CH or aza at each ring position, and
$R_1$ and $R_2$ in Formulae I and IIa are each one, two, or three substituents located at any available ring position and are independently H, F, Cl, $CF_3$, $OCF_2H$, $OCF_3$, or combinations thereof.

2. The compound of claim 1 wherein:

X is O, $OCH_2$, $OCH_2CH=CH$ or $OCH_2C\equiv C$,
Y is any one of Formulae IIa-IIc:

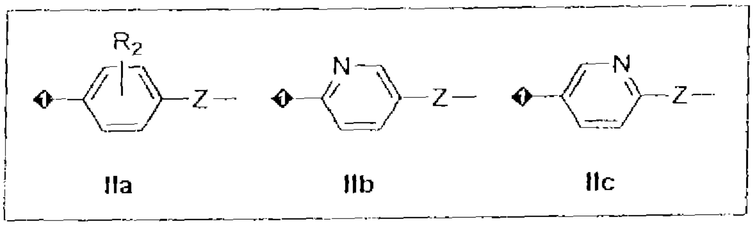

IIa IIb IIc

wherein ◆— is an attachment to X and Formula IIa comprises a ring having $R_2$ as a substituent, and Z in Formulae IIa-IIc is $CH_2$, CH=CH, C=C or a direct bond,

numbers 2, 3, and 4 are ring positions on a terminal ring having $R_1$ as a substituent,
the terminal ring of Formula 1 comprises C, or CH at each ring position, or comprises aza at the 2-position and C, or CH at each remaining ring position, or comprises aza at the 3-position and C, or CH at each remaining ring position,
$R_1$ in Formula I is any one or more of F located at ring position 4, $OCF_3$ located at ring position 4, Cl located at ring position 2, Cl located at ring position 3, F located at ring position 3, $CF_3$ located at ring position 4, or combinations thereof, and
$R_2$ in Formula IIa is any one or two of H or F at any available position.

**3.** A pharmaceutical composition comprising a therapeutically effective amount of the compound of claim 1.

**4.** The pharmaceutical composition of claim 3, further comprising a pharmaceutically acceptable excipient, adjuvant, carrier, buffer or stabilizer.

**5.** The pharmaceutical composition of claim 3, further comprising one or more additional anti-infective treatments.

**6.** A composition of claim 3 for use in preventing and treating microbial infection.

**7.** A composition for use of claim 6, wherein the microbial infection is caused by *Mycobacterium tuberculosis.*

**8.** A compound selected from the group consisting of:

A. (6*S*)-6-{[2'-Chloro-4'-(trifluoromethoxy)[1,1'-biphenyl]-4-yl]methoxy}-2-nitro-6,7-dihydro-5*H*-imidazo[2,1-*b*][1,3]oxazine;
B. (6*S*)-6-{[3'-Fluoro-4'-(trifluoromethoxy)[1,1'-biphenyl]-4-yl]methoxy}-2-nitro-6,7-dihydro-5*H*-imidazo[2,1-*b*]-[1,3]oxazine;
C. (6*S*)-2-Nitro-6-{[4'-(trifluoromethoxy)[1,1'-biphenyl]-4-yl]methoxy}-6,7-dihydro-5*H*-imidazo[2,f-b][1,3]oxazine;
D. (6*S*)-2-Nitro-6-({4-[5-(trifluoromethyl)-2-pyridinyl]benzyl}oxy)-6,7-dihydro-5*H*-imidazo[2,1-*b*][1,3]oxazine;
E. (6*S*)-2-Nitro-6-({4-[6-(trifluoromethyl)-3-pyridinyl]benzyl}oxy)-6,7-dihydro-5*H*-imidazo[2,1-*b*][1,3]oxazine;
F. (6*S*)-6-{[3-Fluoro-4'-(trifluoromethoxy)[1,1'-biphenyl]-4-yl]methoxy}-2-nitro-6,7-dihydro-5*H*-imidazo[2,1-*b*][1,3]oxazine;
G. (6S)-2-Nitro-6-{[4'-(trifluoromethoxy)[1,1'-biphenyl]-4-yl]oxy}-6,7-dihydro-5*H*-imidazo[2,1-*b*][1,3]oxazine;
H. (6*S*)-6-({2-Fluoro-4-[5-(trifluoromethyl)-2-pyridinyl]benzyl}oxy)-2-nitro-6,7-dihydro-5*H*-imidazo[2,1-*b*][1,3]-oxazine;
I. (6*S*)-6-{[2-Fluoro-4'-(trifluoromethoxy)[1,1'-biphenyl]-4-yl]methoxy}-2-nitro-6,7-dihydro-5*H*-imidazo[2,1-*b*]-[1,3]oxazine;
J. (6*S*)-2-Nitro-6-({4-[4-(trifluoromethoxy)benzy]benzyl}oxy)-6,7-dihydro-5*H*-imidazo[2,1-*b*][1,3]oxazine;
K. (6*S*)-2-Nitro-6-({(2*E*)-3-[4'-(trifluoromethoxy)[1,1']biphenyl]-4-yl]-2-propenyl}oxy)-6,7-dihydro-5*H*-imidazo[2,1-*b*][1,3]oxazine;
L. (6*S*)-6-({2-Fluoro-4-[6-(trifluoromethyl)-3-pyridinyl]benzyl}oxy)-2-nitro-6,7-dihydro-5*H*-imidazo[2,1-*b*][1,3]oxazine;
M. (6*S*)-6-{[4-(5-Fluoro-2-pyridinyl)benzyl]oxy}-2-nitro-6,7-dihydro-5*H*-imidazo[2,1-*b*][1,3]oxazine;
N. (6*S*)-2-Nitro-6-({3-[4'-(trifluoromethoxy)[1,1'-biphenyl]-4-yl]-2-propynyl}oxy)-6,7-dihydro-5*H*-imidazo[2,1-*b*]-[1,3]oxazine;
O. (6*S*)-2-Nitro-6-[(4-{(*E*)-2-[4-(trifluoromethoxy)phenyl]ethenyl}benzyl)oxy]-6,7-dihydro-5*H*-imidazo[2,1-*b*][1,3]oxazin;
P. (6*S*)-2-Nitro-6-[(4-{[4-(trifluoromethoxy)phenyl)ethynyl}benzyl)oxy]-6,7-díhydro-5*H*-imidazo[2,1-*b*][1,3]oxazine; and mixtures, optical or geometric isomers, and pharmacologically acceptable salt derivatives thereof.

**9.** A compound selected from the group consisting of:

A. (6*S*)-6-{[6-(4-Fluorophenyl)-3-pyridinyl]methoxy}-2-nitro-6,7-dihydro-5*H*-imidazo[2,1-*b*][1,3]oxazine;
B. (6*S*)-2-Nitro-6-({6-[4-(trifluoromethoxy)phenyl]-3-pyridinyl}methoxy)-6,7-dihydro-5*H*-imidazo[2,1-*b*][1,3]oxazine;
C. (6*S*)-2-Nitro-6-({5-[4-(trifluoromethoxy)phenyl]-2-pyridinyl}methoxy)-6,7-dihydro-5*H*-imidazo[2,1-*b*][1,3]oxazine;
D. (6*S*)-2-Nitro-6-[(5-{[4-(trifluoromethoxy)phenyl]ethynyl}-2-pyridinyl)methoxy]-6,7-dihydro-5*H*-imidazo[2,1-*b*][1,3]oxazine;
E. (6*S*)-6-({6-[3-Fluoro-4-(trifluoromethoxy)phenyl]-3-pyridinyl}methoxy)-2-nitro-6,7-dihydro-5*H*-imidazo[2,1-*b*][1,3]oxazine;
F. (6*S*)-6-({5-(3-Fluoro-4-(trifluoromethoxy)phenyl]-2-pyridinyl}methoxy)-2-nitro-6,7-dihydro-5*H*-imidazo[2,1-*b*][1,3]oxazine;
G. (6*S*)-2-Nitro-6-[(5-{[6-(trifluoromethyl)-3-pyridinyl]ethynyl}-2-pyridinyl)methoxy]-6,7-dihydro-5*H*-imidazo[2,1-*b*][1,3]oxazine;
H. (6*S*)-6-({6-[3-Chloro-4-(trifluoromethoxy)phenyl]-3-pyridinyl}methoxy)-2-nitro-6,7-dihydro-5*H*-imidazo[2,1-*b*][1,3]oxazine;

I. (6*S*)-2-Nitro-6-[(6-{[4-(trifluoromethoxy)phenyl]ethynyl}-3-pyridinyl)methoxy]-6,7-dihydro-5*H*-imidazo[2,1-*b*][1,3]oxazine; and
mixtures, optical or geometric isomers and pharmacologically acceptable salt derivatives thereof.

## Patentansprüche

1. Eine Verbindung mit einer allgemeinen Struktur der Formel I:

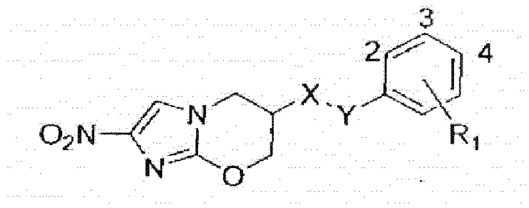

I

wobei X gleich O, $OCH_2$, $OCH_2CH{=}CH$ oder $OCH_2C{\equiv}C$ ist,
Y eine aus den Formeln IIa-IIc ist:

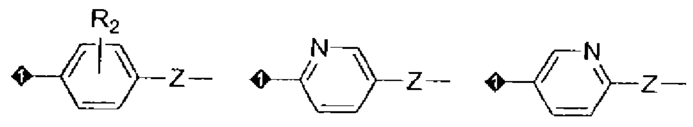

IIa IIb IIc

wobei ◆— eine Bindung an X ist und Formel IIa einen Ring mit $R_2$ als einen Substituenten umfasst, und Z in den Formeln IIa-IIc gleich $CH_2$, CH=CH, C=C oder eine direkte Bindung ist,

die Nummern 2, 3 und 4 Ringpositionen an einem endständigen Ring mit $R_1$ als einem Substituenten sind, der endständige Ring der Formel I an jeder Ringposition C, CH oder Aza umfasst und $R_1$ und $R_2$ in den Formeln I und IIa jeweils ein, zwei oder drei Substituenten sind, die sich an jeder verfügbaren Ringposition befinden und unabhängig H, F, Cl, $CF_3$, $OCF_2H$, $OCF_3$ oder Kombinationen davon sind.

2. Die Verbindung nach Anspruch 1, wobei:

X gleich O, $OCH_2$, $OCH_2CH{=}CH$ oder $OCH_2C{\equiv}C$ ist,
Y eine aus den Formeln IIa-IIc ist:

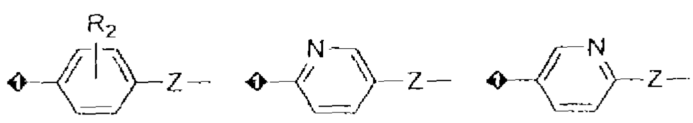

IIa IIb IIc

wobei ◆— eine Bindung an X ist und Formel IIa einen Ring mit $R_2$ als einen Substituenten umfasst, und Z in den Formeln IIa-IIc gleich $CH_2$, CH=CH, C≡C oder eine direkte Bindung ist,

die Nummern 2, 3 und 4 Ringpositionen an einem endständigen Ring mit $R_1$ als einem Substituenten sind, der endständige Ring der Formel I an jeder Ringposition C oder CH umfasst oder Aza an der Position 2 und C oder CH an jeder verbleibenden Ringposition umfasst oder Aza an der Position 3 und C oder CH an jeder

verbleibenden Ringposition umfasst,
$R_1$ in Formel I ein oder mehrere aus F, das sich an Ringposition 4 befindet, $OCF_3$, das sich an Ringposition 4 befindet, Cl, das sich an Ringposition 2 befindet, Cl, das sich an Ringposition 3 befindet, F, das sich an Ringposition 3 befindet, $CF_3$, das sich an Ringposition 4 befindet oder Kombinationen davon ist, und
$R_2$ in Formel IIa ein oder zwei aus H oder F an jeder verfügbaren Position ist.

**3.** Ein Arzneimittel, umfassend eine therapeutisch wirksame Menge der Verbindung nach Anspruch 1.

**4.** Das Arzneimittel nach Anspruch 3, weiter umfassend einen pharmazeutisch verträglichen Exzipienten, Hilfsstoff, Träger, Puffer oder Stabilisator.

**5.** Das Arzneimittel nach Anspruch 3, weiter umfassend eine oder mehrere zusätzliche antiinfektiöse Behandlungen.

**6.** Eine Zusammensetzung nach Anspruch 3 zur Verwendung bei der Vorbeugung und Behandlung von mikrobieller Infektion..

**7.** Eine Zusammensetzung zur Verwendung nach Anspruch 6, wobei die mikrobielle Infektion durch *Mycobacterium tuberculosis* verursacht wird.

**8.** Eine Verbindung, ausgewählt aus der Gruppe bestehend aus:

A. (6*S*)-6-{[2'-Chlor-4'-(trifluormethoxy)[1,1'-biphenyl]-4-yl]methoxy}-2-nitro-6,7-dihydro-5*H*-imidazo[2,1-*b*][1,3]oxazin;
B. (6*S*)-6-{[3'-Fluor-4'-(trifluormethoxy)[1,1'-biphenyl]-4-yl]methoxy}-2-nitro-6,7-dihydro-5*H*-imidazo[2,1-*b*][1,3]oxazin;
C. (6*S*)-2-Nitro-6-{[4'-(trifluormethoxy)[1,1'-biphenyl]-4-yl]methoxy}-6,7-dihydro-5*H*-imidazo[2,1-*b*][1,3]oxazin;
D. (6*S*)-2-Nitro-6-({4-[5-(trifluormethyl)-2-pyridinyl]benzyl}oxy)-6,7-dihydro-5*H*-imidazo[2,1-*b*][1,3]oxazin;
E. (6*S*)-2-Nitro-6-({4-[6-(trifluormethyl)-3-pyridinyl]benzyl}oxy)-6,7-dihydro-5*H*-imidazo[2,1-*b*][1,3]oxazin;
F. (6*S*)-6-{[3-Fluor-4'-(trifluormethoxy)[1,1'-biphenyl]-4-yl]methoxy}-2-nitro-6,7-dihydro-5*H*-imidazo[2,1-*b*][1,3]oxazin;
G. (6*S*)-2-Nitro-6-{[4'-(trifluormethoxy)[1,1'-biphenyl]-4-yl]oxy}-6,7-dihydro-5*H*-imidazo[2,1-*b*][1,3]oxazin;
H. (6*S*)-6-({2-Fluor-4-[5-(trifluormethyl)-2-pyridinyl]benzyl}oxy)-2-nitro-6,7-dihydro-5*H*-imidazo[2,1-b][1,3]oxazin;
I. (6*S*)-6-{[2-Fluor-4'-(trifluormethoxy)[1,1'-biphenyl]-4-yl]methoxy}-2-nitro-6,7-dihydro-5*H*-imidazo[2,1-*b*][1,3]oxazin;
J. (6*S*)-2-Nitro-6-({4-[4-(trifluormethoxy)benzyl]benzyl}oxy)-6,7-dihydro-5*H*-imidazo[2,1-*b*][1,3]oxazin,
K. (6*S*)-2-Nitro-6-({(2*E*)-3-[4'-(trifluormethoxy)[1,1'-biphenyl]-4-yl]-2-propenyl}oxy)-6,7-dihydro-5*H*-imidazo[2,1-*b*][1,3]oxazin;
L. (6*S*)-6-({2-Fluor-4-[6-(trifluormethyl)-3-pyridinyl]benzyl}oxy)-2-nitro-6,7-dihydro-5*H*-imidazo[2,1-*b*][1,3]oxazin;
M. (6*S*)-6-{[4-(5-Fluor-2-pyridinyl)benzyl]oxy}-2-nitro-6,7-dihydro-5*H*-imidazo[2,1-*b*][1,3]oxazin;
N. (6*S*)-2-Nitro-6-({3-[4'-(trifluormethoxy)[1,1'-biphenyl]-4-yl]-2-propinyl}oxy)-6,7-dihydro-5*H*-imidazo[2,1-*b*][1,3]oxazin;
O. (6*S*)-2-Nitro-6-[(4-{(*E*)-2-[4-(trifluormethoxy)phenyl]ethenyl}benzyl)oxy]-6,7-dihydro-5*H*-imidazo[2,1-*b*][1,3]oxazin;
P. (6*S*)-2-Nitro-6-[(4-{[4-(trifluormethoxy)phenyl]ethinyl}benzyl)oxy]-6,7-dihydro-5*H*-imidazo[2,1-b][1,3]oxazin; und

Gemischen, optischen oder geometrischen Isomeren und pharmakologisch verträglichen Salzderivaten davon.

**9.** Eine Verbindung ausgewählt aus der Gruppe bestehend aus:

A. (6*S*)-6-{[6-(4-Fluorphenyl)-3-pyridinyl]methoxy}-2-nitro-6,7-dihydro-5*H*-imidazo[2,1-*b*][1,3]oxazin;
B. (6*S*)-2-Nitro-6-({6-[4-(trifluormethoxy)phenyl]-3-pyridinyl}methoxy)-6,7-dihydro-5*H*-imidazo[2,1-*b*][1,3]oxazin;
C. (6*S*)-2-Nitro-6-({5-[4-(trifluormethoxy)phenyl]-2-pyridinyl}methoxy)-6,7-dihydro-5*H*-imidazo[2,1-*b*][1,3]oxazin;
D. (6*S*)-2-Nitro-6-[(5-{[4-(trifluormethoxy)phenyl]ethinyl}-2-pyridinyl)methoxy]-6,7-dihydro-5*H*-imidazo[2,1-

*b*][1,3]oxazin;

E. (6*S*)-6-({6-[3-Fluor-4-(trifluormethoxy)phenyl]-3-pyridinyl}methoxy)-2-nitro-6,7-dihydro-5*H*-imidazo[2,1-*b*][1,3]oxazin;

F. (6*S*)-6-({5-[3-Fluor-4-(trifluormethoxy)phenyl]-2-pyridinyl}methoxy)-2-nitro-6,7-dihydro-5*H*-imidazo[2,1-*b*][1,3]oxazin;

G. (6*S*)-2-Nitro-6-[(5-{[6-(trifluormethyl)-3-pyridinyl]ethinyl}-2-pyridinyl)methoxy]-6,7-dihydro-5*H*-imidazo[2,1-*b*][1,3]oxazin;

H. (6*S*)-6-({6-[3-Chlor-4-(trifluormethoxy)phenyl]-3-pyridinyl}methoxy)-2-nitro-6,7-dihydro-5*H*-imidazo[2,1-*b*][1,3]oxazin;

I. (6*S*)-2-Nitro-6-[(6-{[4-(trifluormethoxy)phenyl]ethinyl}-3-pyridinyl)methoxy]-6,7-dihydro-5*H*-imidazo[2,1-*b*][1,3]oxazin, und

Gemischen, optischen oder geometrischen Isomeren und pharmakologisch verträglichen Salzderivaten davon.

## Revendications

**1.** Composé ayant une structure générale de formule I:

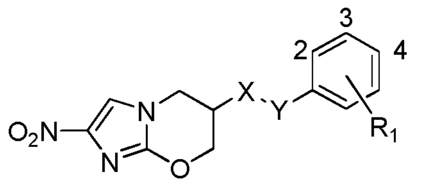

I

où X est O, $OCH_2$, $OCH_2CH=CH$ or $OCH_2C\equiv C$,
Y est l'un quelconque des groupes de formule IIa-IId:

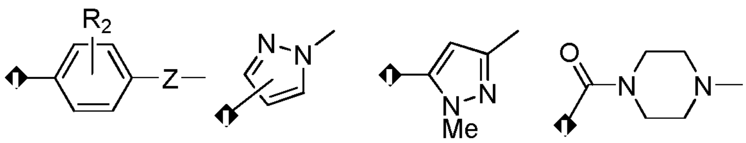

IIa IIb IIc IId

où est un rattachement à X et la formule IIa comprend un cycle ayant $R_2$ comme susbstituant, et Z dans les formules IIa-IIc est $CH_2$, CH=CH, C≡C ou une liaison directe, les nombres 2, 3, et 4 sont des positions de cycle sur un cycle terminal ayant $R_1$ comme substituant,

le cycle terminal de la formule I comprend C, CH ou un aza à chaque position de cycle, et
$R_1$ et $R_2$ dans les formules I et IIa sont chacun un, deux ou trois substituants localisés à toute position de cycle disponible et représentent indépendamment H, F, Cl, $CF_3$, $OCF_2H$, $OCF_3$, ou leurs combinaisons.

**2.** Composé selon la revendication 1, dans lequel :

X est O, $OCH_2$, $OCH_2CH=CH$ ou $OCH_2C\equiv C$,
Y est l'un quelconque des groupes de formules IIa-IIc:

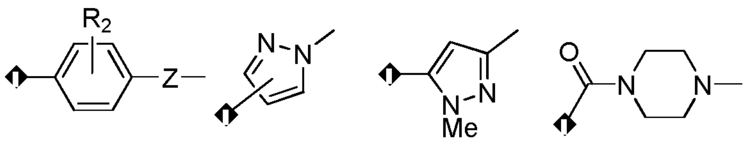

IIa IIb IIc IId

où 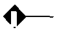 est un rattachement à X et la formule IIa comprend un cycle ayant $R_2$ comme susbstituant, et Z dans les formules IIa-IIc est $CH_2$, CH=CH, C≡C ou une liaison directe,

les nombres 2, 3, et 4 sont des positions de cycle sur un cycle terminal ayant $R_1$ comme substituant,
le cycle terminal de la formule I comprend C ou CH à chaque position de cycle, ou comprend un aza en position 2 et C ou CH à chaque position de cycle restante, ou comprend un aza en position 3 et C ou CH à chaque position de cycle restante,
$R_1$ dans la formule I représente un ou plusieurs groupes choisis parmi: un atome de F localisé en position 4 sur le cycle, un groupe $OCF_3$ localisé en position 4 sur le cycle, Cl localisé en position 2 du cycle, Cl localisé en position 3 du cycle, F localisé en position 3 du cycle, $CF_3$ localisé en position 4 du cycle, ou leurs combinaisons, et
$R_2$ dans la formule IIa représente un groupe ou 2 groupes choisis parmi H ou F, à toute position disponible.

**3.** Composition pharmaceutique comprenant une quantité thérapeutiquement efficace du composé selon la revendication 1.

**4.** Composition pharmaceutique selon la revendication 3, comprenant en outre un excipient, adjuvant, véhicule, tampon ou stabilisant pharmaceutiquement acceptable.

**5.** Composition pharmaceutique selon la revendication 3, comprenant en outre un ou plusieurs traitements anti-infectieux additionnels.

**6.** Composition selon la revendication 3, pour une utilisation dans la prévention et le traitement d'une infection microbienne.

**7.** Composition pour une utilisation selon la revendication 6, dans laquelle l'infection microbienne est causée par le *Mycobacterium tuberculosis*.

**8.** Composé choisi dans le groupe consistant en:

A. (6*S*)-6-{[2'-Chloro-4'-(trifluorométhoxy)[1,1'-biphényl]-4-yl]méthoxy}-2-nitro-6,7-dihydro-5*H*-imidazo[2,1-*b*][1,3]oxazine;
B. (6*S*)-6-{[3'-Fluoro-4'-(trifluorométhoxy)[1,1'-biphényl]-4-yl]méthoxy}-2-nitro-6,7-dihydro-5*H*-imidazo[2,1-*b*][1,3]oxazine;
C. (6*S*)-2-Nitro-6-{[4'-(trifluorométhoxy)[1,1'-biphényl]-4-yl]méthoxy}-6,7-dihydro-5*H*-imidazo[2,1-*b*][1,3]oxazine;
D. (6*S*)-2-Nitro-6-({4-[5-(trifluorométhyl)-2-pyridinyl]benzyl}oxy)-6,7-dihydro-5*H*-imidazo[2,1-*b*][1,3]oxazine;
E. (6*S*)-2-Nitro-6-({4-[6-(trifluorométhyl)-3-pyridinyl]benzyl}oxy)-6,7-dihydro-5*H*-imidazo[2,1-*b*][1,3]oxazine;
F. (6*S*)-6-{[3-Fluoro-4'-(trifluorométhoxy)[1,1'-biphényl]-4-yl]méthoxy}-2-nitro-6,7-dihydro-5*H*-imidazo[2,1-*b*][1,3]oxazine;
G. (6*S*)-2-Nitro-6-{[4'-(trifluorométhoxy)[1,1'-biphényl]-4-yl]oxy}-6,7-dihydro-5*H*-imidazo[2,1-*b*][1,3]oxazine;
H. (6*S*)-6-({2-Fluoro-4-[5-(trifluorométhyl)-2-pyridinyl]benzyl}oxy)-2-nitro-6,7-dihydro-5*H*-imidazo[2,1-*b*][1,3]oxazine;
I. (6*S*)-6-{[2-Fluoro-4'-(trifluorométhoxy)[1,1'-biphényl]-4-yl]méthoxy}-2-nitro-6,7-dihydro-5*H*-imidazo[2,1-*b*][1,3]oxazine;
J. (6*S*)-2-Nitro-6-({4-[4-(trifluorométhoxy)benzyl]benzyl}oxy)-6,7-dihydro-5*H*-imidazo[2,1-*b*][1,3]oxazine;
K. (6*S*)-2-Nitro-6-({(2*E*)-3-[4'-(trifluorométhoxy)[1,1'-biphényl]-4-yl]-2-propényl}oxy)-6,7-dihydro-5*H*-imidazo[2,1-*b*][1,3]oxazine;

L. (6*S*)-6-({2-Fluoro-4-[6-(trifluorométhyl)-3-pyridinyl]benzyl}oxy)-2-nitro-6,7-dihydro-5*H*-imidazo[2,1-*b*][1,3]oxazine;
M. (6*S*)-6-{[4-(5-Fluoro-2-pyridinyl)benzyl]oxy}-2-nitro-6,7-dihydro-5*H*-imidazo[2,1-*b*][1,3]oxazine;
N. (6*S*)-2-Nitro-6-({3-[4'-(trifluorométhoxy)[1,1'-biphényl]-4-yl]-2-propynyl}oxy)-6,7-dihydro-5*H*-imidazo[2,1-*b*][1,3]oxazine;
O. (6*S*)-2-Nitro-6-[(4-{(*E*)-2-[4-(trifluorométhoxy)phényl]ethenyl}benzyl)oxy]-6,7-dihydro-5*H*-imidazo[2,1-*b*][1,3]oxazine;
P. (6*S*)-2-Nitro-6-[(4-{[4-(trifluorométhoxy)phényl]éthynyl}benzyl)oxy]-6,7-dihydro-5*H*-imidazo[2,1-*b*][1,3]oxazine;

et leurs mélanges, isomères optiques ou géométriques, et dérivés sous forme de sels pharmacologiquement acceptables.

**9.** Composé choisi dans le groupe consistant en :

A. (6*S*)-6-{[6-(4-Fluorophényl)-3-pyridinyl]méthoxy}-2-nitro-6,7-dihydro-5*H*-imidazo[2,1-*b*][1,3]oxazine;
B. (6*S*)-2-Nitro-6-({6-[4-(trifluorométhoxy)phényl]-3-pyridinyl}méthoxy)-6,7-dihydro-5*H*-imidazo[2,1-*b*][1,3]oxazine;
C. (6*S*)-2-Nitro-6-({5-[4-(trifluorométhoxy)phényl]-2-pyridinyl}méthoxy)-6,7-dihydro-5*H*-imidazo[2,1-*b*][1,3]oxazine;
D. (6*S*)-2-Nitro-6-[(5-{[4-(trifluorométhoxy)phényl]éthynyl}-2-pyridinyl)méthoxy]-6,7-dihydro-5*H*-imidazo[2,1-*b*][1,3]oxazine,
E. (6*S*)-6-({6-[3-Fluoro-4-(trifluorométhoxy)phényl]-3-pyridinyl}méthoxy)-2-nitro-6,7-dihydro-5*H*-imidazo[2,1-*b*][1,3]oxazine;
F. (6*S*)-6-({5-[3-Fluoro-4-(trifluorométhoxy)phényl]-2-pyridinyl}méthoxy)-2-nitro-6,7-dihydro-5*H*-imidazo[2,1-*b*][1,3]oxazine;
G. (6*S*)-2-Nitro-6-[(5-{[6-(trifluorométhyl)-3-pyridinyl]éthynyl}-2-pyridinyl)méthoxy]-6,7-dihydro-5*H*-imidazo[2,1-*b*][1,3]oxazine;
H. (6*S*)-6-({6-[3-Chloro-4-(trifluorométhoxy)phényl]-3-pyridinyl}méthoxy)-2-nitro-6,7-dihydro-5*H*-imidazo[2,1-*b*][1,3]oxazine;
I. (6*S*)-2-Nitro-6-[(6-{[4-(trifluorométhoxy)phényl]éthynyl}-3-pyridinyl)méthoxy]-6,7-dihydro-5*H*-imidazo[2,1-*b*][1,3]oxazine; et leurs mélanges, isomères optiques ou géométriques, et dérivés sous forme de sels pharmacologiquement acceptables.

# Figure 1

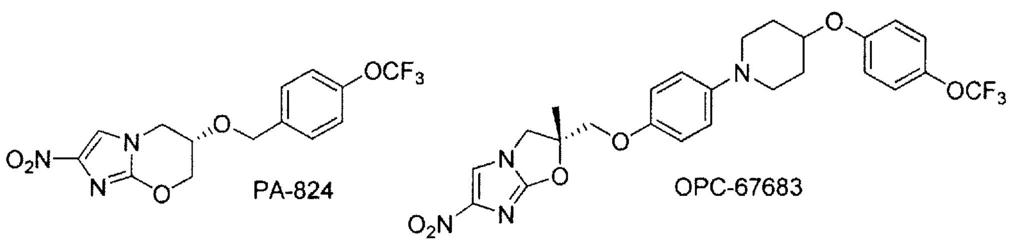

PA-824

OPC-67683

# Figure 2

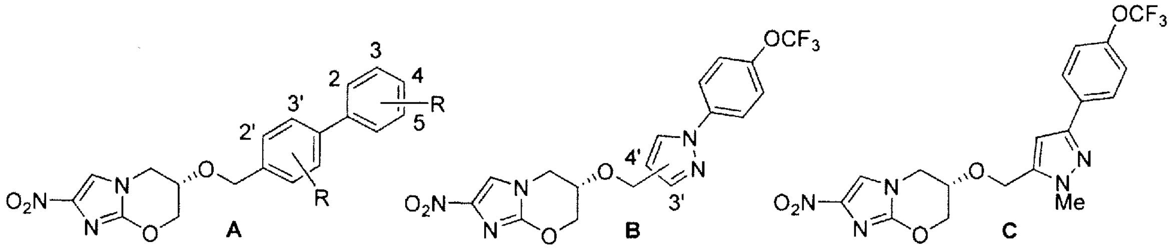

A B C

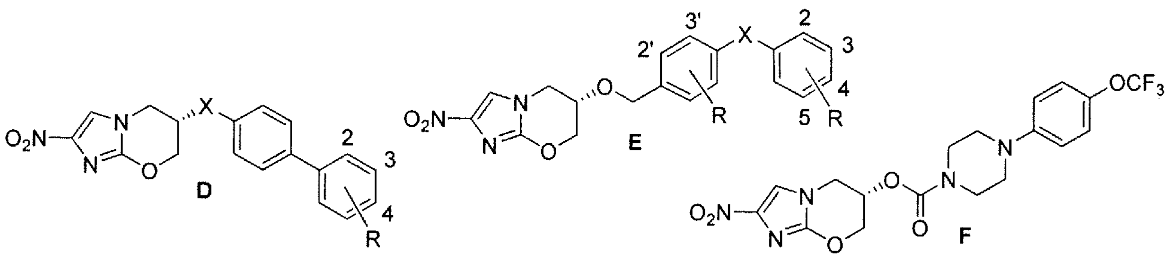

D E F

# Figure 3

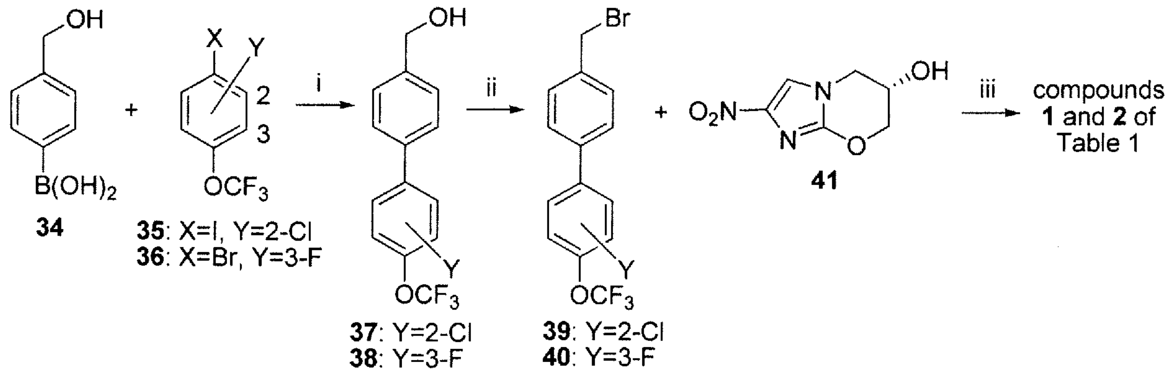


**34**

**35**: X=I, Y=2-Cl
**36**: X=Br, Y=3-F

**37**: Y=2-Cl
**38**: Y=3-F

**39**: Y=2-Cl
**40**: Y=3-F

**41**

# Figure 4

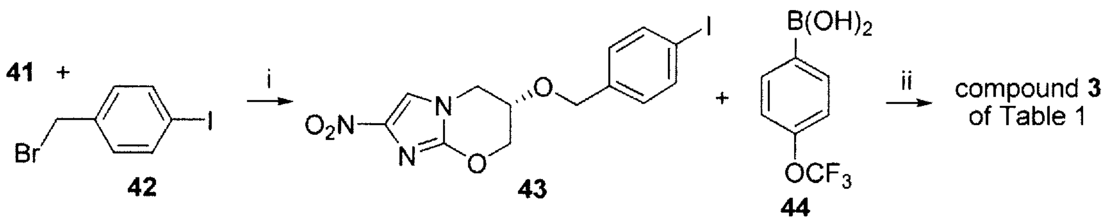


41 + 42 → 43 + 44 → compound **3** of Table 1

# Figure 5

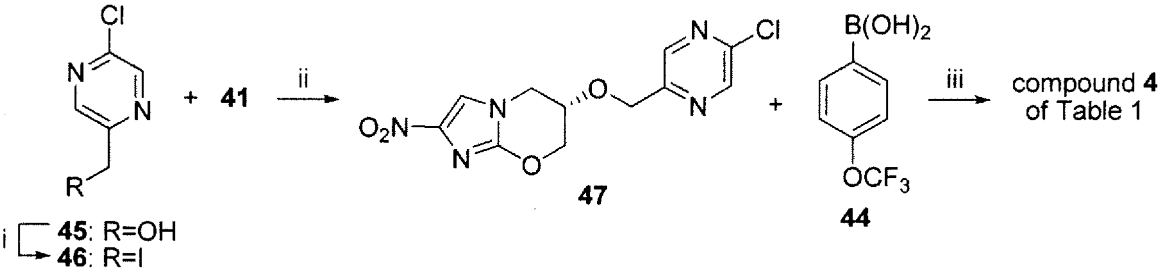

# Figure 6

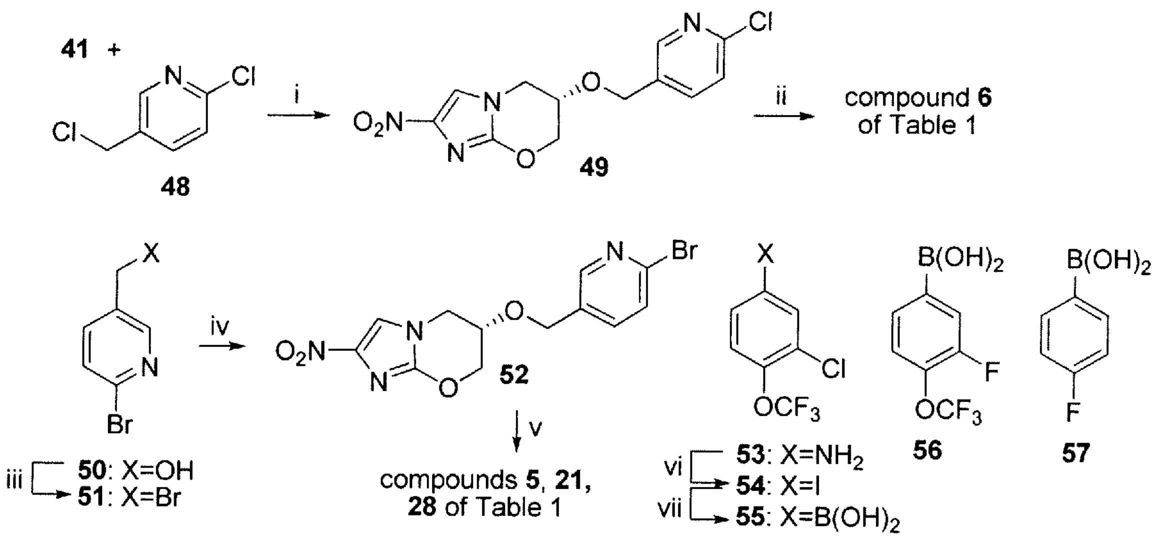
41 +
48
i
49
ii
compound 6 of Table 1
iv
52
v
compounds 5, 21, 28 of Table 1
iii
50: X=OH
51: X=Br
vi
53: X=NH2
54: X=I
vii
55: X=B(OH)2
56
57

# Figure 7

41 +
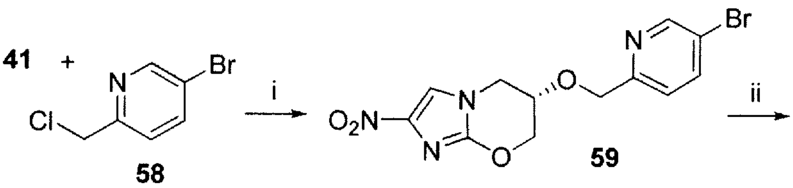

58
59
compound **7** of Table 1

# Figure 8

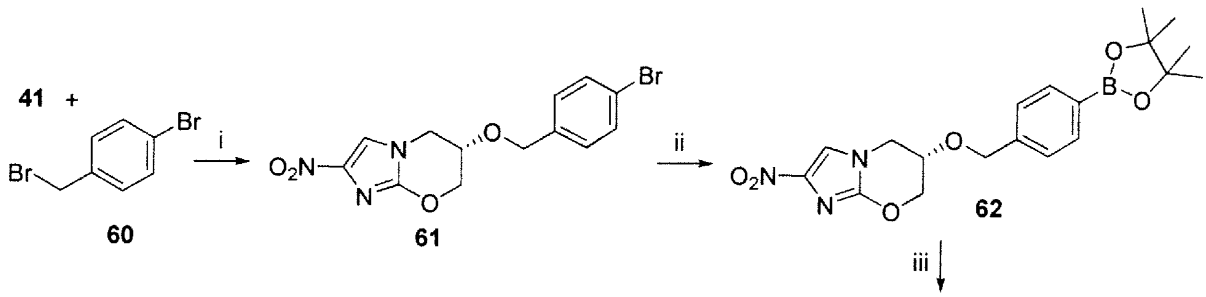


41 +

60

61

62

compounds **8** and **9** of Table 1

# Figure 9

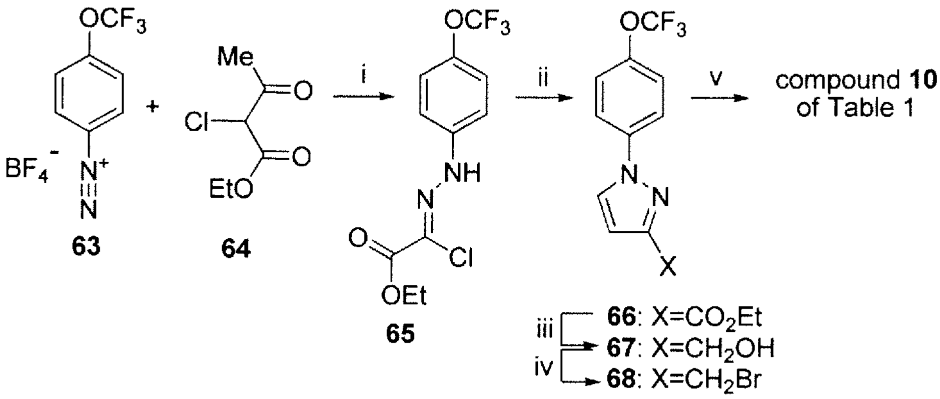


63 64 65

66: X=$CO_2Et$
67: X=$CH_2OH$
68: X=$CH_2Br$

# Figure 10

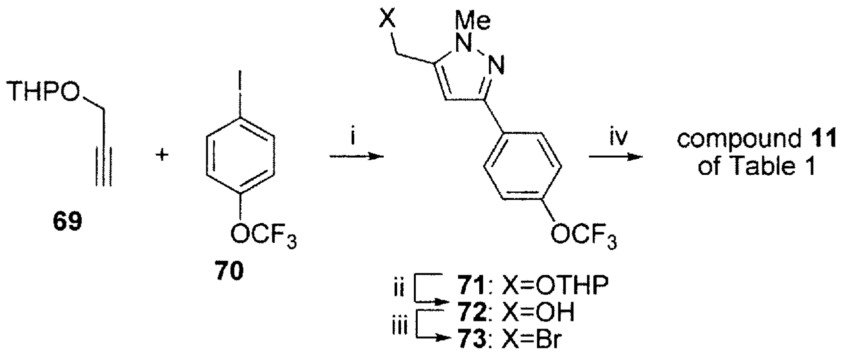


69

70

71: X=OTHP
72: X=OH
73: X=Br

# Figure 11

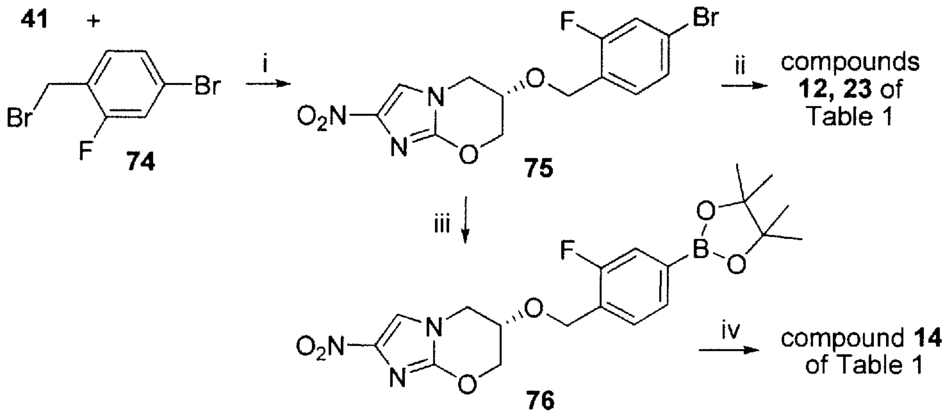
41 +
74
i
75
ii
compounds
12, 23 of
Table 1
iii
76
iv
compound 14
of Table 1

# Figure 12

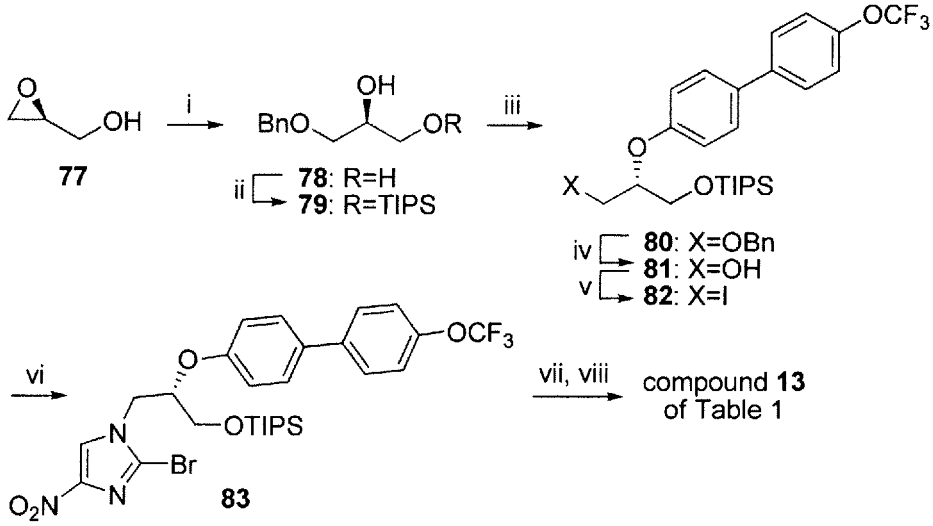


77

78: R=H

79: R=TIPS

80: X=OBn

81: X=OH

82: X=I

83

compound **13** of Table 1

# Figure 13

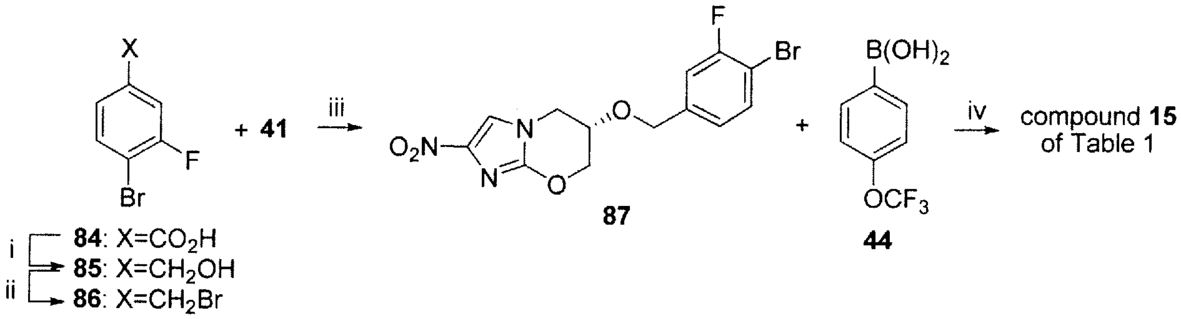


84: X=$CO_2H$
85: X=$CH_2OH$
86: X=$CH_2Br$
87
44

# Figure 14

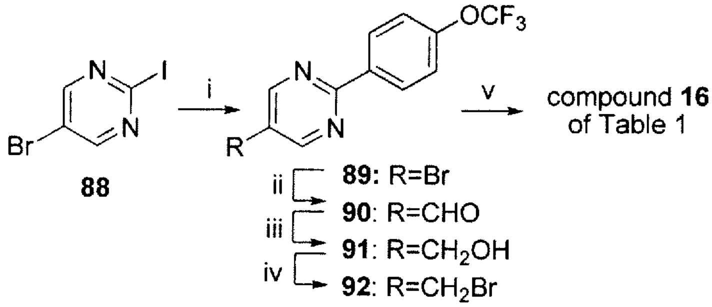


**88**

**89:** R=Br

**90**: R=CHO

**91**: R=$CH_2OH$

**92**: R=$CH_2Br$

# Figure 15

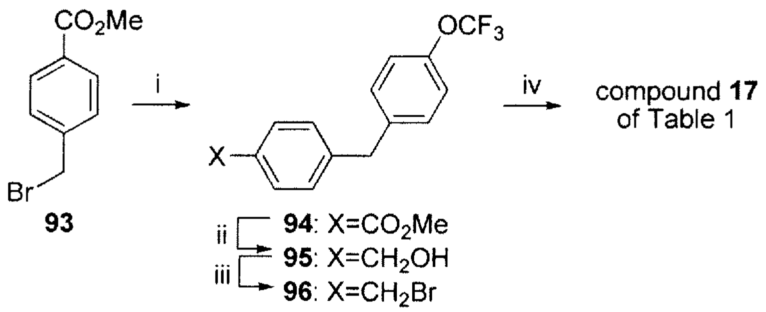


93

94: X=$CO_2Me$

95: X=$CH_2OH$

96: X=$CH_2Br$

compound **17** of Table 1

# Figure 16

59

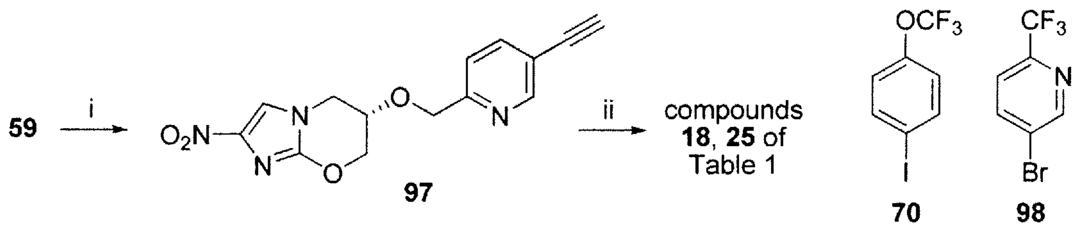


97

compounds **18**, **25** of Table 1

70 98

# Figure 17

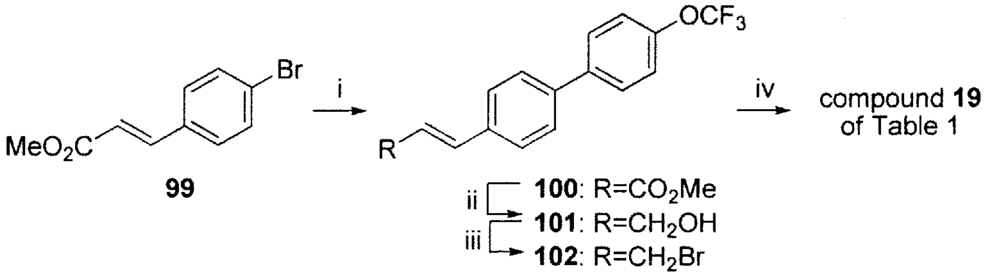


**99**

**100**: R=$CO_2Me$

**101**: R=$CH_2OH$

**102**: R=$CH_2Br$

# Figure 18

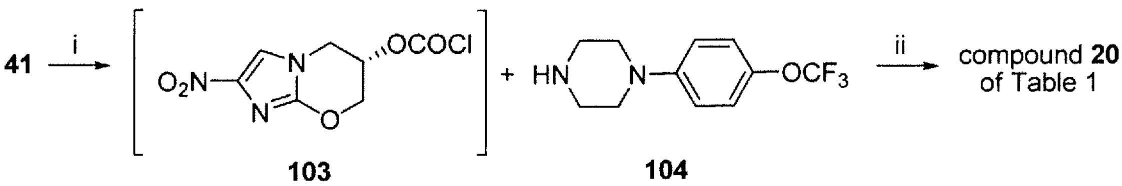


41

103

104

compound **20** of Table 1

# Figure 19

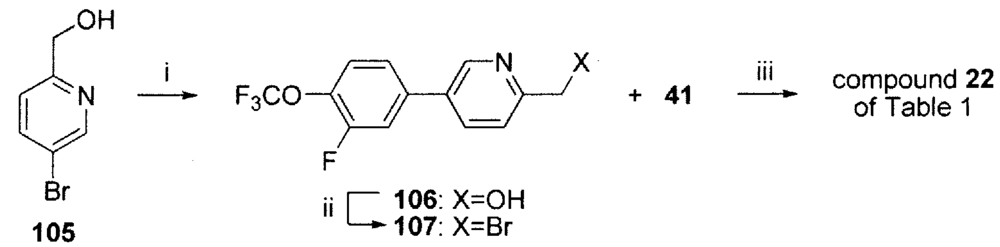


105

106: X=OH

107: X=Br

+ 41

compound 22 of Table 1

# Figure 20

**41** +

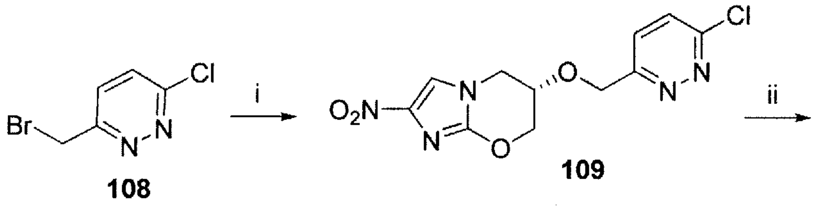


**108**

**109**

compound **24** of Table 1

# Figure 21

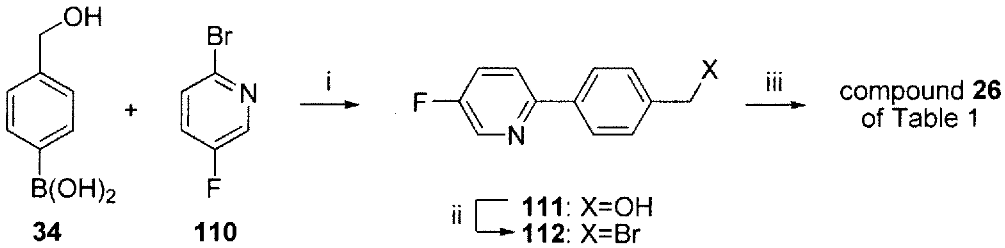


**34** **110**

# Figure 22

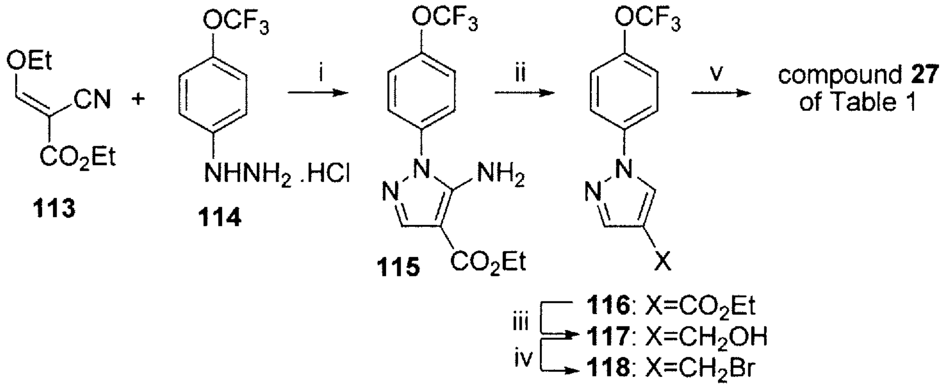


113

114

115

compound **27** of Table 1

**116**: X=$CO_2Et$

**117**: X=$CH_2OH$

**118**: X=$CH_2Br$

# Figure 23

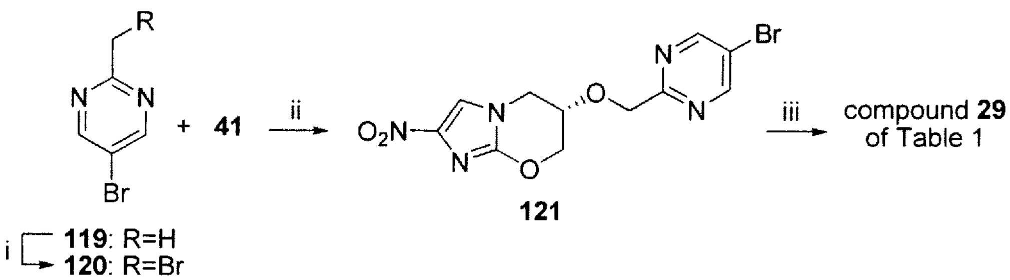


+ 41

121

compound **29** of Table 1

**119**: R=H
**120**: R=Br

# Figure 24

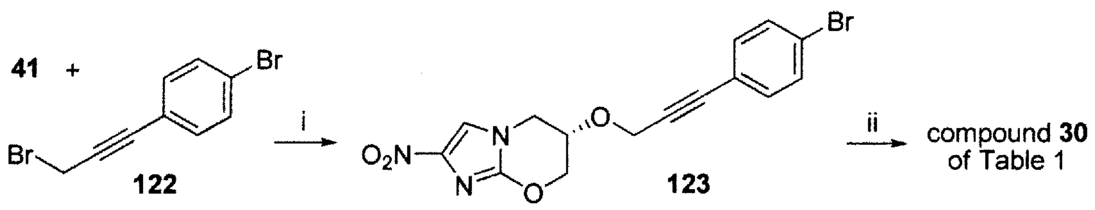


41 + 122 → 123

# Figure 25

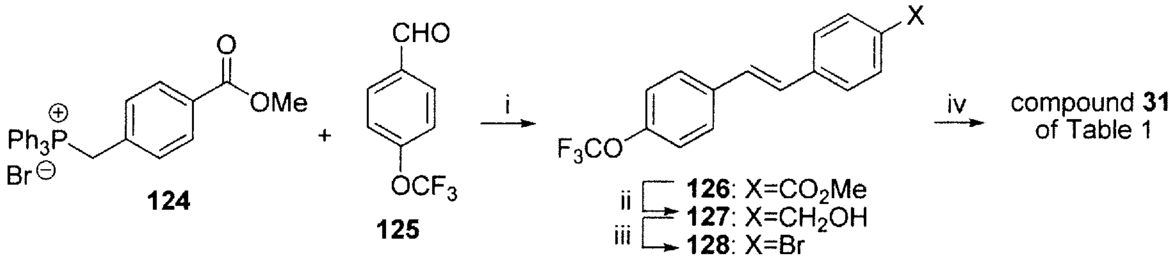


124

125

**126**: X=$CO_2Me$
**127**: X=$CH_2OH$
**128**: X=Br

compound **31** of Table 1

# Figure 26

43

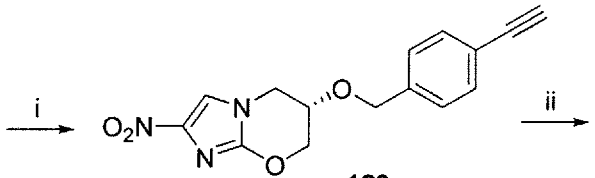


129

compound **32** of Table 1

52

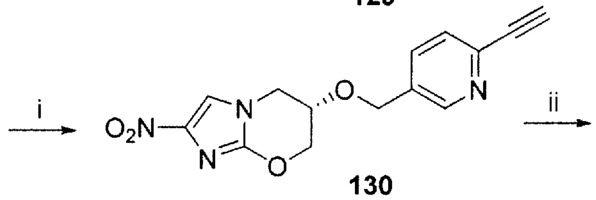


130

compound **33** of Table 1

# Figure 27

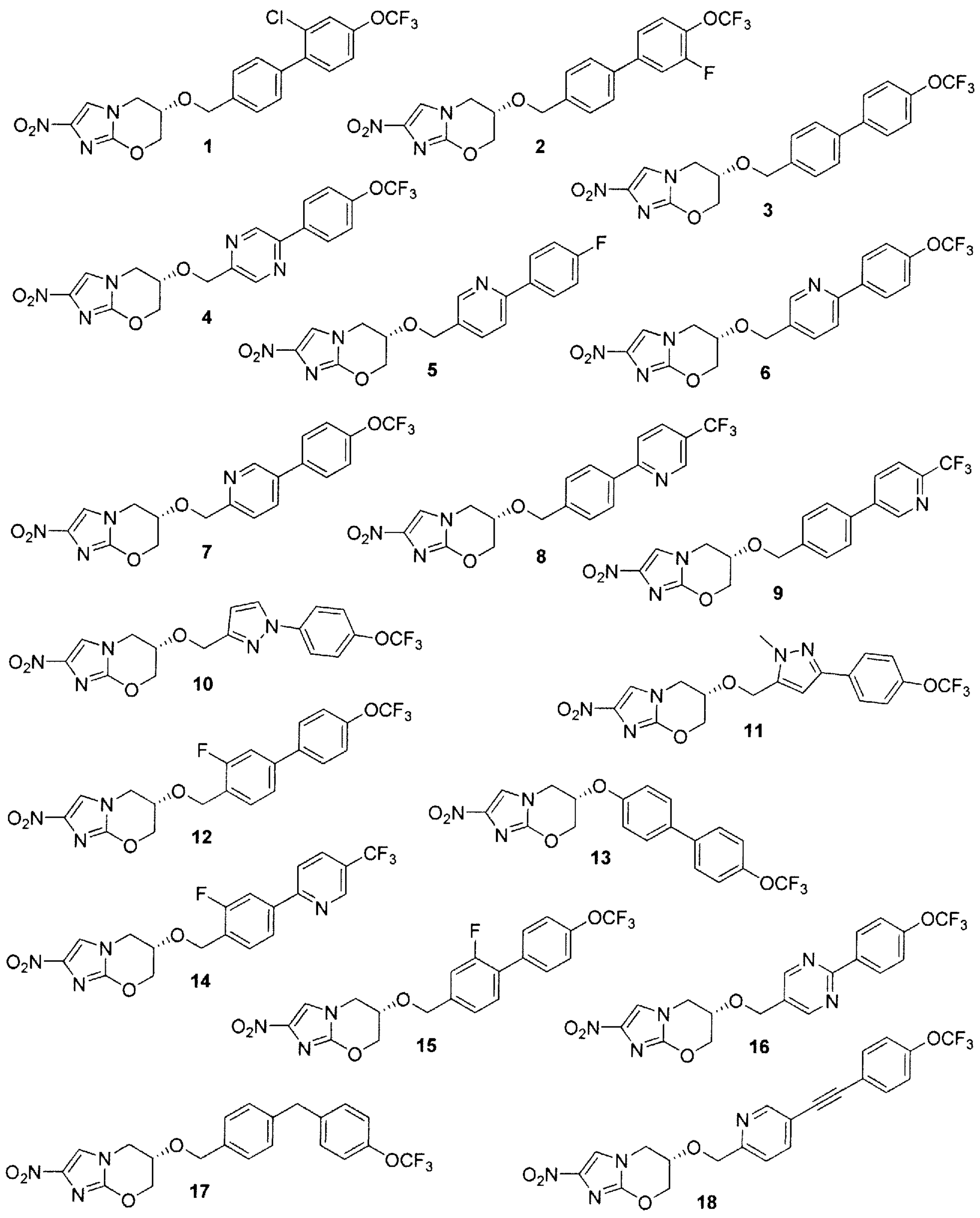

1 2 3 4 5 6 7 8 9 10 11 12 13 14 15 16 17 18

# Figure 28

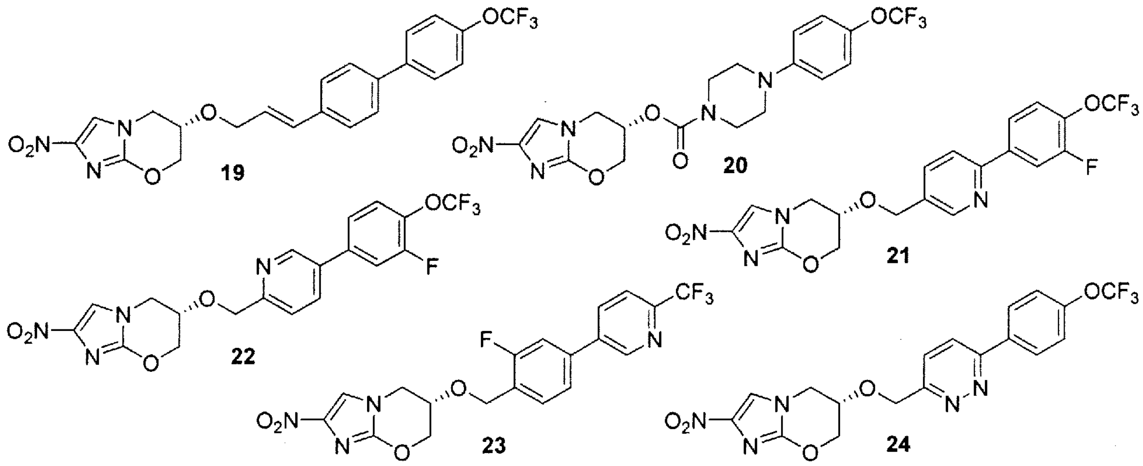

19 20 21 22 23 24

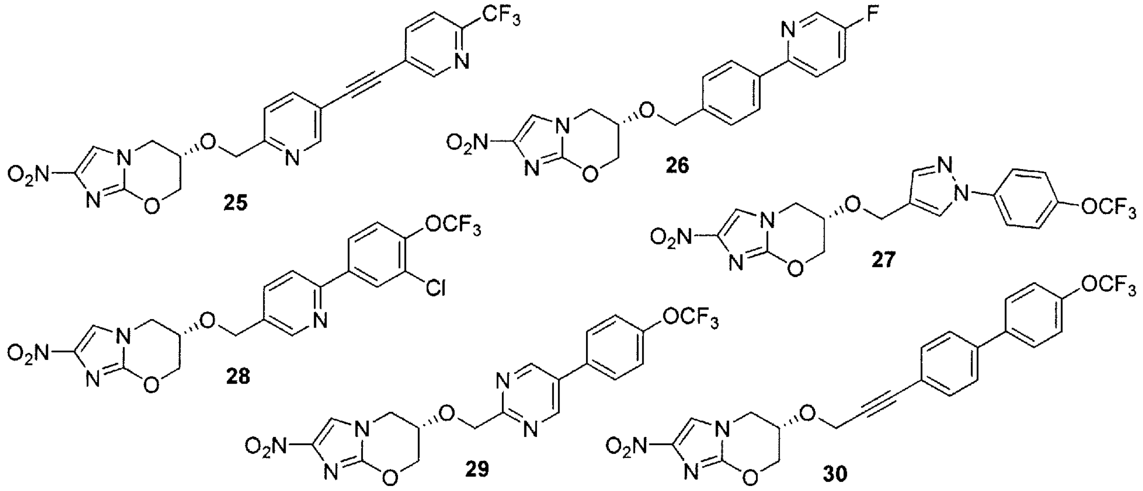

25 26 27 28 29 30

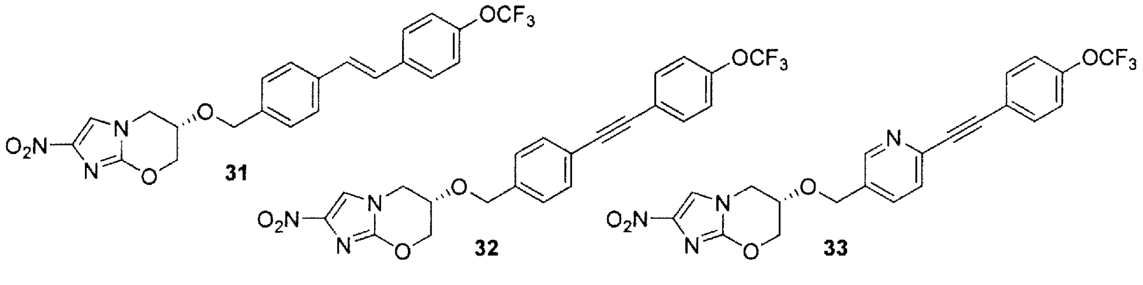

31 32 33

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5668127 A
- US 6087358 A
- US 2000 A
- WO 2007075872 A2, Jiricek
- EP 1555259 A
- WO 9524400 A
- WO 2007075872 A


### Non-patent literature cited in the description


- **ANDERSON et al.** *Org. Biomol. Chem.,* 2008, vol. 6, 1973-1980
- **CHO et al.** *Antimicrob. Agents Chemother.,* 2007, vol. 51, 1380-1385
- **COLLINS et al.** *Antimicrob. Agents Chemother.,* 1997, vol. 41, 1004-1009
- **DESOLMS et al.** *J. Med. Chem.,* 2003, vol. 46, 2973-2984
- **EDSALL et al.** *Bioorg. Med. Chem.,* 2003, vol. 11, 3457-3474
- **FALZARI et al.** *Antimicrob. Agents Chemother.,* 2005, vol. 49, 1447-1454
- **FERRARA et al.** *Lancet,* 2006, vol. 367, 1328-1334
- **KIENER et al.** *Synlett,* 1994, vol. 10, 814-816
- **KIM et al.** *J. Med. Chem.,* 2009, vol. 52, 1317-1328, 1329-1344
- **LI et al.** *Bioorg. Med. Chem. Lett.,* 2008, vol. 18, 2256-2262
- **MANJUNATHA et al.** *Proc, Natl. Acad. Sci. USA,* 2006, vol. 103, 431-436
- **SASAKI et al.** *J. Med. Chem.,* 2006, vol. 49, 7854-7860
- **SCHUBERT et al.** *Synlett,* 1999, vol. 3, 342-344
- **SINGH et al.** *Science,* 2008, vol. 322, 1392-1395
- **STOVER et al.** *Nature,* 2000, vol. 405, 962-966
- **TYAGI et al.** *Antimicrob. Agents Chemother.,* 2005, vol. 49, 2289-2293
- **VAN DEN HEUVEL et al.** *J. Org. Chem.,* 2004, vol. 69, 250-262